# EUROPEAN PATENT SPECIFICATION

(11) **EP 3 762 037 B1**
(45) Date of publication and mention of the grant of the patent: **27.12.2023**
(21) Application number: 19717146.5
(22) Date of filing: 11.03.2019
(51) Int. Cl.: A61K 47/50, A61K 47/68, A61P 35/00

(54) **RELEASABLE ANTIBODY CONJUGATES**
LÖSBARE ANTIKÖRPERKONJUGATE
CONJUGUÉS D'ANTICORPS LIBÉRABLES

(30) Priority: 09.03.2018 US 201862640733 P
(43) Date of publication of application: 13.01.2021
(73) Proprietor: QuiaPEG Pharmaceuticals AB, 75450 Uppsala (SE)
(72) Inventor: KWIATKOWSKI, Marek, 756 45 Uppsala (SE); SUND, Christian, 143 43 Varby (SE)
(74) Representative: Fish & Richardson P.C.
(86) International application number: PCT/IB2019/051956
(87) International publication number: WO 2019/171358

(56) References cited:
- WO-A1-2015/095755
- WO-A1-2015/195904
- WO-A2-2013/186632

## Description

### CROSS-REFERENCE TO RELATED APPLICATIONS

This application claims priority to U.S. Application Serial No. 62/640,733, filed on March 9, 2018.

### TECHNICAL FIELD

This document relates to conjugates of a cytotoxic or chemotherapeutic compound bound directly or indirectly to a targeting antibody, wherein the cytotoxic or chemotherapeutic compound can be released from the conjugate in vivo. Such conjugates are referred to herein as "releasable antibody conjugates". This document further discloses methods and materials for making and using such releasable antibody conjugates.

### BACKGROUND

Antibody-drug conjugates or ADCs are an important class of highly potent biopharmaceutical drugs designed as a targeted therapy (e.g., for the treatment of cancer). For example, unlike chemotherapy, ADCs are intended to target and kill only the cancer cells and spare healthy cells. ADCs are complex molecules composed of an antibody linked to a biologically active molecule (e.g., a cytotoxic (anticancer) payload or drug).

WO 2015/195904 A1 describes antibody-drug conjugates inclduing an antobody as a targeting moiety, a linker which contains an acyl unit, an optional spacer unit for providing distance between the drug moiety and the targeting moiety, a peptide linker which can be cleaved under appropriate conditions, a hydrophilic self-immolative linker, and an optional second self-immolative spacer or cyclization self-elimination linker.

WO 2015/095755 A1 describes ligand-drug conjugates, where the ligand-drug conjugate is comprised of a self-immolative assembly unit having a methylene carbamate unit for conjugation of a drug to a targeting ligand.

### SUMMARY

The present invention is defined by independent claim 1. The dependent claims depict additional embodiments of the invention.

Provided herein are conjugates of a cytotoxic or chemotherapeutic compound bound indirectly (e.g., through a linking moiety, for example an aliphatic polymer), to an antibody (e.g., a monoclonal antibody or antibody fragment), wherein the cytotoxic or chemotherapeutic compound can be released from the conjugate in vivo. Such conjugates are referred to herein as "releasable antibody conjugates". This document further includes methods and materials for making and using such releasable antibody conjugates.

The releasable antibody conjugates provided herein are based on the discovery that 3' phosphotriester groups of a ribonucleoside are unstable in the presence of free vicinal 2' hydroxyl/amino moieties and can decompose following intramolecular nucleophilic attack of a 2' hydroxyl/amino group moiety at the 3' phosphotriester group. The subsequent decomposition reaction is thought to be controlled by the geometry of the attacking nucleophile and the phosphorus atom, with the ribo configuration being the most reactive species and analog arabino-geometries being practically unreactive. The conjugates provided herein advantageously provide release of a cytotoxic or chemotherapeutic compound from an antibody-containing conjugate with little to no trace of the previously existing linker and targeting antibody on the cytotoxic or chemotherapeutic compound. The same is true for other targeting affinity compounds. One such example is shown in Scheme 1 as follows: 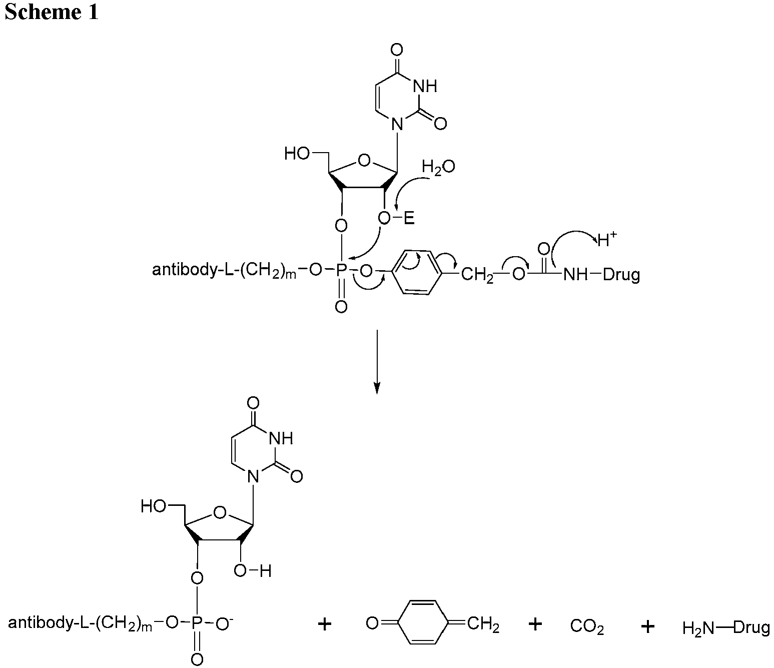 In this example, cleavage of group E, the trigger moiety, results in a nucleophilic attack of the liberated hydroxyl on the phosphorus atom leading to the formation of a cyclic phosphotriester, quinone methide, carbon dioxide (CO₂), and the cytotoxic or chemotherapeutic compound. The cyclic phosphotriester may be further hydrolyzed at physiological pH resulting in the opening of the 5-membered ring and formation of both isomeric phosphodiesters. Quinone methide may also be further hydrolyzed (e.g., reacting with water) at physiological pH to form 4-(hydroxymethyl)-phenol.

Such a system provides several advantages over alternative antibody targeted conjugates. For example, the system is modifiable, and cleavage can be altered based upon the identity of the trigger moiety, "E", as exemplified above. For example, E can contain an enzyme-labile group, an acid labile functionality, or be a cleavable upon reduction, dithiol containing functional group. Moreover, as group E is not bound directly to the antibody, the need to extensively modify each E for every conjugate is avoided as the basic (non-derivatized) form of any E moiety can be appended to the conjugates as described herein. In addition, the use of non-substituted trigger groups offers the potential for better control over the kinetics of prodrug disintegration and liberation of the free biologically active molecule *in vivo.* Finally, the releasable antibody conjugates provided herein exhibit substantial synthetic freedom. For example, looking at Scheme 1 above, it is not required to have the E moiety introduced selectively on the 2' hydroxyl along with introduction of the phosphotriester on the 3' hydroxyl of the linker moiety. In fact, the opposite placement behaves similarly, making both positional isomers, whether alone or in combination, suitable and useful as releasable antibody conjugates.

The chemical methods described herein allow for the drug to be released selectively and completely in response to the hydrolysys of trigger E and liberation of nucleophile A, however, this method leaves some freedom of choice in regard to the position of the linkage of the antibody moiety. For example, in the structure below, the antibody moiety can be placed in any of the positions indicated by an arrow:

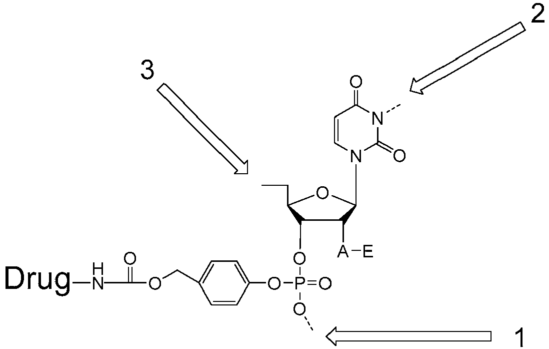

Each of these positions has its own advantages and their practical exemplification is demonstrated herein.

In a first general aspect, this document provides a compound of Formula (I): 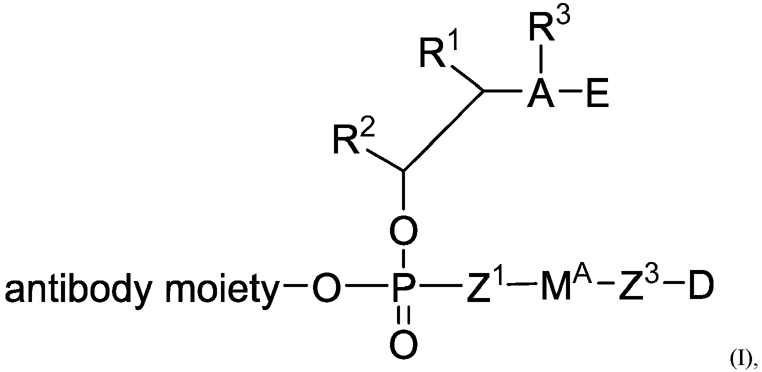 or a pharmaceutically acceptable salt thereof, wherein:
the antibody moiety is selected from:
antibody-L-(CH₂)_{q}- and antibody-L-(aliphatic moiety)-;
the aliphatic moiety is selected from a polymer, R^{P}, and a group selected from: polymer-L-(CH₂)ₘ- and polymer-L-(CH₂-CH₂-O)ₚ-(CH₂)ₘ-;
R^{P} is selected from optionally substituted C₁₋₆ alkyl, optionally substituted C₁₋₃ alkyl-O-(CH₂-CH₂-O)ₚ-(CH₂)ₘ-, and optionally substituted C₃₋₇ cycloalkyl;
each L is independently a linking group;
m, p, and q are each independently an integer from 1 to 10;
D is a residue of a cytotoxic or chemotherapeutic compound;
Z¹ is selected from O, S, and N(R^{N});
Z³ is selected from O and N(R^{N}), or Z³ is absent;
A is O or N, wherein when A is O then R³ is absent;
R^{N} is selected from H and optionally substituted C₁₋₆ alkyl;
R³ is selected from H and C₁₋₆ alkyl, or
R³ and R¹, together with A and the carbon atom to which R¹ is attached, form an optionally substituted 4 to 7 membered aliphatic heterocyclic ring; or
R³ and R², together with A, the carbon atom to which R¹ is attached, and the carbon atom to which R² is attached, form an optionally substituted 4 to 8 membered aliphatic heterocyclic ring;
M^{A} is a self-immolative group having any one of formulae (a)-(i): 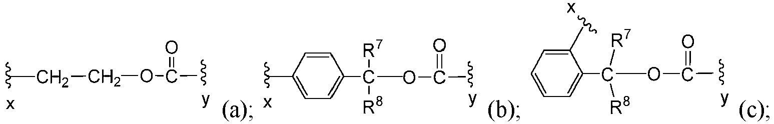 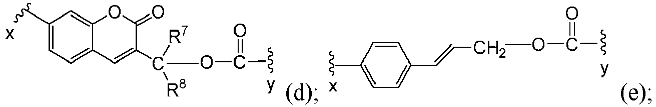 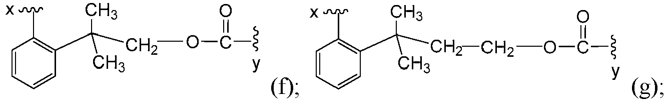 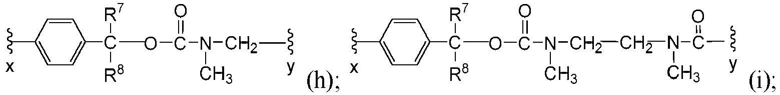
wherein x denotes a point of attachment to Z¹ and y denotes a point of attachment to z3.
R¹ and R² are independently selected from the group consisting of hydrogen, optionally substituted C₁₋₆ alkyl, optionally substituted C₆₋₁₀ aryl and optionally substituted 5- to 14-membered heteroaryl;
or R¹and R² are joined together with the carbon atoms to which they are attached to form an optionally substituted C₃₋₇ cycloalkyl ring, an optionally substituted 4 to 7 membered aliphatic heterocyclic ring, an optionally substituted C₆₋₁₀ aryl or an optionally substituted 5- to 14-membered heteroaryl;
or R¹ and R² are joined together to form a ribose ring system;
R⁷ and R⁸ are independently selected from H and C₁₋₆ alkyl; and
E is a cleavable moiety.

In a third general aspect, this document provides a pharmaceutical composition comprising any one of the compounds described herein, or a pharmaceutically acceptable salt thereof, and a pharmaceutically acceptable carrier or excipient.

In a fourth general aspect, this document provides a compound or a pharmaceutically acceptable salt thereof, or the pharmaceutical composition for use in a method of treating cancer in a subject in need thereof, the method comprising administering to the subject a therapeutically effective amount of any one of the compounds described herein, or a pharmaceutically acceptable salt thereof, or a pharmaceutical composition comprising any one of the compounds described herein, or a pharmaceutically acceptable salt thereof.

Unless otherwise defined, all technical and scientific terms used herein have the same meaning as commonly understood by one of ordinary skill in the art to which the present application pertains. Methods and materials are described herein for use in the present application; other, suitable methods and materials known in the art can also be used. The materials, methods, and examples are illustrative only and not intended to be limiting. In case of conflict between the present specification and a reference mentioned herein, the present specification, including definitions, will control.

Other features and advantages of the present application will be apparent from the following detailed description and figures, and from the claims.

### DETAILED DESCRIPTION

When designing properties for a drug, including a protein drug, to be administered to humans and animals (S.B. van Witteloostuijn et al, Half-life extension of biopharmaceuticals using chemical methods: Alternatives to PEGylation, ChemMedChem Reviews, 11: 2474-2495, 2016.; M. C. Manning, et al., Stability of Protein Pharmaceuticals: An Update, Pharmaceutical Research, 27: 544-575, 2010) certain key properties include one or more of the following:
1. Ability to bind to specific target sites
2. Reasonable soluble in the circulation at effective doses
3. Exhibit circulation half-lives that allow for effective targeting and dosing. This is often associated with extended half-lives due to reduced glomerular filtration or liver clearance.
4. Not enzymatically or otherwise chemically altered in the circulation, in a manner which reduces their efficacy.
5. Physically (conformationally) stable in formulation, delivery and circulation.
6. Do not aggregate in the circulation, which can lead to inefficacy, as well as various complications.
7. Not immunogenic under acute or chronic usage.

There are many approaches to achieve the above, however, these approaches tend to be grouped under (I) methods involving chemical alteration of the drug, or (II) methods involving alteration in how the drug is administered. Type I methods can include adding a hydroxyl group so as to enhance aqueous solubility or modification of the drug with a hydrophilic polymer so as to both increase size (and reduce glomerular filtration) as well as solubility. Type II methods can include administering a poorly aqueous soluble drug such as doxorubicin in a drug delivery vehicle such as a water-soluble liposomal drug carrier, or delivering the drug subcutaneously in a polymeric hydrogel carrier from which the drug slowly diffuses into the circulation (van Witteloostuijn et al.; Manning et al., A. Kumari, et al.; Biodegradable polymeric nanoparticles based drug delivery systems, Colloids and Surfaces B: Biointerfaces 75: 1-18, 2010).

One of the most common methods to enhance the serum half-life of a small molecular weight drug or a protein drug is via their modification with a hydrophilic polymer such as poly(ethylene glycol) or PEG. At present, there are approximately 17 "PEGylated" registered pharmaceuticals which have been FDA/EMA approved (e.g. Adagen^{®}, Oncospar^{®}, PEGintron^{®}, Pegasys^{®}, Somavert^{®}, Neulasta^{®}, Mircera^{®}, Cimzia^{®}, Krystexxa^{®}, Longuex^{®}, Plegridy^{®}, Adynovate^{®}, Rebinyn^{®}, Jivi^{®}, Calaspargase^{®}, Movantik^{®}, Omontys^{®}) (van Witteloostuijn et al.). The hydrophilic, biocompatible nature of PEG is such that when covalently attached to a small molecular weight drug or protein it effectively coats it with a PEG-rich surface layer (C. J. Fee and J. M. Van Alstine, Prediction of the Viscosity Radius and the Size Exclusion Chromatography Behavior of PEGylated Proteins, Bioconjugate Chem., 15: 1304-1313, 2004) whose flexibility allows the molecules to still bind to targets, while potentially enhancing all of the above noted desired properties (C. J. Fee and J. M. Van Alstine, PEG-proteins: Reaction engineering and separation issues, Chemical Engineering Science 61: 924 - 939, 2006, Fee and Van Alstine, 2004; van Witteloostuijn et al.).

Modification of small molecular weight drugs or biopharmaceuticals with PEG or other hydrophilic polymers (collectively referred to herein as PEGylation) provides great flexibility to production of complicated pharmaceuticals including antibody drug conjugates. One reason for this is that PEG and many related polymers are soluble in both organic and aqueous solutions. Modification of hydrophobic drugs or proteins can be carried out in organic solutions and the polymer-modified drug can then be further modified e. g., coupled to a targeting protein or ligand, or further purified in aqueous solution. Aqueous solution-based purifications are typically less expensive, safer to operate, more environmentally friendly, and require less complicated process lines.

Modification of small molecular weight drugs or biopharmaceuticals with PEG or other hydrophilic polymers (collectively referred to herein as PEGylation) comes with several challenges. To begin with, normally PEGylation is undertaken on already purified substances. For example, many polymer modified biopharmaceuticals with yearly sales of over one billion dollars (e.g., PEGintron, Pegasys, Neulasta) are PEGylated enhanced serum half-life versions of already proven biopharmaceuticals. In an effort to have a chemically well-defined product, normally such PEG-biopharmaceuticals are modified at one amino acid site (e.g., a carboxyl or amino terminal group, the free amino group of a lysine amino acid residue, or a cysteine sulfhydryl group). The resulting reaction product mixture may contain one third each of monoPEGylated drug product, multi-PEGylated entities, unmodified native drug or protein. As a result, the amount of functional drug product is reduced to one third of the starting material (on a mole ratio) and it must be purified from the reaction product mixture - which reduces yield even further while adding on costs related to purification, quality control, etc. To make matters more challenging, a mixture of monoPEGylated drug product may not be PEGylated at the same sites leading to what are called PEGameric mixtures where different "PEGamers" may exhibit different pharmacokinetic properties. The latter is acceptable if the reaction conditions are highly controlled and reproducible (C. J. Fee and J. M. Van Alstine, PEG-proteins: Reaction engineering and separation issues, Chemical Engineering Science 61: 924 - 939, 2006). The value of the benefits of PEGylation are such that, as evidenced by the large number of approved PEGylated drugs, the challenges related to PEGylation are often economically or medically outweighed by the benefits. However, the above PEGylation challenges have yet to be overcome and much work remains to be done to improve covalently PEGylated biopharmaceuticals (van Witteloostuijn et al.; R. L. Kwant, et al., Controlled levels of protein modification through a chromatography-mediated bioconjugation. Royal Society of Chemistry, Chem. Sci., 6: 2596-2601, 2015).

One advantage of releasable (reversible) PEGylation technology is that since the polymer is not coupled to the pharmaco-active substance when it is functional, the exact nature or heterogeneity of polymer modification in regard to the exact site of modification, exact polymer molecular weight, etc., becomes less important in regards to drug efficacy and variability. The polymer modified drug, which can also be conjugated to a targeting agent such as an antibody, is in effect a prodrug which should allow for much more predictabile *in vivo* behavior, particularly if the drug and antibody the polymer is linking to are approved or have been clinically well-characterized (i.e., in Clinical Phase Trials).

PEGylation at a site physically removed from the active site typically reduces a drug or protein's affinity for its target by about one order of magnitude (discussed in Fee and Van Alstine, 2004, 2006). In some cases, this does not prevent PEG-drug efficacy and approval, and but it is believed that in other cases, especially where PEGylation occurs near the active site or otherwise greatly reduces drug targeting or activity, it prohibits development of an effective long acting, better tolerated, PEG-drug. Releasable linkages can provide a solution to this challenge.

While PEG-conjugation is a common approach to modification of drugs or biopharmaceuticals with polymers, many other hydrophilic polymers (Veeran Gowda Kadajji and Guru V. Betageri, Water Soluble Polymers for Pharmaceutical Applications, Polymers, 3: 1972-2009, 2011) including polyoxazolines are known to be similarly useful (e.g., Zalipsky et al., US5395619A; Harris, WO 2010/006282 A2).

Since the time of Pasteur doctors have dreamed of being able to deliver highly active drug substances to specific parts of the body. Effective targeting of drugs is a common problem with many highly toxic drugs such as anticancer drugs like doxorubicin or daunomycin requiring administration at very high doses, which can lead to systemic patient complications and often limit the ability of many patients to take certain drugs, or to take such drugs in doses high enough to be fully effective. The idea of using antibodies to delivery cell toxins to cancer cells was first investigated by Mathe et al., in 1958 (G. Mathe, T. B. Loc, J. Bernard. Effect on mouse leukemia 1210 of a combination by diazo-reaction of amethopterin and gammaglobulins from hamsters inoculated with such leukemia by heterografts. C R Hebd Seances Acad Sci 246:1626-1628, 1958; K. Tsuchikama, Z. An., Antibody-drug conjugates: recent advances in conjugation and linker chemistries, Protein & Cell, published online 14 Oct. 2016. DOI 10.1007/s13238-016-0323-0). This was revived in the 1970's when researchers became interested in the possibility of coupling various drugs to antibodies targeted to sites of optimal drug delivery (R. Levy, et al., The Specific Cytotoxic Effects of Daunomycin Conjugated to Antitumor Antibodies, Cancer Research, 35, 1182-1186, 1975; E. Hurwitz, et al., The effect in vivo of chemotherapeutic drug-antibody conjugates in two murine experimental tumor systems, International J. of Cancer, 21, 747-755, 1978). Hurwitz et al. noted that effective drug to antibody linking was crucial, and that polymers may play a significant role as linkers, and that polymer modified drugs if released from such conjugates may still be effective.

The above early antibody-drug-conjugate (ADC) studies were limited due to the inability to only obtain highly targeted antibodies from some patients. In 1986, the first monoclonal antibody biopharmaceutical product Orthoclone OKT3^{®} was approved. It was a mouse IgG2a anti-CD3 that was used to treat human organ transplant rejection and marketed for over twenty-five years. Its success led to the more than 80 mAb-related approved biopharmaceuticals currently approved (see below) and illustrates some key features of mAb biopharms. Firstly, they are well tolerated with even some early nonhuman mAbs being functional. Secondly, they had reasonably long circulation life due to their size precluding them from being filtered through the kidneys and their Fc regions allowing them to be "shunted" through (and not removed by) the liver. This last point is one reason why mAbs have not previously been considered as targets for PEGylation, and also why recombinant addition of Fc regions to (approximately ten) non-mAb drugs has also been a successful half-life extension method (van Witteloostuijn et al).

The advent of highly target specific, and well tolerated, monoclonal antibodies (mAbs), and mAb-related substances such as mAb fragments (Fabs), made such targeting possible. The drug substances of interest, which are highly toxic substances and lethal if delivered systemically, must be used in small, directed doses to kill cancer or other pathogenic cells. As such, they need to be coupled to antibodies in a manner that allows for mAb targeting function and controlled release of the drug at the site of binding. This can be effected via direct antibody-drug conjugation - typically via a short organic linker molecule, or indirectly via use of a polymer which sterically allows the antibody drug conjugate to bind effectively to target and then be released (A. Beck et al, Strategies and challenges for the next generation of antibody-drug conjugates, Nature Reviews, Drug Discovery, 16, 315-337, 2016). Presence of such (linking) polymers in the conjugate is expected to favorably enhance the pharmacokinetic and patient tolerance (i.e., immune stimulating) properties of the antibody drug conjugates, as well as their ease of formulation, reduction of aggregate formation, reduction of enzymatic or other degradation (C. Fee and J. M. Van Alstine, PEG-proteins: Reaction engineering and separation issues, Chemical Engineering Science, 61:924-934, 2006; S.B. van Witteloostuijn et al, Half-life extension of biopharmaceuticals using chemical methods: Alternatives to PEGylation, ChemMedChem Reviews, 11:2474-2495, 2016).

Perhaps of equal importance is the fact that many drugs of interest for ADC incorporation are of small molecular weight (MW) compared to that of an antibody (150,000 Daltons) or even a relatively small antibody fragment (e.g. 25,000 Daltons) as such it can be desired to be able to bind more than one (often relatively hydrophobic) drug molecules per ADC and hydrophilic polymeric linkers may provide a ready means to effect such enhanced loading of an ADC without negatively affecting the targeting properties of the antibody via multiple covalent modifications, or the ADC solubility. In the case of where ADCs are formed using hydrophilic polymer linkers it is not only controlled release of the drug that may be desired. Once the antibody has bound to the target and its drug payload has been released, release of the antibody from the polymer may also be desired so as to aid its rapid metabolic breakdown.

To date, four ADCs have been approved for different types of cancer treatment. Mylotarg^{®} (Pfizer/Wyeth) was approved in 2001. Adcentris^{®} (Seattle Genentics/Millenium/Takeda) was approved in 2011, Kadcyla^{®} (Genentech/Roche) in 2013 and Besponsa^{®} (Pfizer/Wyeth) in 2017. Kadcyla^{®} uses an already US Food and Drug Administration (FDA) approved mAb (Herceptin^{®}) as a targeting agent. Adcentris^{®} uses a polypeptide linker that is cleaved intracellularly by a cathepsin enzyme (there are several types) to release a "statin" cytotoxic drug. Kadcyla^{®}'s cell growth inhibitor drug (mertansine) can function without release.

At present, there are hundreds of drugs that physicians would like to specifically target to various body sites. In addition, there are approximately 80 US FDA approved monoclonal antibody-based medicines with perhaps 600 in late stage (Phase III) trials having demonstrated good patient tolerance and target binding. (W. R. Strohl, Current progress in innovative engineered antibodies, Protein Cell, 9:86-120, 2018). All of these mAbs represent potential targeting agents to be used in antibody drug conjugates.

Although some ADC delivered drugs may function without release, there are several advantages to ADCs based on cleavable linkers. One is that cleavable (releasable) linkers can be tailored to be released under extracellular or intracellular conditions. The latter allows the killing of target cells while the former can eliminate nearby cells, which also may be pathogenic (see Beck review noted above).

Given the above, there is much interest in novel linkers and linking chemistries. This is evidenced in a number of reviews (e.g. J. R. McCombs and S. C. Owen, Antibody Drug Conjugates: Design and selection of linker, payload and conjugation chemistry, The AAPS Journal, 17: 339-351, 2015) and research publications). Axup et al. presented synthesis of site-specific antibody-drug conjugates using unnatural amino acids (Proceedings of the National Academy of Sciences. 109 (40): 16101-16106, 2012). R. P. Lyon et al. presented on self-hydrolyzing maleimide linkages (Bioconjugate Chem. 32 (10): 1059-1062, 2014) with S. Kolodych et al. presenting on novel amine-to-thiol coupling reagents *(*Bioconjugate Chem. 26(2): 197-200, 2015). US Patent No. 7,745,394 assigned to Seattle Genetics covers specific cathepsin susceptible "val-cit-PAB" linkages while US Patent No. 5,208,020 covers exemplary maleimide linkers. As another example, US Patent No. 9,872,924 relates to a specific organic hydrophilic linker while WO 2017/031034 refers to a linker capable of covalently coupling one or more free-thiols of an antibody.

As noted above, in 1978 it was recognized that effectively linking cytoxic payloads to antibodies was critical for antibody-drug-conjugate (ADC) function (Hurwitz et al.). Over forty years later it is still recognized as crucial for mAb based ADCs (L. Ducry and B. Stump, Antibody-Drug Conjugates: Linking Cytotoxic Payloads to Monoclonal Antibodies, Bioconjugate Chem., 21: 5-13, 2010; Jessica R. McCombs and Shawn C. Owen. Antibody Drug Conjugates: Design and Selection of Linker, Payload and Conjugation Chemistry, The AAPS Journal, 17: 339-351, 2015; N. Jain, et al., Current ADC Linker Chemistry, Pharm. Res., Published online 11 March 2015, DOI 10.1007/s11095-015-1657-7).

Releasable (cleavable) linkers have recently been reviewed in Jain et al. and McCombs et al. Jain et al. noted that there are two families of linkers, non-cleavable and cleavable, with the latter related to "selective release of the cytotoxin from the ADC. There are three commonly used mechanisms: 1) protease-sensitivity, 2) pH-sensitivity, and 3) glutathione-sensitivity." With pH-sensitivity taking advantage of the lower pH (e.g. pH 5-6) in the endosomal and lysosomal (cancer cell) compartments as compared to the cytosol (pH 7.4), while glutathione sensitivity takes advantage of relatively higher intracellular concentrations of glutathione. More recently, Lyon et al. noted that "more hydrophilic auristatin ADCs obtained through hydrophilic linker design or attachment of polyethylene glycol (PEG) chains ("PEGylation") could improve efficacy" (R. P. Lyon, T. D. Bovee, S. O. Doronina, et al. Reducing hydrophobicity of homogeneous antibody-drug conjugates improves pharmacokinetics and therapeutic index.

Several approaches to releasable polymer modification of biologically active molecules and formation of polymer linked ADC's have been described. Improved methods for designing, preparing, and using such releasable conjugates are provided herein.

For example, Table 1 provides a series of ADC combinations as provided herein.

**Table 1. ADC Combinations of Releasable and Non-Releasable Tethers***

| | **Antibody to Tether** | **Tether to Drug** | **Exemplary Characteristics** |
|---|---|---|---|
| 1 | NR | NR | Good *in vitro* delivery stability. NR nature of linkages may allow targeted drug (e.g., enzyme) to function locally for an extended period. |
| 2 | R1 | R2 | Can have R1 release before R2 to facilitate active drug delivery; for example, in the case of a polymer tethered drug. Can have R1 and R2 release under different conditions to facilitate, for |
| | | | example, release of a drug at a target with controlled released of the drug within cells. |
| 3 | NR | R | Tether stays with targeting agent and native drug is released. |
| 4 | R | NR | Tether stays with (and protects) drug, which in the case of an enzyme or binder may not be affected by the presence of the tether. |
| 5 | Single Direct NR Link | | Classic NR linkage incorporated into the compounds as described herein allows for reporter and other groups to be added. It also allows for release of native drug and for more efficient and less expensive pharmaceutical production. |
| 6 | Single Direct R Link | | Classic R linkage incorporated into the compounds as described herein allows for reporter and other groups to be added. It also allows for release of native drug and for more efficient and less expensive pharmaceutical production. |

| | | | |
|---|---|---|---|
| * Releasable (R) and Non-Releasable (R) linkers refer to controlled releasing linkages. Antibody represents the targeting molecule, which could be an antibody, antibody fragment, or other target affinity group. Tether represents the molecule tethering the drug to the targeting agent, which could be, for example, a polymer. The drug could be a variety of drugs, including enzymatic or other proteins, which could be tethered with one or more drugs using (polymer) tethers and one or more (polymer) tethers per targeting molecule. Details are provided herein, which also notes that the R and NR chemistry of the application can be used to directly bind drug to targeting agent, via R or NR linkages without an independent tether (see, e.g., rows 5 and 6 in the table). | | | |

### COMPOUNDS

Provided herein are compounds of Formula (I): 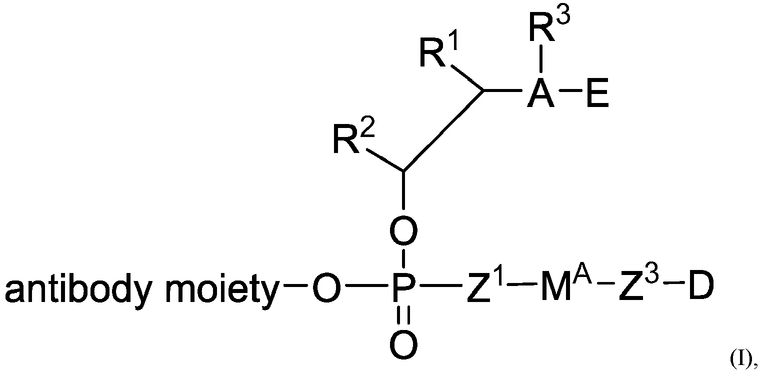 or a pharmaceutically acceptable salt thereof, wherein:
the antibody moiety is selected from:
antibody-L-(CH₂)_{q}- and antibody-L-(aliphatic moiety)-;
the aliphatic moiety is selected from a polymer, R^{P}, and a group selected from: polymer-L-(CH₂)ₘ- and polymer-L-(CH₂-CH₂-O)ₚ-(CH₂)ₘ-;
R^{P} is selected from optionally substituted C₁₋₆ alkyl, optionally substituted C₁₋₃ alkyl-O-(CH₂-CH₂-O)ₚ-(CH₂)ₘ-, and optionally substituted C₃₋₇ cycloalkyl;
each L is independently a linking group;
m, p, and q are each independently an integer from 1 to 10;
D is a residue of a cytotoxic or chemotherapeutic compound;
Z¹ is selected from O, S, and N(R^{N});
Z³ is selected from O and N(R^{N}), or Z³ is absent;
A is O or N, wherein when A is O then R³ is absent;
R^{N} is selected from H and optionally substituted C₁₋₆ alkyl;
R³ is selected from H and C₁₋₆ alkyl, or
R³ and R¹, together with A and the carbon atom to which R¹ is attached, form an optionally substituted 4 to 7 membered aliphatic heterocyclic ring; or
R³ and R², together with A, the carbon atom to which R¹ is attached, and the carbon atom to which R² is attached, form an optionally substituted 4 to 8 membered aliphatic heterocyclic ring;
M^{A} is a self-immolative group having any one of formulae (a)-(i): 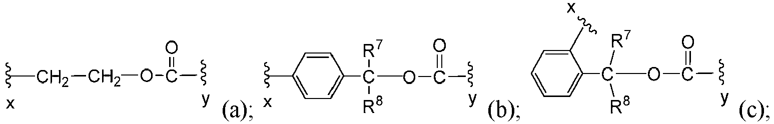 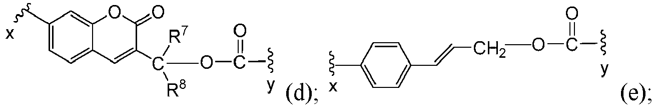 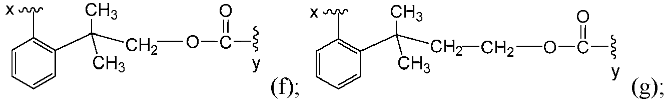 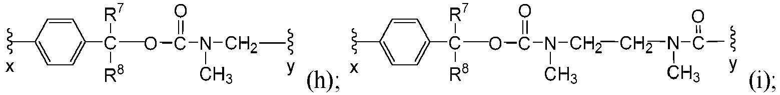
wherein x denotes a point of attachment to Z¹ and y denotes a point of attachment to z3.
R¹ and R² are independently selected from the group consisting of hydrogen, optionally substituted C₁₋₆ alkyl, optionally substituted C₆₋₁₀ aryl and optionally substituted 5- to 14-membered heteroaryl;
or R¹and R² are joined together with the carbon atoms to which they are attached to form an optionally substituted C₃₋₇ cycloalkyl ring, an optionally substituted 4 to 7 membered aliphatic heterocyclic ring, an optionally substituted C₆₋₁₀ aryl or an optionally substituted 5- to 14-membered heteroaryl;
or R¹ and R² are joined together to form a ribose ring system;
R⁷ and R⁸ are independently selected from H and C₁₋₆ alkyl; and
E is a cleavable moiety.

In some embodiments, this document provides a compound of Formula (III): 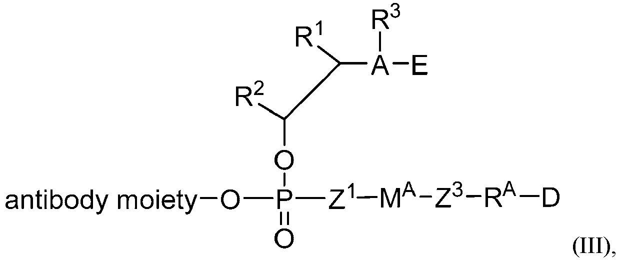 or a pharmaceutically acceptable salt thereof, wherein:
the antibody moiety is selected from:
antibody-L-(CH₂)_{q}- and antibody-L-(aliphatic moiety)-;
each aliphatic moiety is selected from a polymer, R^{P}, and a group selected from:
   polymer-L-(CH₂)ₘ- and polymer-L-(CH₂-CH₂-O)ₚ-(CH₂)ₘ-;
R^{P} is selected from optionally substituted C₁₋₆ alkyl, optionally substituted C₁₋₃ alkyl-O-(CH₂-CH₂-O)ₚ-(CH₂)ₘ-, and optionally substituted C₃₋₇ cycloalkyl;
each L is independently a linking group;
each m and p are independently an integer from 1 to 10;
D is a residue of a cytotoxic or chemotherapeutic compound;
R^{A} is selected from selected from a -polymer- and a group selected from: -polymer-L-(CH₂)ₘ- and -polymer-L-(CH₂-CH₂-O)ₚ-(CH₂)ₘ-;
Z¹ is selected from O, S, and N(R^{N});
Z³ is selected from O and N(R^{N}), or Z³ is absent;
A is O or N, wherein when A is O then R³ is absent;
R^{N} is selected from H and optionally substituted C₁₋₆ alkyl;
R³ is selected from H and C₁₋₆ alkyl, or
R³ and R¹, together with A and the carbon atom to which R¹ is attached, form an optionally substituted 4 to 7 membered aliphatic heterocyclic ring; or
R³ and R², together with A, the carbon atom to which R¹ is attached, and the carbon atom to which R² is attached, form an optionally substituted 4 to 8 membered aliphatic heterocyclic ring;
M^{A} is a self-immolative group having any one of formulae (a)-(i): 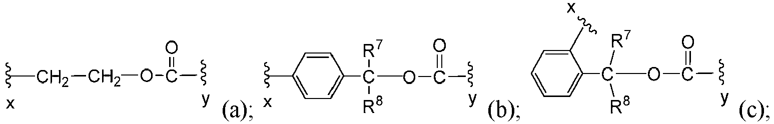 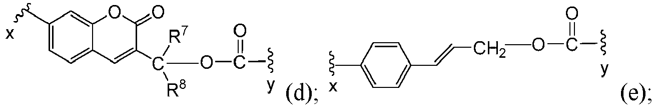 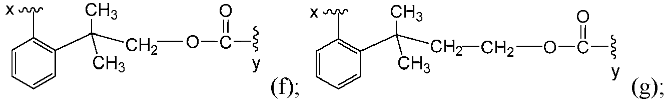 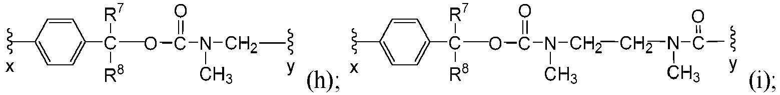
wherein x denotes a point of attachment to Z¹ and y denotes a point of attachment to z3.
R¹ and R² are independently selected from the group consisting of hydrogen, optionally substituted C₁₋₆ alkyl, optionally substituted C₆₋₁₀ aryl and optionally substituted 5- to 14-membered heteroaryl;
or R¹ and R² are joined together with the carbon atoms to which they are attached to form an optionally substituted C₃₋₇ cycloalkyl ring, an optionally substituted 4 to 7 membered aliphatic heterocyclic ring, an optionally substituted C₆₋₁₀ aryl or an optionally substituted 5- to 14-membered heteroaryl;
or R¹ and R² are joined together to form a ribose ring system;
R⁷ and R⁸ are independently selected from H and C₁₋₆ alkyl; and
E is a cleavable moiety.

In some embodiments of Formula (I) or Formula (III), the antibody moiety is comprises an antibody or an antibody fragment or similarly reactive targeting entity. Such targeting entities include, for example, lectins, cell surface enzyme inhibitors, small molecular weight polypeptides with specific affinities, and aptamers (e.g., L. Gold, SELEX: How it happened, where it will go, J Mol Evol 81:140-143, 2015). It should be noted that all such targeting entities are reactive under similar chemical conditions and using similar chemical reactions as described herein (G. T. Hermanson, Bioconjugation Techniques, 3rd Edn., Academic Press, New York, 20013). In some embodiments, the antibody moiety comprises an antibody (e.g., a monoclonal antibody) or an antibody fragment as described herein, wherein the antibody or antibody fragment is linked via a linking group as described herein. In some embodiments, the antibody moiety is selected from antibody-L-(CH₂)_{q}- and antibody-L-(aliphatic moiety)-. In some embodiments, more than one antibody moiety is present. In such embodiments, the antibody moieties may be the same or different.

In some embodiments of Formula (I) or Formula (III), wherein the aliphatic moiety is selected from a polymer, R^{P}, and a group of formula -polymer-L-(CH₂)ₘ-; R^{P} is selected from optionally substituted C₁₋₆ alkylene and optionally substituted C₃₋₇ cycloalkylene; and m is an integer from 1 to 10.

In some embodiments of Formula (I) or Formula (III), the aliphatic moiety is a group of formula: -polymer-L-(CH₂)ₘ-. In some aspects of these embodiments, L is a linking group comprising a heterocycloakylene or a heteroarylene. For example, L is a linking group comprising a succinimide or a triazole. In some embodiments, L is a linking group of any one of the following formulae: 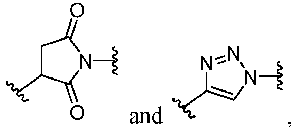 wherein 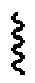 indicates a point of attachment of the linking group to the polymer or to the CH₂ group.

In some embodiments, the linking group L is a linking group of formulae: 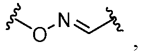 wherein 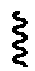 indicates a point of attachment of the linking group to the polymer or to the CH₂ group.

In some embodiments, the linking group L is a linking group of formulae: 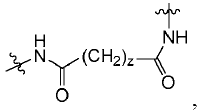 wherein 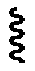 indicates a point of attachment of the linking group to the polymer or to the CH₂ group, and z is an integer from 1 to 10 (e.g., 4).

In some embodiments of Formula (I) or or Formula (III), the antibody moiety is any one of the following formulae:

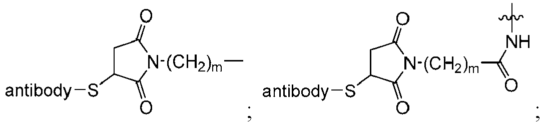

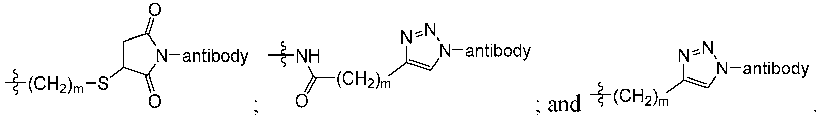

In some embodiments, L comprises a group of formula (L¹): 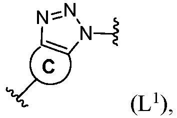 wherein ring C is selected from the group consisting of an optionally substituted C₈₋₁₆ cycloalkyl and an optionally substituted 8-16-membered heterocycloalkyl, and 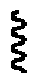 indicates a point of attachment of the linking group to the polymer or to the CH₂ group. In some aspects of these embodiments, C₈₋₁₆ cycloalkyl is a cyclooctenyl which is optionally fused with 1 or 2 benzene rings. In other aspects of these embodiments, C₈₋₁₆ cycloalkyl is a cyclooctenyl which is optionally substituted with 1, 2 or 3 substituents selected from halogen, OH, C₁₋₃ alkyl and C₁₋₃ alkoxy. For example, cyclooctenyl may be substituted with 1 or 2 fluoro, or with 1 or 2 methoxy groups.

In some embodiments, the group of formula (L¹) is selected from any one of the following formulae:

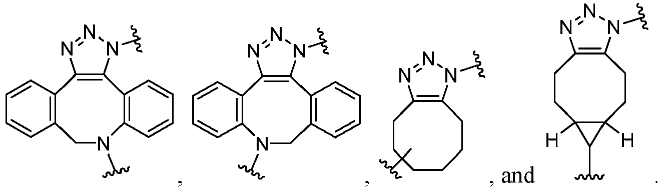

In some embodiments, the group of formula (L¹) is

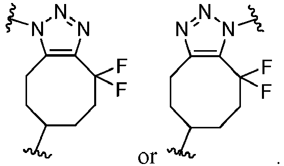

In some embodiments, the group of formula (L¹) is

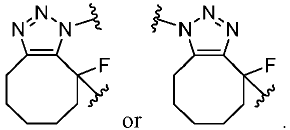

In some embodiments, the group of formula (L¹) is

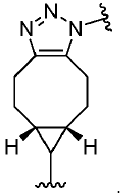

In some embodiments, the group of formula (L¹) is

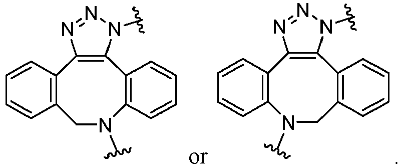

In some embodiments, the group of formula (L¹) is

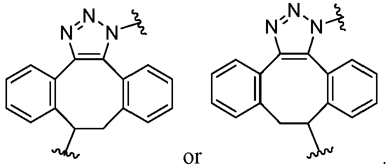

In some embodiments, the group of formula (L¹) is

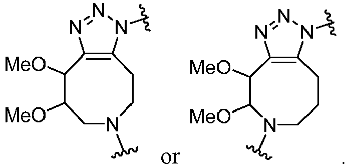

In some embodiments, m is an integer from 1 to 6. For example, m is 1, 2, 3, 4, 5, or 6. In some embodiments, m is an integer from 1 to 4.

In some embodiments, p is an integer from 1 to 6. For example, p is 1, 2, 3, 4, 5, or 6. In some embodiments, p is an integer from 1 to 4.

In some embodiments, q is an integer from 1 to 6. For example, q is 1, 2, 3, 4, 5, or 6. In some embodiments, q is an integer from 1 to 4.

In some embodiments of Formula (I), or Formula (III), the aliphatic moiety is a polymer (e.g., any one of the polymers described herein). The polymer in the aliphatic moiety can be selected from poly(alkylene glycol), poly(oxyethylated polyol), poly(olefinic alcohol), poly(α-hydroxy acid), poly(vinyl alcohol), polyoxazoline, or copolymers thereof. In some embodiments, the polymer in the aliphatic moiety is polyethylene glycol. For example, the aliphatic moiety comprises a linear polyethylene glycol or a branched polyethylene glycol.

In some embodiments of Formula (I) or Formula (III), the aliphatic moiety is R^{P}. In some embodiments, R^{P} is an optionally substituted C₁₋₆ alkylene or an optionally substituted C₃₋₇ cycloalkylene. For example, R^{P} is selected from C₁₋₆ alkylene, C₁₋₆ cyanoalkylene and C₃₋₇ cycloalkylene. When the aliphatic moiety is R^{P}, the aliphatic moiety can be C₁₋₆ alkylene (e.g., methylene, ethylene, propylene, isopropylene, n-butylene, isobutylene, tert-butylene, amylene or hexylene). For example, the aliphatic moiety can be cyanoethylene. In some embodiments, the aliphatic moiety can be 2-cyanoethylene. In other embodiments, the aliphatic moiety is C₃₋₇ cycloalkylene (e.g., cyclopropylene, cyclobutylene, cyclopentylene or cyclohexylene). In some embodiments, R^{P} is isopropylene. In some embodiments, R^{P} is cyanoethylene.

In some embodiments of Formula (I), or Formula (III), Z¹ is selected from O, S, and N(R^{N}). In some embodiments, Z¹ is O. In some embodiments, Z¹ is NH. In some embodiments, Z¹ is N(C₁₋₆ alkyl). In some embodiments, Z¹ is S.

In some embodiments of Formula (I), or Formula (III), Z³ is selected from O and N(R^{N}). In some embodiments, Z³ is absent. In some embodiments, Z³ is O. In some embodiments, Z³ is NH. In some embodiments, Z³ is N(C₁₋₆ alkyl).

In some embodiments of Formula (I), or Formula (III), Z¹ is O and Z³ is NH. In some embodiments, Z¹ is NH and Z³ is O. In some embodiments, Z¹ is O and Z³ is absent. In some embodiments, Z¹ is O and Z³ is O. In some embodiments, Z¹ is NH and Z³ is NH. In some embodiments, Z¹ is NH and Z³ is absent. In some embodiments, Z¹ is S and Z³ is O. In some embodiments, Z¹ is S and Z³ is NH. On some embodiments, Z¹ is S and Z³ is absent.

In some embodiments of Formula (I) or Formula (III), M^{A} is a diradical characterized in that, alone or together with Z¹, upon nucleophilic attack on the phosphorus atom in Formula (I), or Formula (III), M^{A} (along with Z³-D or Z³-R^{A}-D) creates a better leaving group than any one of the antibody moieties (Formula (I) or Formula (III)) described herein. For example, as shown in Scheme 2 below, upon nucleophilic attack on the phosphorus atom by the 2' hydroxyl group of the ribose unit, both 2-hydroxy propionate and the antibody fragment create equally good leaving groups and the nucleophilic substitution reaction is non-selective.

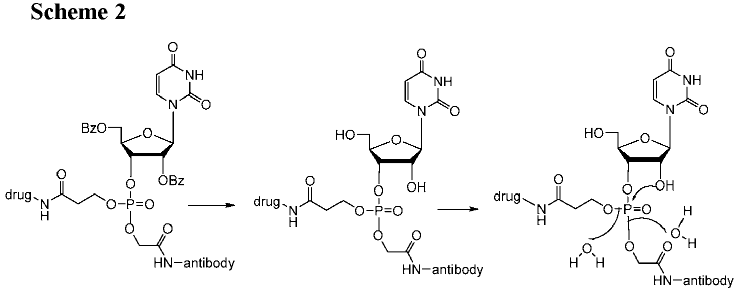

In contrast, in some embodiments of Formula (I), for example, as described herein, a group comprising the -Z¹-M^{A}- fragment is a better leaving group than the antibody moeity, such that under similar conditions as compared to Scheme 2, upon nucleophilic attack on the phosphorus atom by the 2' hydroxyl group of the ribose unit, the fragment containing the antibody moiety remains covalently attached to the phosphorus atom, while the group comprising the -Z¹-M^{A}- fragment is selectively cleaved.

In some embodiments of Formula (I) or Formula (III), M^{A} is a diradical, characterized in that, alone or together with Z¹, upon nucleophilic attack on the phosphorus atom in Formula (I), or Formula (III), M^{A} (along with Z³-D or Z³-R^{A}-D) creates a better leaving group than antibody moiety (Formula (I) or Formula (III)). In some embodiments, the conjugate acid of the Z¹-M^{A}-Z³-D moiety, represented by the formula HZ¹-M^{A}-Z³-D, has a lower pKa value than the conjugate acid of the antibody moiety or the aliphatic moiety that is conjugated to a compound of Formula (I). In some embodiments, the group HZ¹-M^{A}-Z³D has a lower pKa value than a polyethylene glycol or an alcohol. In some aspects of these embodiments, Z¹ is oxygen and M^{A} comprises an aromatic moiety (e.g., M^{A} comprises a phenylene). In some embodiments, Z¹ is nitrogen and M^{A} comprises an aromatic moiety (e.g., M^{A} comprises a phenylene), and the conjugated base of the comound of formula HZ¹-M^{A}-Z³-D is a better leaving group than any one of the antibody moieties (Formula (I)) described herein due to delocalization of the lone pair of electrons on the nitrogen atom of Z¹ into the aromatic ring of M^{A}.

In some embodiments, the compound of Formula (I) or Formula (III) comprises a single immolative functionality. In some embodiments of Formula (I), or Formula (III), M^{A} is a self-immolative group. In some embodiments, M^{A} is any one of the self-immolative groups described, for example, in Alouane, A. et al., "Self-immolative spacers: kinetic aspects, structure-property relationships, and applications", Angew. Chem. Int. Ed., 2015, 54, 7492 - 7509. In other embodiments, M^{A} is any one of the self-immolative groups described, for example, in Kolakowski, R. et al., "The Methylene Alkoxy Carbamate Self-immolative Unit: Utilization for the Targeted Delivery of Alcohol-Containing Payloads with Antibody-Drug Conjugates", Angew. Chem. Int. Ed., 2016, 55 (28), 7948-7951.

In some embodiments, M^{A} is a self-immolative group having any one of formulae (a)-(i): 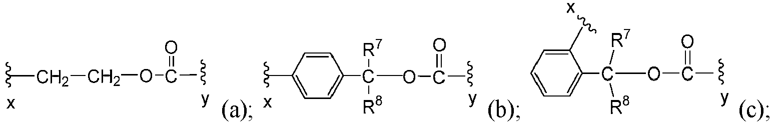 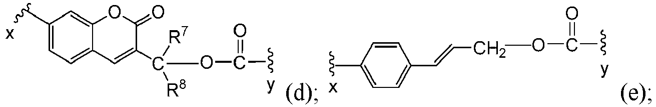 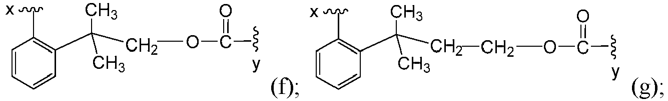 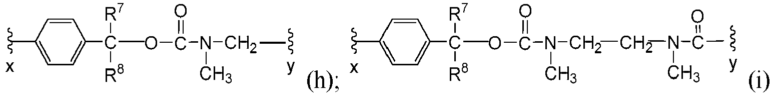 wherein x denotes a point of attachment to Z¹ and y denotes a point of attachment to Z3.

In some embodiments of Formula (I) or Formula (III), the self-immolative group is characterized in that the cleavage of the P-Z¹ bond generates a cascade of decomposition reactions (e.g., a hydrolysis cascade as described herein) ultimately leading to:
i) release of the conjugate base of the compound HZ³-D when Z³ is present; or
ii) release of the conjugate base of the compound HO-(C=O)-D when Z³ is absent.

In some embodiments, the conjugated base of the compound of formula HZ³-D is a moiety of formula ⁻Z³-D. In some embodiments, the conjugated base of the compound of formula HO-(C=O)-D is a moiety of formula ⁻O-(C=O)-D.

In some embodiments of Formula (I), or Formula (III), Z³ is present (e.g., Z³ is O or NH) and M^{A} is a self-immolative group. In some embodiments, the self-immolative group is characterized in that the cleavage of the P-Z¹ bond generates a cascade of decomposition reactions ultimately leading to the release of the conjugate base of the compound HZ³-D.

In some aspects of these embodiments, cleavage of the P-Z¹ bond results in the formation of Z¹=M^{A'}, CO₂ and the conjugate base of compound HZ³-D, where M^{A'} is a fragment of a self-immolative group (e.g. self-immolative group of any of formula (a)-(g)) which lacks a moiety of formula:

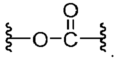

In one example, M^{A} is a self-immolative group of formula (b), and M^{A'} is a fragment of formula: 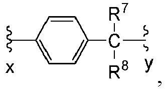 wherein x denotes a point of attachment to Z¹ and y denotes a point of attachment to Z3.

In one example, M^{A} is a self-immolative group of formula (d), and M^{A'} is a fragment of formula: 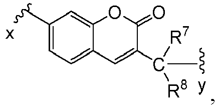 wherein x denotes a point of attachment to Z¹ and y denotes a point of attachment to Z3.

In some embodiments, in the compound Z¹=M^{A'}, the second bond between Z¹ and M^{A'} connects Z¹ and any one of atoms of the M^{A'} group. For instance, when M^{A} is a self-immolative group of formula (a), the second bond between Z¹ and M^{A'} connects Z¹ and the carbon atom of formula (a) that was in position β to Z¹ prior to the decomposition reaction:

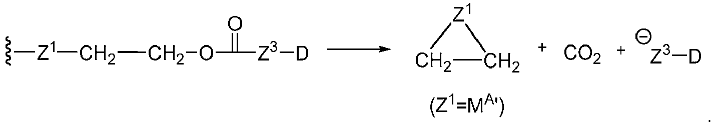

In another example of the compound Z¹=M^{A'}, when M^{A} is a self-immolative group of formula (b-1), the second bond between Z¹ and M^{A'} connects Z¹ and the carbon atom in M^{A}' to which Z¹ is attached, and the remaining bonds in M^{A'} are delocalized:

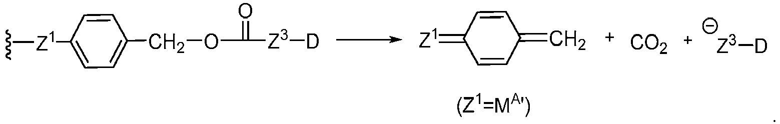

In other aspects of the above embodiments, cleavage of the P-Z¹ bond results in the formation of the conjugate base of the compound HZ³-D and a compound of formula:

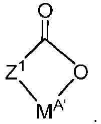

In some embodiments of Formula (I), or Formula (III), Z³ is absent, and M^{A} is a self-immolative group. In some embodiments, the self-immolative group is characterized in that the cleavage of the P-Z¹ bond generates a cascade of decomposition reactions ultimately leading to the release of the conjugare base of the compound HO-(C=O)-D. In some aspects of these embodiments, the cleavage of the P-Z¹ bond results in the formation of Z¹=M^{A'} (as described herein) and the conjugate base of the compound HO-(O=C)-D.

In some embodiments of Formula (I), or Formula (III), Z¹ is S and M^{A} is a self-immolative group of formula (a): 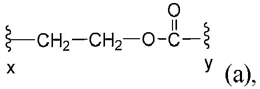 wherein x denotes a point of attachment to Z¹ and y denotes a point of attachment to z3.

In some embodiments of Formula (I), or Formula (III), the self-immolative group of formula (b) has the formula (b-1): 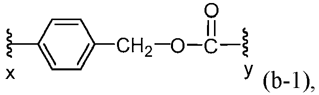 wherein x denotes a point of attachment to Z¹ and y denotes a point of attachment to z3.

In some embodiments of Formula (I) or Formula (III), the self-immolative group of formula (b) has the formula (b-2): 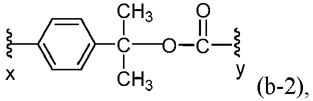 wherein x denotes a point of attachment to Z¹ and y denotes a point of attachment to Z3.

In some embodiments of Formula (I), or Formula (III), the self-immolative group of formula (c) has the formula (c-1): 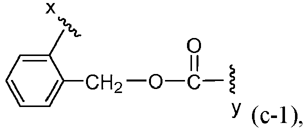 wherein x denotes a point of attachment to Z¹ and y denotes a point of attachment to z3.

In some embodiments of Formula (I), or Formula (III), the self-immolative group of formula (d) has the formula (d-1): 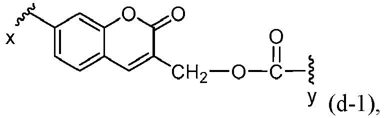 wherein x denotes a point of attachment to Z¹ and y denotes a point of attachment to z3.

In some embodiments of Formula (I), or Formula (III), Z¹ is O or NH and M^{A} is a self-immolative group of any one of formula (b)-(i). In some embodiments, Z¹ is O or NH, Z³ is absent, and M^{A} is a self-immolative group of any one of formula (b)-(i).

In some embodiments of Formula (I), or Formula (III), Z¹ is O or NH, Z³ is absent, and M^{A} is a self-immolative group of formula (b), wherein R⁷ and R⁸ are each C₁₋₆ alkyl, or a formula (b-2).

In some embodiments of Formula (I), or Formula (III), Z¹ is O, Z³ is O, and M^{A} is a self-immolative group of formula (h-1): 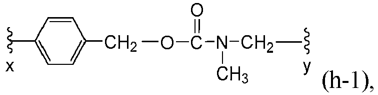 wherein x denotes a point of attachment to Z¹ and y denotes a point of attachment to z3.

In some embodiments of Formula (I), or Formula (III), Z¹ is O, Z³ is O, and M^{A} is a self-immolative group of formula (i-1): 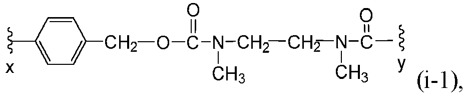 wherein x denotes a point of attachment to Z¹ and y denotes a point of attachment to z3.

In some embodiments of Formula (I), or Formula (III), Z¹ is NH, Z³ is O, and M^{A} is a self-immolative group of formula (h-1). In some embodiments, wherein Z¹ is NH, Z³ is O, and M^{A} is a self-immolative group of formula (i-1).

In some embodiments of Formula (I), or Formula (III), M^{A} is a stable diradical. For example, the stable diradical is not a self-immolative group (e.g. upon nucleophilic attack on the phosphorus atom in Formula (A), the stable diradical does not lead to the cleavage of the Z¹-M^{A} bond, or the M^{A}-Z³ bond). In some embodiments, the stable diradical is characterized in that the cleavage of the P-Z¹ bond generates a conjugate base of the compound of formula HZ¹-M^{A}-Z³-D, which is stable and does not undergo the decomposition reaction.

In some embodiments, M^{A} is a stable diradical having any one of formulae (j)-(l): 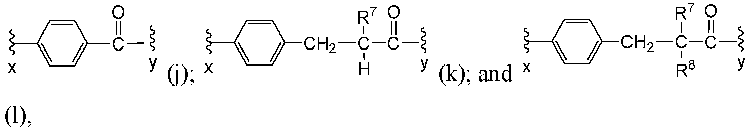 wherein x denotes a point of attachment to Z¹ and y denotes a point of attachment to Z³. In some aspects of these embodiments, Z¹ and Z³ are independently O or NH (e.g., Z¹ is O and Z³ is NH). In some embodiments, when M^{A} is a stable diradical of formula (j), Z¹ is O. In some embodiments, when M^{A} is a stable diradical of formula (j), Z¹ is NH.

In some embodiments of Formula (I), or Formula (III), M^{A} is a stable diradical having the formula (m): 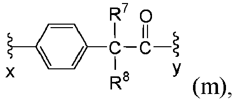 wherein x denotes a point of attachment to Z¹ and y denotes a point of attachment to Z³. In some aspects of these embodiments, Z¹ and Z³ are independently O or NH (e.g., Z¹ is O and Z³ is NH). In some embodiments, when M^{A} is a stable diradical of formula (m), Z¹ is O and R⁷ and R⁸ are each hydrogen.

In some embodiments of Formula (I), or Formula (III), R⁷ and R⁸ are independently selected from H, C₁₋₆ alkyl, amino, (C₁₋₆ alkyl)amino, di-(C₁₋₆ alkyl)amino, acylamino, and a protected amino group (e.g., the protecting group for the amino group may be selected from any one of the amino-protecting groups described, for example, in Greene and Wuts, Protective Groups in Organic Synthesis, 3rd Ed., John Wiley & Sons, New York, N.Y., 1999). In some embodiments, R⁷ and R⁸ are independently selected from H, methyl, amino, and acylamino. In some embodiments of Formula (I), or Formula (III), R⁷ and R⁸ are independently selected from H and C₁₋₆ alkyl. In some embodiments, R⁷ and R⁸ are independently selected from H and methyl.

In some embodiments of Formula (I), or Formula (III), R⁷ is selected from C₁₋₆ alkyl, C₃₋₇ cycloalkyl, amino, acylamino, and a protected amino group, and R⁸ is H. In some embodiments, R⁸ is selected from C₁₋₆ alkyl, C₃₋₇ cycloalkyl, amino, acylamino, and a protected amino group, and R⁷ is H.

In some embodiments of Formula (I), or Formula (III), R⁸ is H and R⁷ is C₁₋₆ alkyl (e.g., methyl, ethyl, propyl, isopropyl or tert-butyl). In some embodiments, R⁷ is H and R⁸ is C₁₋₆ alkyl (e.g., methyl, ethyl, propyl, isopropyl or tert-butyl). In some embodiments, R⁷ and R⁸ are both H. In some embodiments, R⁷ and R⁸ are both C₁₋₆ alkyl. In another example, R⁷ and R⁸ are both methyl. In another example, R⁷ is methyl, and R⁸ is ethyl. In some embodiments, R⁷ and R⁸ are both C₃₋₇ cycloalkyl (e.g., cyclopropyl or cyclobutyl).

In some embodiments of Formula (I), or Formula (III), R⁷ and R⁸ are each independently H or acylamino (e.g., acetylamino, propionylamino, or butyramino). In some embodiments, R⁷ is amino or acetylamino. In some embodiments, R⁸ is amino or acetylamino. In some embodiments, R⁷ is acetylamino and R⁸ is H. In some embodiments, R⁷ is H. In some embodiments, R⁸ is H.

In some embodiments of Formula (I), or Formula (III), M^{A} is a self-immolative group of any one of formulae (b), (c) or (d), and R⁷ and R⁸ are both C₁₋₆ alkyl (e.g., methyl, ethyl, propyl, isopropyl or tert-butyl). In some embodiments, M^{A} is a stable diradical of formula (k) or formula (l), and R⁷ and R⁸ are independently selected from H or acylamino (e.g., acetylamino, propionylamino, or butyramino).

In some embodiments of Formula (I), or Formula (III), the stable diradical of formula (k) has the formula (k-1): 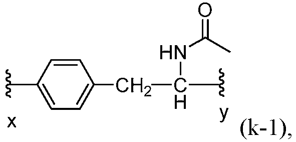 wherein x denotes a point of attachment to Z¹ and y denotes a point of attachment to Z3.

In some embodiments of Formula (I), or Formula (III), R¹ and R² are independently selected from the group consisting of hydrogen, optionally substituted C₁₋₆ alkyl, optionally substituted C₆₋₁₀ aryl and optionally substituted 5- to 14-membered heteroaryl. In some embodiments, R¹ and R² are each hydrogen. In some embodiments, R¹ and R² together form a chemical bond (i.e., a carbon-carbon double bond is formed between the carbon to which R¹ is attached and the carbon atom to which R² is attached).

In some embodiments of Formula (I), or Formula (III), R¹and R² are joined together with the carbon atoms to which they are attached to form an optionally substituted C₃₋₇ cycloalkyl ring, an optionally substituted 4- to 7-membered aliphatic heterocyclic ring, an optionally substituted C₆₋₁₀ aryl or an optionally substituted 5- to 14-membered heteroaryl. In some embodiments, R¹ and R² together form a C₃₋₇ cycloalkyl ring (e.g., cyclopropyl, cyclobutyl, cyclopentyl and cyclohexyl). In some embodiments, R¹ and R² together form a 4- to 7-membered aliphatic heterocyclic ring (e.g., pyrrolidine, piperidine, tetrahydrofuran and tetrahydropyran).

In some embodiments of Formula (I), or Formula (III), R¹ and R² are joined together to form a ribose ring system (e.g., adenosine, guanosine, 5-methyluridine, uridine, 5-methylcytidine, cytidine, inosine, xanthosine, and wybutosine, each of which is substituted as described herein). In some embodiments, the ribonucleoside is uridine. In some embodiments, R¹ and R² together form a ribose ring system of formula: 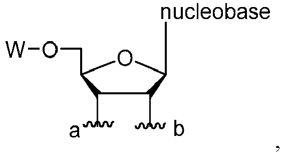 wherein either a denotes a point of attachment to O and b denotes a point of attachment to A, or a denotes a point of attachment to A and b denotes a point of attachment to O, and wherein W is selected from the group consisting of H, an acyl group and a protecting group (e.g., a protecting group other than acyl). Without being bound by any theory, it is believed that a lyxofuranose-based nucleotide has similar reactivity when compared to the ribofuranose analogues described herein.

Similarly, in one example, it is possible to assume that the 5'-OH can be involved in the intramolecular attack on a phosphotriester even if it is located 3 carbons away. This is more demanding that the typical 2-carbon distance interaction described herein, but this lyxo-isomer could facilitate the reaction by reversing the orientation of the 3' OH. Thus, 2'-deoxy,3'-xylo nucleosides are a suitable alternative to a ribonucleotide ribose scaffold described above for use in the cleavable unit.

In some embodiments, the ribonucleoside is uridine. In some embodiments, R¹ and R² together form a ribose ring system of formula: 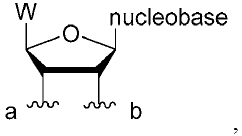 wherein either a denotes a point of attachment to O and b denotes a point of attachment to A, or a denotes a point of attachment to A and b denotes a point of attachment to O, and wherein W is selected from the group consisting of H and an antibody moiety as described herein. Without being bound by any theory, it is believed that a lyxofuranose-based nucleotide has similar reactivity when compared to the ribofuranose analogues described herein.

In some embodiments of Formula (I), or Formula (III), the nucleobase is selected from the group consisting of adenine, cytosine, guanine, thymine, uracil, and other natural and non-natural nucleobases.

In some embodiments of Formula (I), or Formula (III), the nucleobase is uracil. In some embodiments, the nucleobase is selected from the group consisting of adenine, cytosine, guanine, thymine and uracil. In some embodiments, the nucleobase is selected from the group consisting of 5-methylcytosine, pseudouridine, dihydrouridine, inosine, 7-methylguanosine, hypoxanthine and xanthine.

In some embodiments of Formula (I), or Formula (III), the nucleobase comprises a fluorescent group (e.g., a traditional fluorophore). In some embodiments, the nucleobase is a fluorescent analog of adenine, cytosine, guanine, thymine or uracil.

In some embodiments of Formula (I), or Formula (III), R¹ and R² together form a ribose ring system of any one of the following formulae: 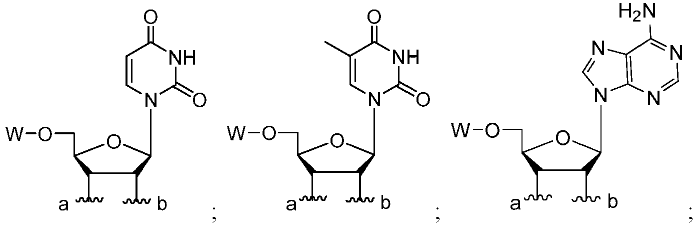 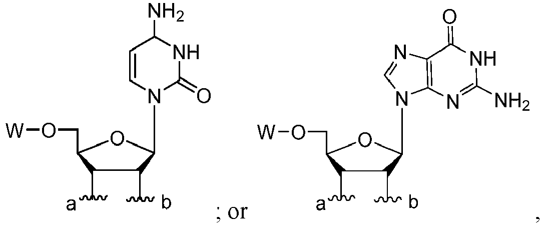 wherein either a denotes a point of attachment to O and b denotes a point of attachment to A, or a denotes a point of attachment to A and b denotes a point of attachment to O, and wherein W is selected from the group consisting of H, an acyl group and a protecting group (e.g., a protecting group other than acyl), or an antibody moiety as described herein.

In some embodiments of Formula (I), or Formula (III), R¹ and R² together form a ribose ring system of any one of the following formulae: 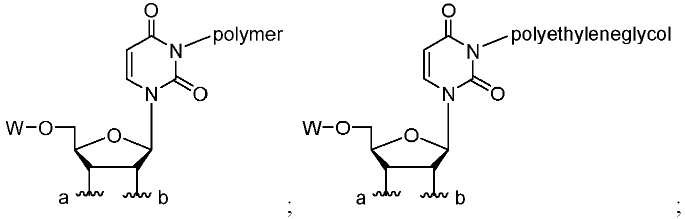 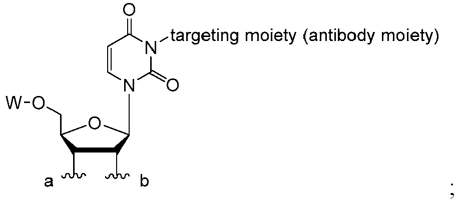 or 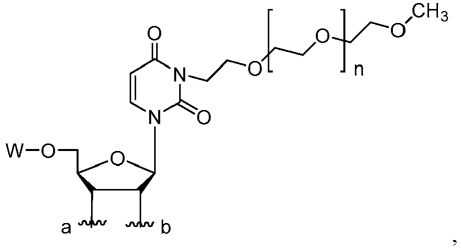 wherein either a denotes a point of attachment to O and b denotes a point of attachment to A, or a denotes a point of attachment to A and b denotes a point of attachment to O, and wherein W is selected from the group consisting of H, an acyl group, a protecting group (e.g., a protecting group other than acyl), or an antibody moiety as described herein.

In some embodiments of Formula (I), or Formula (III), R¹ and R² together form a ribose ring system of the following formula: 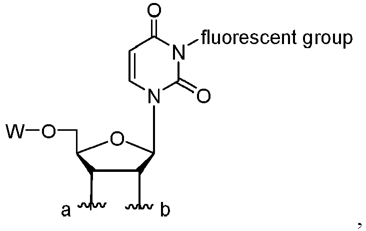 wherein either a denotes a point of attachment to O and b denotes a point of attachment to A, or a denotes a point of attachment to A and b denotes a point of attachment to O, and wherein W is selected from the group consisting of H, an acyl group, a protecting group (e.g., a protecting group other than acyl), and an antibody moiety as described herein.

In some embodiments of Formula (I), or Formula (III), R¹ and R² together form a ribose ring system having a nucleobase containing exocyclic amino group used as a point of attachment to a fluorophore or a linking molecule connected to a fluorescent group or an antibody moiety, as exemplified in the following formula:

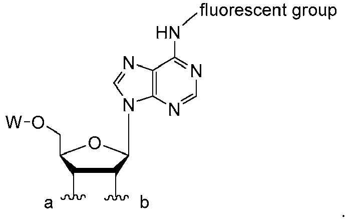

In some aspects of the above embodiments, the aliphatic moiety is R^{P}. For example, R^{P} is C₁₋₆ alkyl (e.g., ethyl or isopropyl). In another example, R^{P} is cyanoethyl. In other aspects of the above embodiments, the aliphatic moiety is a polymer (e.g., polyethylene glycol). In other aspects of the above embodiments, the aliphatic moiety is a group of formula: polymer-L-(CH₂)ₘ-.

In some embodiments of Formula (I), or Formula (III), W is a protecting group. For example, W may be a hydroxyl protecting group such as methoxymethyl ether (MOM), benzyloxymethyl ether (BOM), benzyl ether, p- methoxybenzyl ether (PMB), trityl ether, silyl ether (e.g., TMS, TIPS), or any of the hydroxyl protecting groups described, for example, in P. G. M. Wuts and T. W. Greene, Protective Groups in Organic Synthesis, 4th Ed., Wiley & Sons, Inc., New York (2006). In some embodiments, W is an alcohol protecting group selected from the group consisting of t-butyldimethylsilyl, diethylisopropylsilyl, triphenylsilyl, formate, methoxymethylcarbonate, *t*-butylcarbonate, 9-fluorenylmethylcarbonate, N-phenylcarbamate, 4,4'-dimethoxytrityl, monomethoxytrityl, trityl, and pixyl.

In some embodiments, W is hydrogen.

In some embodiments, W is an acyl group.

In some embodiments of Formula (I), or Formula (III), W is any one of the acyl groups described herein (e.g., W is an acyl group selected from formyl, acetyl, propionyl, acrylyl, pivaloyl, and benzoyl). In some embodiments, W is pivaloyl, adamantoyl carboxylate, or benzoyl. In some embodiments, W and E are the same (e.g., W and E are each an acyl group). In some embodiments, W is an acyl group and E is a cleavable group other than an acyl group. In some embodiments, an acyl group is hydrolyzable in the presence of any one of the numerous hydrolase enzymes (e.g., intracellular hydrolase enzymes) existing *in vivo.* In some embodiments, W is an antibody moiety as described herein. For example, an antibody moiety bound directly or indirectly through a linker (L) to the O linked to the ribose moiety. In some such embodiments, the antibody moiety is selected from an antibody, an antibody fragment, a group , a group showing affinity to a receptor present at the surface of the tumor cell (e.g., cyclopeptides, bicyclopeptides, aptamers, or a combination thereof), etc. and a group selected from:
antibody-L-(CH₂)_{q}- and antibody-L-(aliphatic moiety)-,
wherein L, aliphatic moiety, antibody, and q are as described herein.

In some embodimetns of or one of wherein one of R¹ or R² is substituted directly or indirectly through a linker (L) with an antibody moiety selected from an antibody, an antibody fragment, a group showing affinity to a receptor present at the surface of the tumor cell (e.g., cyclopeptides, bicyclopeptides, aptamers, or a combination thereof), etc. (see, e.g., O. J. Finn, Human Tumor Antigens Yesterday, Today, and Tomorrow, Cancer Immunological Res., 5: 2017), and a group selected from:
antibody-L-(CH₂)_{q}- and antibody-L-(aliphatic moiety)-;
wherein the R¹ or R² that is substituted with the antibody moiety is not H. In some embodiments, R¹ is substituted with an antibody moiety. In some embodiments, R² is substituted with an antibody moiety. In some embodiments, when R¹ is substituted with an antibody moiety as described herein, the antibody moiety is linked through the 5'position of the nucleoside. In some embodiments, when R² is substituted with an antibody moiety as described herein, the antibody is linked through the N3 position of the uracil. In some embodiments, the antibody moiety is linked to the R¹ or R² positions through a linker (L) as described herein. In some embodiments, the antibody moiety is linked to the R¹ or R² positions through a linker (L-(CH₂)ₘ) as described herein.

In some embodiments of Formula (I), or Formula (III), A is selected from O and N(R^{N}). In some embodiments, A is O. In some embodiments, A is N(R^{N}). In some embodiments, A is NH. In some embodiments, A is N(C₁₋₆ alkyl). In some embodiments, A is N(CH₃). In some embodiments, A is N(CH₂CH₃).

In some embodiments, when A is N(R^{N}), R^{N} and R¹, together with A and the carbon atom to which R¹ is attached, form an optionally substituted 4 to 7 membered aliphatic heterocyclic ring. In some aspects of these embodiments, the 4 to 7 membered aliphatic heterocyclic ring is selected from the group consisting of: 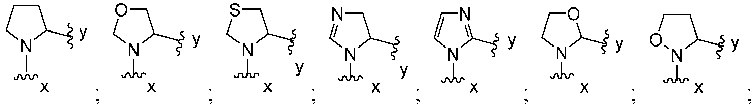 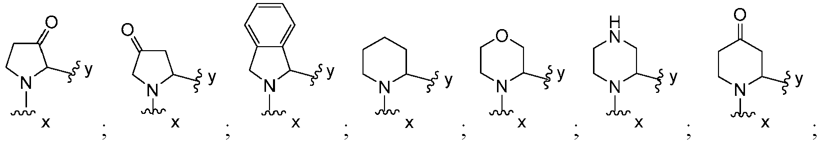 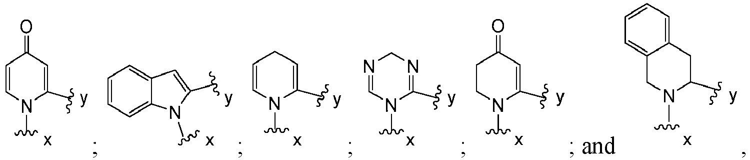 wherein x denotes a point of attachment to E, and y denotes a point of attachment to the carbon atom to which R¹ is attached.

In some embodiments, R^{N} and R², together with A, the carbon atom to which R¹ is attached, and the carbon atom to which R² is attached, form an optionally substituted 4 to 8 membered aliphatic heterocyclic ring.

In some embodiments, R³ is selected from H and C₁₋₆ alkyl, or
R³ and R¹, together with A and the carbon atom to which R¹ is attached, form an optionally substituted 4 to 7 membered aliphatic heterocyclic ring; or
R³ and R², together with A, the carbon atom to which R¹ is attached, and the carbon atom to which R² is attached, form an optionally substituted 4 to 8 membered aliphatic heterocyclic ring.

In some embodiments of Formula (I), or Formula (III), A is N. In other embodiments, A is O.

In some embodiments of Formula (I), or Formula (III), A is NR³.

In some embodiments of Formula (I), or Formula (III), when A is NR³, R³ and R¹, together with A and the carbon atom to which R¹ is attached, form an optionally substituted 4 to 7 membered aliphatic heterocyclic ring. In some aspects of these embodiments, the 4 to 7 membered aliphatic heterocyclic ring is selected from the group consisting of: 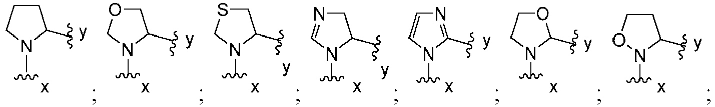 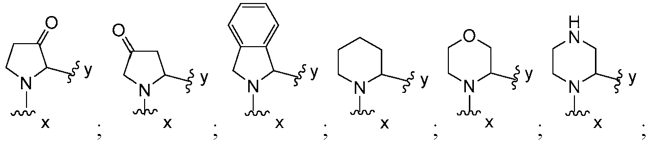 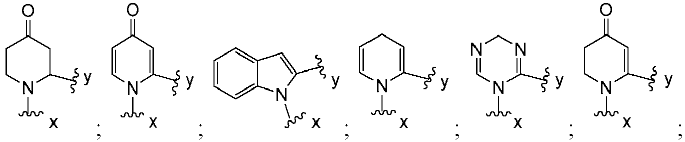 and 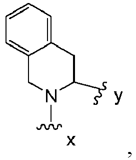 wherein x denotes a point of attachment to E, and y denotes a point of attachment to the carbon atom to which R¹ is attached.

In some embodiments of Formula (I), or Formula (III), when A is NR³, R³ and R², together with A, the carbon atom to which R¹ is attached, and the carbon atom to which R² is attached, form an optionally substituted 4 to 8 membered aliphatic heterocyclic ring.

### Cleavable E-groups

In some embodiments of Formula (I), or Formula (III), E is a cleavable moiety that upon cleavage liberates a free AH group, where H is hydrogen. The cleavable moiety E can include, for example:

### 1) An E moiety cleavable by any one of the following enzymes (e.g., intracellular enzymes):

### a) Esterases

All esters, carbonates and methyloxy esters can be hydrolyzed by an esterase enzyme. The reactivity of these functional groups in the enzymatic reaction can be modulated by selection of a carboxylic acid component of the ester functional group containing different electron donating groups or by making the ester sterically hindered. Both the acid component and the alcohol component of the ester may be sterically hindered.

### b) Reductases

Methyl azido, azido, 3-(2-nitrophenyl)propyl carbamate, [2,2-dimethyl-2-(2-nitrophenyl) acety- l]oxy}methyl (DAM) ethers, 2-nitrophenyl- methyl carbamate groups and 2-oxymethyleneantraquinone carbamates (MAQC) are examples of cleavable moieties E, that both can be cleaved by a reductase enzyme. For example, a moiety E cleavable by a reductase is a methyl azido group, or the moiety E may be of the following formulae:

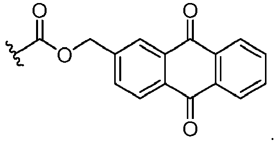

### c) Glycosidases

If A-E represents a heteroatom substituted by a sugar residue forming a glycosidic bond with the rest of the compound of Formula (I), or Formula (III), then an action of a glycosidase in vivo can cleave E and liberate the free A-H.

In some embodiments, E is cleavable by an enzyme (e.g., an intracellular enzyme) selected from the group consisting of an esterase, a specific or an unspecific peptidase, a reductase, an oxidase, a glycosidase, a hydrolase, a glycosyl transferase, and a transaminase. In some embodiments, E is cleavable by an enzyme (e.g., an intracellular enzyme) selected from the group consisting of an esterase, a reductase, an oxidase, a glycoside, a hydrolase and glycosyl transferase.

See, e.g., U.S. Publication No. 2018/0360974.

### 2) Acid-cleavable moieties.

Any acid-cleavable alcohol protecting group can be used as cleavable moiety E. For example, acetals, ortho-esters and phenyl substituted ethers can be used. Examples of such cleavable moieties include protecting groups such as THF, MTHP or MDMP, and also more labile acetals such as methoxy isopropyl acetal or methoxy cyclohexenyl acetal. Other examples of cleavable moieties E of this type that are cleaved in an acidic environment include dimethoxytrityl, trimethoxytrityl and pixyl groups. See, e.g., U.S. Publication No. 2018/0360974.

### 3) Glutathione

In some embodiments, E contains a dithio group, cleavable by a biogenic thiol. In some embodiments of Formula (I), or Formula (III), moiety E is cleavable by a glutathione.

In some embodiments, E is a group of any one of the following formulae: 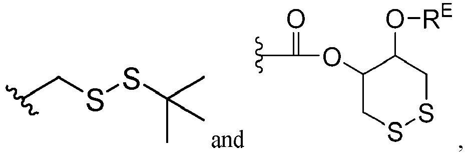 wherein R^{E} is selected from the group consisting of C₁₋₆ alkyl and benzyl. In some embodiments, R^{E} is C₁₋₆ alkyl. In some embodiments, R^{E} is benzyl.

In some embodiments, A is O, and E is a group of formula:

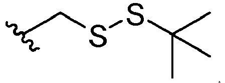

In some aspects of these embodiments, A is NH, and E is a group of formula:

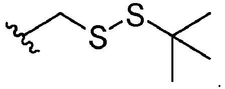

In some embodiments, A is O, and E is a group of formula:

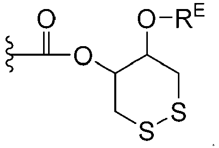

In some aspects of these embodiments, A is NH, and E is a group of formula:

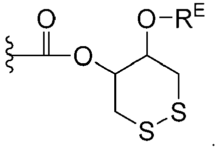

### 4) Non-specific peptidase

In some embodiments, E contains a short peptide connected via an amido bond with group A (e.g., when A = NH). In some embodiments, this amido bond is cleaved by proteases present within a tumor cell, as demonstrated in several publications, e.g. D. Neri et al. Bioconjugate Chem. 2017, (28), 1826-1833 and in "List of Enzyme Inhibitors Inhibitors and Substrates" issued by Peptide Institute Inc. Japan. Enzymatic hydrolysis of the amido bond can liberate a free vicinal amino group, which can then attack the phosphotriester group as described herein.

In some embodiments of Formula (I), or Formula (III), E is cleavable by an enzyme selected from the group consisting of an esterase, a reductase, an oxidase and a glycosidase or glycosyl transferase.

In some embodiments of Formula (I), or Formula (III), E is cleavable by a reductase.

In some aspects of these embodiments, A is O and E is a group of formula:

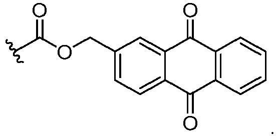

In some aspects of these embodiments, A is NH and E is a group of formula:

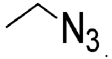

In some embodiments of Formula (I), or Formula (III), E is cleavable by a glutathione. In some aspects of these embodiments, A is NH. In other aspects of these embodiments, E is a moiety of formula:

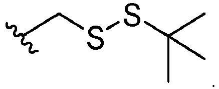

In some embodiments of Formula (I), or Formula (III), E is cleavable by a glutathione. In some aspects of these embodiments, A is NH. In other aspects of these embodiments, E is a moiety of formula:

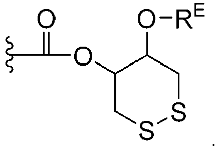

In some embodiments of Formula (I), or Formula (III), E is cleavable by a glutathione. In some aspects of these embodiments, A is O. In other aspects of these embodiments, E is a moiety of formula:

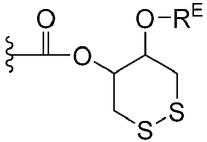

In some embodiments of Formula (I), or Formula (III), E is cleavable by a glycosidase. In some aspects of these embodiments, E is a residue of a sugar (e.g., glucose, galactose or mannose).

In some embodiments of Formula (I), or Formula (III), a moiety E is attached to A using a group of formula (L^{E}): 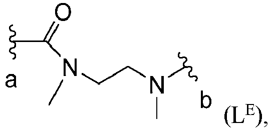 wherein a denotes a point of attachment to A, and b denotes a point of attachment to E. In some aspects of these embodiments, A is O. In other aspects of these embodiments, upon cleavage of the moiety E, the group of formula L^{E} undergoes decomposition reaction to yield CO₂ and a compound of formula: 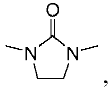 thus liberating a free A⁻ group, which may then undergo a nucleophilic attack on the poshorus atom in the compound of Formula (I), or Formula (III). The A⁻ group may also be protonated to yield the group AH prior to the nucleophilic attack.

In some embodiments, E is a cleavable moiety of any one of the following formulae (E-1) to (E-12) and (E-37): 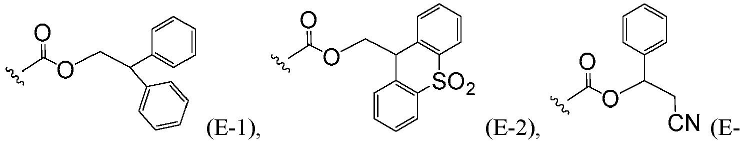 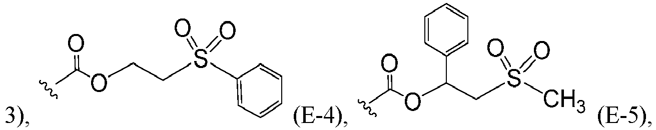 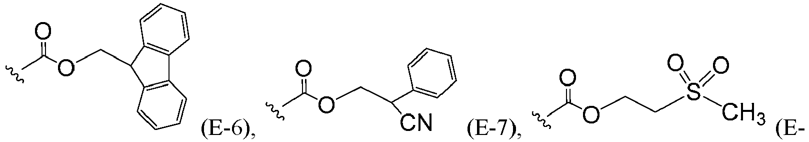 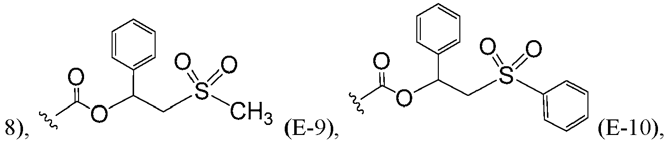 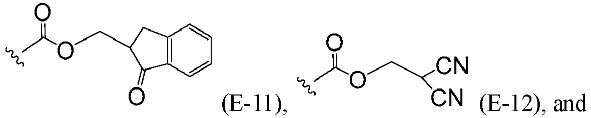 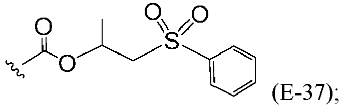 wherein any one of the phenyl rings in the formulae (E-1) to (E-12) and (E-37) is optionally substituted with 1, 2, 3, 4, or 5 substituents selected from C₁₋₁₀ alkyl, C₁₋₁₀ haloalkyl, C₁₋₁₀ alkoxy, OH, NO₂, CN, halogen and acyl.

In some embodiments, E is a cleavable moiety of any one of the following formulae (E-1) to (E-12).

In some embodiments, any one of the phenyl rings in the formulae (E-1) to (E-12) is optionally substituted with 1, 2, 3, or 4 substituents selected from F, Cl, CN, acetyl, NO₂ and CF₃.

In some embodiments, E is a group of any one of the following formulae (E-13) to (E-36): 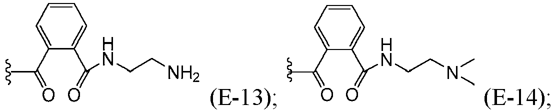 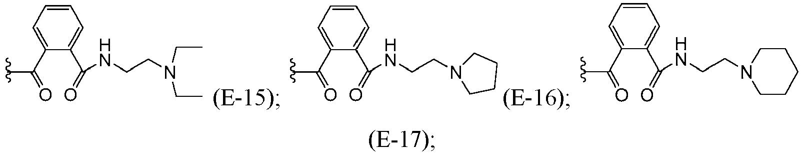 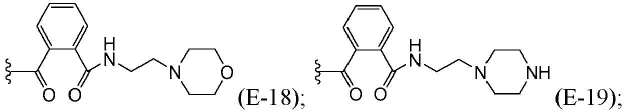 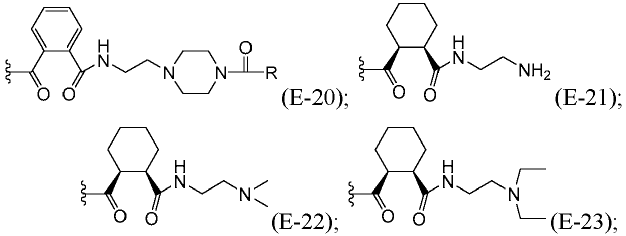 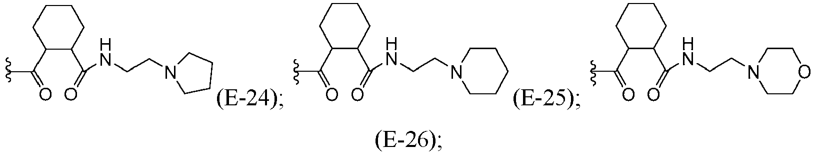 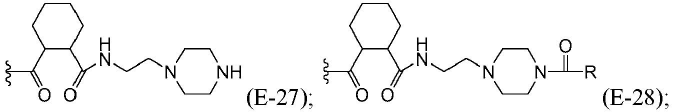 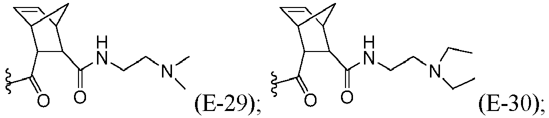 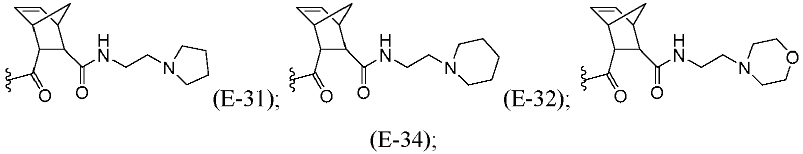 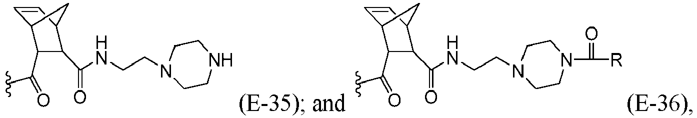 wherein R is C₁₋₆ alkyl.

In some embodiments, any one of cleavable moieties (E-1) to (E-36) may be attached to A using a moiety of formula (L^{E}).

In some embodiments, E is a 4 to 7 membered aliphatic heterocyclic ring. For example, the 4 to 7 membered aliphatic heterocyclic ring can be selected from the group consisting of pyrrolidine, piperidine, tetrahydrofuran and tetrahydropyran.

In some embodiments, E is N-Ala-Val-Ac.

### Cytotoxic or chemotherapeutic compounds

In some embodiments of Formula (I), or Formula (III), D is a residue of any one of the cytotoxic or chemotherapeutic compounds described herein. The cytotoxic or chemotherapeutic compounds may be a therapeutic protein or a small-molecule (e.g., low molecular weight) drug as described herein. The residue of cytotoxic or chemotherapeutic compounds in Formula (I), or Formula (III) may be shown as "D" or "Drug", which symbols are used herein interchangeably.

In one example, prior to being conjugated to form a compound of Formula (I) as described herein, the cytotoxic or chemotherapeutic compound may be described as a compound of formula HZ³D, wherein HZ³- represents a reactive amino- or hydroxyl-group of the cytotoxic or chemotherapeutic compound (when Z³ is nitrogen or oxygen, respectively), and D is a residue of the cytotoxic or chemotherapeutic compound (e.g., a small molecule drug). For example, when the cytotoxic or chemotherapeutic compound is a small-molecule drug containing a reactive amino group such as lisinopril: 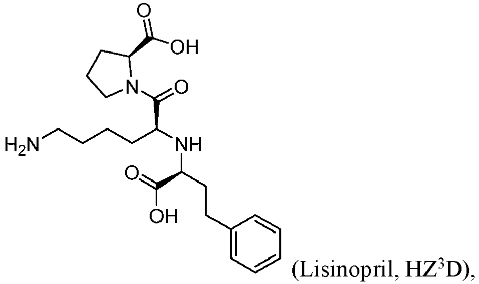 D is a residue of the cytotoxic or chemotherapeutic compound having the following formula: 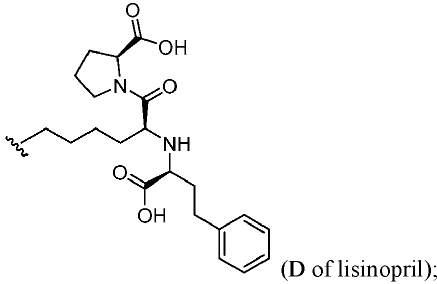 and Z³ is -NH-.

In some embodiments of Formula (I) or or when the cytotoxic or chemotherapeutic compound is a protein, HZ³- may represent an amino group or a hydroxyl group of the side chain of an amino acid within the protein backbone (e.g., lysine), and D represents the rest of the protein backbone. For example, HZ³- may be a ε-amino group of a lysine. In another example, HZ³- may be an OH- group as in a serine residue.

In some embodiments of a compound of Formula (I) or or when Z³ is absent, prior to being conjugated to form the compound of Formula (I) or or the cytotoxic or chemotherapeutic compound may be described as a compound of formula HO-(C=O)-D. In this example, the moiety of formula: 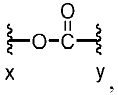 in the self-immolative group M^{A} of any one of the formulae (a)-(g) represents the part of the drug, after the drug is being conjugated to form the compound of Formula (I). In this moiety, x represents a point of attachment to M^{A'} (as described herein) of the self-immolative group, or to the H- of the drug HO-(C=O)-D prior to conjugation, and y represents a point of attachment to D.

For example, if D was a small-molecule drug containing a carboxyl group such as ibuprofen: 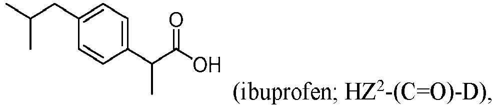 D would be a residue of the biologically active drug having the following formula: 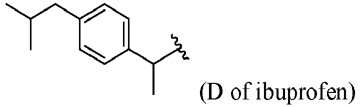 and HZ²-(C=O)- is HO-(C=O)-.

In some embodiments of Formula (I), or Formula (III), the cytotoxic or chemotherapeutic compound is an oligopeptide, peptidomimetic substance, polypeptide, a protein, or an oligonucleotide. In some embodiments, the cytotoxic or chemotherapeutic compound is a therapeutic protein, such as an antibody, a hormone, a transmembrane protein, a growth factor, an enzyme, or a structural protein.

In some embodiments, the protein therapeutic is any one of the protein therapeutics described in, e.g., B. Leader et al., Nature Reviews 2008, 7, 21-39.

In some embodiments, the therapeutic antibody is useful in treating cancer. In some embodiments, the therapeutic antibody useful in treating cancer is abagovomab, adecatumumab, afutuzumab, alacizu pegol, altumomab pentetate, amatuximab, anatumomab mafenatox, apolizumab, arcitumomab, bavituximab, bectumomab, belimumab, bevacizumab, bivatuzumab mertansine, blinatumomab, brentuximab vedotin, cantuzumab mertansine, cantuzumab ravtansine, capromab pendetide, cetuximab, citatuzumab bogatox, cixutumumab, clivatuzumab tetraxetan, dacetuzumab, demcizumab, detumomab, drozitumab, ecromeximab, eculizumab, elotuzumab, ensituximab, epratuzumab, etaracizumab, farletuzumab, figitumumab, flanvotumab, galiximab, gemtuzumab ozogamicin, girentuximab, ibritumomab tiuxetan, imgatuzumab, ipilimumab, labetuzumab, lexatumumab, lorvotuzumab mertansine, nimotuzumab, ofatumumab, oregovomab, panitumumab, pemtumomab, pertuzumab, tacatuzumab tetraxetan, tositumomab, trastuzumab, totumumab, or zalutumumab.

In some embodiments of Formula (I), or Formula (III), the cytotoxic or chemotherapeutic compound is a small-molecule drug. Small molecule drugs are low molecular weight compounds (typically about 2000 daltons or less). In some embodiments, the molecular weight of the drug molecule is in the range from about 200 to about 2000, from about 200 to about 1800, from about 200 to about 1600, from about 200 to about 1400, from about 200 to about 1200, from about 200 to about 1000, from about 200 to about 800, from about 200 to about 600 daltons, from about 300 to about 2000, from about 300 to about 1800, from about 300 to about 1600, from about 300 to about 1400, from about 300 to about 1200, from about 300 to about 1000, from about 300 to about 800, and/or from about 300 to about 600 daltons.

In some embodiments, the small-molecule drug comprises an amino group (e.g., when Z³ is NH) or a carboxyl group (e.g., when Z³ is absent).

Examples of suitable chemotherapeutic agents include any of: abarelix, aldesleukin, alitretinoin, allopurinol, altretamine, anastrozole, asparaginase, azacitidine, bexarotene, bleomycin, bortezomib, busulfan, calusterone, capecitabine, carboplatin, carmustine, chlorambucil, cisplatin, cladribine, clofarabine, cyclophosphamide, cytarabine, dacarbazine, dactinomycin, dalteparin, dasatinib, daunorubicin, decitabine, denileukin, dexrazoxane, docetaxel, doxorubicin, dromostanolone, epirubicin, erlotinib, estramustine, etoposide, exemestane, filgrastim, floxuridine, fludarabine, fluorouracil, fulvestrant, gefitinib, gemcitabine, goserelin acetate, histrelin acetate, idarubicin, ifosfamide, imatinib, irinotecan, lapatinib ditosylate, lenalidomide, letrozole, leucovorin, leuprolide, levamisole, lomustine, meclorethamine, megestrol, melphalan, mercaptopurine, methotrexate, methoxsalen, mitomycin C, mitotane, mitoxantrone, nandrolone, nelarabine, nofetumomab, oxaliplatin, paclitaxel, pamidronate, pegaspargase, pegfilgrastim, pemetrexed, pentostatin, pipobroman, plicamycin, procarbazine, quinacrine, rasburicase, ruxolitinib, sorafenib, streptozocin, sunitinib, tamoxifen, temozolomide, teniposide, testolactone, thalidomide, thioguanine, thiotepa, topotecan, toremifene, tretinoin, uracil mustard, valrubicin, vinblastine, vincristine, vinorelbine, vorinostat, and zoledronate, or a pharmaceutically acceptable salt thereof.

In some embodiments, more than one cytotoxic agent is coupled to a single cleavable linker (e.g., two cytotoxic agents, three cytotoxic agents, four cytotoxic agents, etc.). In some embodiments, more than one cytotoxic agent is coupled to a single antibody moiety (e.g., two cytotoxic agents, three cytotoxic agents, four cytotoxic agents, five cytotoxic agents, etc.).

### Targeting Antibodies

A targeting antibody or an antibody fragment as described herein can be any antibody or antibody fragment that is targeted to an enzyme or surface receptor (e.g., an enzyme or surface receptor targeted in monoclonal antibody therapy). As used herein, the term "antibody" refers to proteins that bind to a specific antigen. "Antibodies" include, but are not limited to, immunoglobulins, including polyclonal, monoclonal, chimeric, single chain, humanized antibodies, Fab fragments, F(ab')2 fragments, and Fab expression libraries.

As used herein the term "antibody" refers to a glycoprotein evoked in an animal by an immunogen (antigen). An antibody demonstrates specificity to the immunogen, or, more specifically, to one or more epitopes contained in the immunogen. Native antibody comprises at least two light polypeptide chains and at least two heavy polypeptide chains. Each of the heavy and light polypeptide chains contains at the amino terminal portion of the polypeptide chain a variable region (i.e., V_{H} and V_{L} respectively), which contains a binding domain that interacts with antigen. Each of the heavy and light polypeptide chains also comprises a constant region of the polypeptide chains (generally the carboxy terminal portion) which may mediate the binding of the immunoglobulin to host tissues or factors influencing various cells of the immune system, some phagocytic cells and the first component (C1q) of the classical complement system. The constant region of the light chains is referred to as the "CL region," and the constant region of the heavy chain is referred to as the "CH region." The constant region of the heavy chain comprises a CH1 region, a CH2 region, and a CH3 region. A portion of the heavy chain between the CH1 and CH2 regions is referred to as the hinge region (i.e., the "H region"). The constant region of the heavy chain of the cell surface form of an antibody further comprises a spacer-transmembranal region (M1) and a cytoplasmic region (M2) of the membrane carboxy terminus. The secreted form of an antibody generally lacks the M1 and M2 regions.

As used herein, the term "antigen" refers to any molecule or molecular group that is recognized by at least one antibody. By definition, an antigen must contain at least one epitope (i.e., the specific biochemical unit capable of being recognized by the antibody). The term "immunogen" refers to any molecule, compound, or aggregate that induces the production of antibodies. By definition, an immunogen must contain at least one epitope.

In some embodiments, a targeting antibody can be selected from the group consisting of 20-(74)-(74) (milatuzumab; veltuzumab), 3F8, 74-(20)-(20) (milatuzumab; veltuzumab), 8H9, A33, AB-16B5, abagovomab, abciximab, abituzumab, ABP 494 (cetuximab biosimilar), abrilumab, ABT-700, ABT-806, actoxumab, adalimumab, ADC-1013, adecatumumab, afelimomab, AFM13, afutuzumab, AGEN1884, alemtuzumab, alirocumab, altumomab, amatuximab, AMG 228, anifrolumab, anrukinzumab, APN311, apolizumab, APX005M, aselizumab, atezolizumab, atinumab, atlizumab (also referred to as tocilizumab), atorolimumab, Avelumab, B-701, bapineuzumab, basiliximab, bavituximab, bectumomab, begelomab, belimumab, benralizumab, bertilimumab, bevacizumab, bevacizumab (CBT124), BEVZ92 (bevacizumab biosimilar), bezlotoxumab, BGB-A317, BHQ880, BI-505, bimagrumab, bimekizumab, BIW-8962, blinatumomab, blosozumab, BMS-936559, BMS-986012, BMS-986016, BMS-986178, BNC101, bococizumab, BrevaRex, briakinumab, brodalumab, brolucizumab, brontictuzumab, canakinumab, carlumab, carotuximab (TRC105), catumaxomab, CC-90002, cedelizumab, cemiplimab (REGN2810), cetuximab, CGEN-15001T, CGEN-15022, CGEN-15029 (PVRIG), CGEN-15049, CGEN-15052, CGEN-15092, cixutumumab, claudiximab (IMAB262), clazakizumab, clazakizumab (ALD518), clenoliximab, CM-24, codrituzumab, conatumumab, concizumab, cR6261, crenezumab, DA-3111 (trastuzumab biosimilar), dacetuzumab, daclizumab, dalotuzumab, daratumumab, Daratumumab Enhanze (daratumumab), dectrekumab, demcizumab, denosumab, detumomab, DI-B4, dinutuximab (Ch 14.18), diridavumab, DKN-01, drozitumab, DS-1123, DS-8895, duligotumab, dupilumab, durvalumab, dusigitumab, ecromeximab, eculizumab, edobacomab, edrecolomab, efalizumab, efungumab, eldelumab, elgemtumab, elotuzumab, elsilimomab, emactuzumab, emibetuzumab, enavatuzumab, enoblituzumab, enokizumab, enoticumab, ensituximab, epratuzumab, erlizumab, ertumaxomab, etaracizumab, etrolizumab, evinacumab, evolocumab, exbivirumab, faralimomab, farletuzumab, fasinumab, FBTA05, felvizumab, fezakinumab, ficlatuzumab, figitumumab, firivumab, flanvotumab, fletikumab, Flotetuzumab, fontolizumab, foralumab, foravirumab, FPA144, fresolimumab, fulranumab, futuximab, galiximab, ganitumab, gantenerumab, Gatipotuzumab (PankoMab-GEX^{®}), gavilimomab, Gerilimzumab, gevokizumab, girentuximab, GNR-006, GNR-011, golimumab, gomiliximab, GSK2849330, GSK3174998, GSK3359609, guselkumab, Hu14.18K322A MAb, hu3S193, Hu8F4, HuL2G7, HuMab-SB1, ibalizumab, icrucumab, idarucizumab, IGN523, IMAB362, imalumab, IMC-CS4, IMC-D11, imgatuzumab, IMMU-114, ImmuTune IMP701 Antagonist Antibody, INCAGN1876, inclacumab, INCSHR1210, inebilizumab (MEDI-551), infliximab, inolimomab, intetumumab, IPH4102, ipilimumab, iratumumab, isatuximab, Istiratumab, itolizumab, ixekizumab, JNJ-56022473, JNJ-61610588, keliximab, KTN3379, Labetuzumab, LAG525, LAG525, lambrolizumab, lebrikizumab, lemalesomab, lenzilumab, lerdelimumab, Leukotuximab, lexatumumab, libivirumab, ligelizumab, lintuzumab, lirilumab, lodelcizumab, lokivetmab, lucatumumab, lumiliximab, lumretuzumab, LY3164530, mapatumumab, margetuximab, maslimomab, matuzumab, mavrilimumab, MB311, MCS-110, MEDI0562, MEDI-0639, MEDI0680, MEDI-3617, MEDI-565, MEDI6469, mepolizumab, metelimumab, MGB453, MGD007, MGD009, MGD011, milatuzumab, minretumomab, mitumomab, MK-4166, MM-111, mogamulizumab, MOR202, MOR208, MORAb-066, morolimumab, motavizumab, muromonab-CD3, namilumab, namatumab, natalizumab, nebacumab, necitumumab, nemolizumab, nerelimomab, nesvacumab, nimotuzumab, nivolumab, NIZ985, obiltoxaximab, obinutuzumab, ocaratuzumab, ocrelizumab, odulimomab, ofatumumab, olaratumab, olokizumab, omalizumab, OMP-131R10, OMP-305B83, onartuzumab, ontuxizumab, opicinumab, oregovomab, orticumab, otelixizumab, otlertuzumab, oxelumab, ozanezumab, ozoralizumab, pagibaximab, palivizumab, panitumumab, pankomab, panobacumab, panobacumab, parsatuzumab, pascolizumab, pasotuxizumab, pateclizumab, patritumab, PAT-SC1, PAT-SM6, PDR001, pembrolizumab, pemtumomab, perakizumab, pertuzumab, pexelizumab, PF-06671008, PF-06801591, pidilizumab, pintumomab, placulumab, ponezumab, PR0131921 , priliximab, pritoxaximab, pritumumab, PRO 140, quilizumab, racotumomab, rafivirumab, ralpancizumab, ramucirumab, raxibacumab, refanezumab, regavirumab, REGN1400, reslizumab, RG7356, RG7386, RG7802 (CEA TCB), RG7876, RG7888, rilotumumab, rinucumab, rituximab, RO7009789, robatumumab, roledumab, romosozumab, rontalizumab, rovelizumab, ruplizumab, samalizumab, sarilumab, SCT200, SCT400, SEA-CD40, secukinumab, seribantumab, setoxaximab, sevacizumab (APX003, SIM-BD0801) , sevirumab, sibrotuzumab, sifalimumab, siltuximab, simtuzumab, siplizumab, sirukumab, solanezumab, solitomab, sonepcizumab, sontuzumab, stamulumab, suvizumab, SYM004 (futuximab and modotuximab), TAB08, tabalumab, tadocizumab, talizumab, tanezumab, Tanibirumab, tarextumab, TB-403, tefibazumab, tenatumomab, teneliximab, teplizumab, teprotumumab, tesidolumab, tetulomab, TG-1101, ticilimumab, tigatuzumab, tildrakizumab, TNX-650, tocilizumab, toralizumab, tosatoxumab, tositumomab, tovetumab, tralokinumab, trastuzumab, TRBS02, TRBS07, tregalizumab, tremelimumab, trevogrumab, TRPH 011, TRX518, TSR-042, TTI-200.7, tuvirumab, U3-1565, U3-1784, ublituximab, ulocuplumab, urelumab, urtoxazumab, ustekinumab, utomilumab (PF-05082566), vantictumab, vanucizumab, vapaliximab, varlilumab, vatelizumab, vedolizumab, veltuzumab, vepalimomab, vesencumab, visilizumab, volociximab, YYB-101, zalutumumab, zanolimumab, zatuximab, and ziralimumab.

In some embodiments, the targeting antibody can be selected from the antibody portion of an ADC selected from the group consisting of ABBV-085, ABBV-221, ABBV-838, AbGn-107, ADCT-301, ADCT-402, AGS15E, AGS-16C3F, AGS62P1, AGS67E, AMG 172, AMG 224, AMG 595, anetumab ravtansine, ARX788, ASG-15ME, AVE9633, BAY1129980 (Lupartumab amadotin), BAY1187982 (Aprutumab ixadotin), BIIB015, bivatuzumab mertansine, BMS-936561, BMS-986148, brentuximab vedotin, cantuzumab mertansine, cantuzumab ravtansine, CBR96-doxorubicin immunoconjugate, CDX-014, CMB-401, CMD-193, coltuximab ravtansine, Cotara (iodine 1-131 derlotuximab biotin), denintuzumab mafodotin, Depatuxizumab mafodotin, derlotuximab biotin, DMOT4039A, dorlimomab aritox, DS-8201a, DSTA4637S, enfortumab vedotin, FGFR2 Antibody-Drug Conjugate, gemtuzumab ozogamicin, glembatumumab vedotin, GSK2857916, HKT288, HuMax-AXL-ADC, IMGN388, IMGN632, IMGN779, indatuximab ravtansine, indusatumab vedotin, inotuzumab ozogamicin, Labetuzumab Govitecan, laprituximab emtansine, lifastuzumab vedotin, LOP 628, lorvotuzumab mertansine, LY3076226, MEDI4276, MEDI547, Milatuzumab doxorubicin, Milatuzumab-SN-38, mirvetuximab soravtansine, MLN2704, MM-302, moxetumomab pasudotox, nacolomab tafenatox, Naratuximab emtansine (IMGN529), OX002 (MEN1309), PCA062, PF-06263507, PF-06647020, PF-06647263, PF-06650808, PF-06664178, pinatuzumab vedotin, polatuzumab vedotin, PSMA ADC, RC48-ADC, RG7636, RG7841, RG7882, RG7986, RM-1929, Rovalpituzumab tesirine, sacituzumab govitecan, SAR408701, SAR428926, SAR566658, SC-002, SC-003, SGN-CD123A, SGN-CD19A, SGN-CD19B, SGN-CD33A, SGN-CD352A, SGN-CD70A, SGN-LIV1A, sirukumab vedotin, SYD985, telisotuzumab vedotin, Tisotumab vedotin, trastuzumab duocarmazine, trastuzumab emtansine, U3-1402, vadastuximab talirine, vandortuzumab vedotin, vorsetuzumab mafodotin, and XMT-1522.

In some embodiments, the targeting antibody can be selected from an antibody portion of a molecule selected from the group consisting of Actimab-A (actinium Ac-225 lintuzumab), alacizumab, alacizumab pegol, altumomab pentetate, anatumomab mafenatox, APN301, arcitumomab, ATL101, besilesomab, Betalutin, BI 836880, biciromab, C2-2b-2b, caplacizumab, capromab pendetide, CDX-1401, certolizumab pegol, citatuzumab bogatox, clivatuzumab tetraxetan, dapirolizumab pegol, Darleukin, enlimomab pegol, epitumomab cituxetan, epratuzumab tetraxetan, fanolesomab, FF-21101, Fibromun, FS102, ibritumomab tiuxetan, IGN002, igovomab, imciromab, Ipafricept, L19IL2/L19TNF, lampalizumab, L-DOS47, lilotomab satetraxetan, LKZ145, lulizumab pegol, MM-151, naptumomab estafenatox, nofetumomab merpentan, oportuzumab monatox, Proxinium, radretumab, ranibizumab, RFM-203, RG7813, SAT 012, satumomab pendetide, sulesomab, Sym015, tacatuzumab tetraxetan, taplitumomab paptox, Teleukin, telimomab aritox, Thorium-227-Epratuzumab Conjugate, tositumomab-I131, tucotuzumab celmoleukin, VB6-845, votumumab, zolimomab aritox

Various procedures known in the art can be used for the production of polyclonal antibodies. For the production of antibody, various host animals can be immunized by injection with the peptide corresponding to the desired epitope including but not limited to rabbits, mice, rats, sheep, goats, etc. In some embodiments, the peptide is conjugated to an immunogenic carrier (e.g., diphtheria toxoid, bovine serum albumin [BSA], or keyhole limpet hemocyanin [KLH]). Various adjuvants can be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides production of antibody molecules by continuous cell lines in culture may be used. (See e.g., Harlow and Lane, Antibodies: A Laboratory Manual, Cold Spring Harbor Laboratory Press, Cold Spring Harbor, N.Y.). These include, but are not limited to, the hybridoma technique originally developed by Kohler and Milstein (Köhler and Milstein, Nature, 256:495-497 [1975]), as well as the trioma technique, the human B-cell hybridoma technique (See e.g., Kozbor et al., Immunol. Today, 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]). In other embodiments, suitable monoclonal antibodies, including recombinant chimeric monoclonal antibodies and chimeric monoclonal antibody fusion proteins are prepared as described herein.

Techniques described for the production of single chain antibodies (U.S. Pat. No. 4,946,778) can be adapted to produce specific single chain antibodies as desired. In some embodiments, methods for the construction of Fab expression libraries can be used (Huse et al., Science, 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab*'* )2 fragment that can be produced by pepsin digestion of an antibody molecule; the Fab' fragments that can be generated by reducing the disulfide bridges of an F(ab*'* )2 fragment, and the Fab fragments that can be generated by treating an antibody molecule with papain and a reducing agent.

### Polymers

In some embodiments of Formula (I), or Formula (III), the aliphatic moiety can be a polymer. A polymer, as described herein, can be branched or linear. For example, a polymer can have from 2 to 100 termini (e.g., 2 to 80, 2 to 75, 2 to 60, 2 to 50, 2 to 40, 2 to 35, 2 to 25, 2 to 10, 2 to 5, 4 to 20, 5 to 25, 10 to 50, 25 to 75, 3 to 6, 5 to 15 termini). In some embodiments, a polymer can have from 2 to 5, 4 to 6, 5 to 6, or 3 to 6 termini. In some embodiments, a polymer is linear and therefore has 2 termini. In some embodiments of or one termini of a polymer is covalently bonded to the structure of any one of the formulae provided herein.

A polymer can be, for example, poly(alkylene glycol), poly(oxyethylated polyol), poly(olefinic alcohol), poly(β-hydroxy acid), poly(vinyl alcohol), polyoxazoline, or a copolymer thereof. A polyalkylene glycol includes linear or branched polymeric polyether polyols. Such polyalkylene glycols, include, but are not limited to, polyethylene glycol (PEG), polypropylene glycol, polybutylene glycol, and derivatives thereof. Other exemplary embodiments are listed, for example, in commercial supplier catalogs, such as Shearwater Corporation's catalog "Polyethylene glycol and Derivatives for Biomedical Applications" (2001).

In some embodiments, such polymeric polyether polyols have average molecular weights between about 0.1 kDa to about 100 kDa. For example, such polymeric polyether polyols include, but are not limited to, between about 500 Da and about 100,000 Da or more. The molecular weight of the polymer may be between about 500 Da and about 100,000 Da. For example, a polymer used herein can have a molecular weight of about 100,000 Da, 95,000 Da, 90,000 Da, 85,000 Da, 80,000 Da, 75,000 Da, 70,000 Da, 65,000 Da, 60,000 Da, 55,000 Da, 50,000 Da, 45,000 Da, 40,000 Da, 35,000 Da, 30,000 Da, 25,000 Da, 20,000 Da, 15,000 Da, 10,000 Da, 9,000 Da, 8,000 Da, 7,000 Da, 6,000 Da, 5,000 Da, 4,000 Da, 3,000 Da, 2,000 Da, 1,000 Da, 900 Da, 800 Da, 700 Da, 600 Da, and 500 Da. In some embodiments, the molecular weight of the polymer is between about 500 Da and about 50,000 Da. In some embodiments, the molecular weight of the polymer is between about 500 Da and about 40,000 Da. In some embodiments, the molecular weight of the polymer is between about 1,000 Da and about 40,000 Da. In some embodiments, the molecular weight of the polymer is between about 5,000 Da and about 40,000 Da. In some embodiments, the molecular weight of the polymer is between about 10,000 Da and about 40,000 Da.

In some embodiments, a polymer is a linear or branched polyethylene glycol).

In some embodiments, the poly(ethylene glycol) molecule is a linear polymer. Linear PEG can be alkylated (e.g., methylated or ethylated), at one termini, but they can by incorporated to the conjugate of any one of the formulae disclosed herein using the free terminus in the non-derivatized hydroxyl form. The molecular weight of the linear chain PEG may be between about 1,000 Da and about 100,000 Da. For example, a linear chain PEG used herein can have a molecular weight of about 100,000 Da, 95,000 Da, 90,000 Da, 85,000 Da, 80,000 Da, 75,000 Da, 70,000 Da, 65,000 Da, 60,000 Da, 55,000 Da, 50,000 Da, 45,000 Da, 40,000 Da, 35,000 Da, 30,000 Da, 25,000 Da, 20,000 Da, 15,000 Da, 10,000 Da, 9,000 Da, 8,000 Da, 7,000 Da, 6,000 Da, 5,000 Da, 4,000 Da, 3,000 Da, 2,000 Da, and 1,000 Da. In some embodiments, the molecular weight of the linear chain PEG is between about 1,000 Da and about 50,000 Da. In some embodiments, the molecular weight of the linear chain PEG is between about 1,000 Da and about 40,000 Da. In some embodiments, the molecular weight of the linear chain PEG is between about 5,000 Da and about 40,000 Da. In some embodiments, the molecular weight of the linear chain PEG is between about 5,000 Da and about 20,000 Da.

In some embodiments, the poly(ethylene glycol) molecule is a branched polymer. For example, branched PEG can be V-shaped, or T-shaped, depending on the method by which PEG has been synthesized. The molecular weight of the branched chain PEG may be between about 1,000 Da and about 100,000 Da. For example, a branched chain PEG used herein can have a molecular weight of about 100,000 Da, 95,000 Da, 90,000 Da, 85,000 Da, 80,000 Da, 75,000 Da, 70,000 Da, 65,000 Da, 60,000 Da, 55,000 Da, 50,000 Da, 45,000 Da, 40,000 Da, 35,000 Da, 30,000 Da, 25,000 Da, 20,000 Da, 15,000 Da, 10,000 Da, 9,000 Da, 8,000 Da, 7,000 Da, 6,000 Da, 5,000 Da, 4,000 Da, 3,000 Da, 2,000 Da, and 1,000 Da. In some embodiments, the molecular weight of the branched chain PEG is between about 1,000 Da and about 50,000 Da. In some embodiments, the molecular weight of the branched chain PEG is between about 1,000 Da and about 40,000 Da. In some embodiments, the molecular weight of the branched chain PEG is between about 5,000 Da and about 40,000 Da. In some embodiments, the molecular weight of the branched chain PEG is between about 5,000 Da and about 20,000 Da.

In some embodiments, the polyethylene glycol (linear or branched) has an average molecular weight from about 500 Da to about 40,000 Da, from about 1,000 Da to about 30,000 Da, from about 1,000 Da to about 20,000 Da, from about 5,000 Da to about 20,000 Da.

In some embodiments, the polymer (e.g., the polyethylene glycol) as provided herein has the following structural formula:

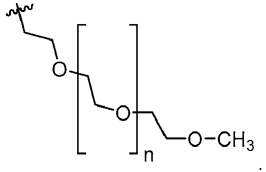

In some embodiments, n is an integer from 1 to 1,000, from 1 to 800, from 1 to 300, or from 1 to 100. In some embodiments, n is selected from 10, 20, 50, 100, 200, 250, 300, 500, 600, and 1000.

### Pharmaceutically acceptable salts

In some embodiments, a salt of a compound of Formula (I), or Formula (III) disclosed herein is formed between an acid and a basic group of the compound, such as an amino functional group, or a base and an acidic group of the compound, such as a carboxyl functional group. According to another embodiment, the compound is a pharmaceutically acceptable acid addition salt.

In some embodiments, acids commonly employed to form pharmaceutically acceptable salts of the compounds of Formula (I), or Formula (III) disclosed herein include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, mandelate and other salts. In one embodiment, pharmaceutically acceptable acid addition salts include those formed with mineral acids such as hydrochloric acid and hydrobromic acid, and especially those formed with organic acids such as maleic acid.

In some embodiments, bases commonly employed to form pharmaceutically acceptable salts of the compounds of Formula (I), or Formula (III) disclosed herein include hydroxides of alkali metals, including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-(C1-C6)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine, lysine, and the like.

In some embodiments, the compounds of Formula (I), or Formula (III) disclosed herein, or pharmaceutically acceptable salts thereof, are substantially isolated.

In some embodiments, this document provides a compounds of Formula (I), or Formula (III) disclosed herein, or pharmaceutically acceptable salts thereof, prepared by any one of the processes described herein.

Similar additions of antibody moieties or replacement of polymer backbones and/or aliphatic moieties can be made on other phosphotriester compounds such as those disclosed in WO 2012/080836 and WO 2013/186632.

In some embodiments, the antibody moieties described herein can include other groups in place of the antibodies or antibody fragments. For example, nucleotide or polynucleotides (apatamers*), bicycloppeptides, hormones or other substances that bind to specific cell surface receptors. See, for example, WO 2017/191460, US 2018/0200378, Mater Sci Eng CMater Biol Appl. 2017 Jan 1; 70(Pt 1):505-511; Chem Rev. 2017 Oct 11; 117(19):12133-12164, Pusuluri, A. et al. Angew Chem Int Ed Engl. 2019 Jan 28; 58(5):1437-1441, and Greg T. Hermanson, Bioconjugate Techniques, 3rd Edition, Academic Press, New York, 2013.

In some embodiments, the compounds provided herein may further comprise reporter groups. See, for example, Inorganic Materials: Synthesis and Processing, Volume 64, Issue 3 March 2018 Pages 835-859 and Mater Sci Eng CMater Biol Appl. 2017 Jan 1; 70(Pt 1):505-511. In addition to aptameric targeting agents (e.g. Gupta et al., J Biol, Chem, 289, 8706, 2014) in some embodiments, nucleoside or nucleotide substances, including non-natural ones, can provide a tracer function.

### METHODS OF MAKING

### Compounds of Formula (1)

Exemplary synthetic methods for preparing compounds of Formula (I) of the present document are described below.

In some embodiments, compounds of Formula (I) can be prepared as described in **Scheme 3.** 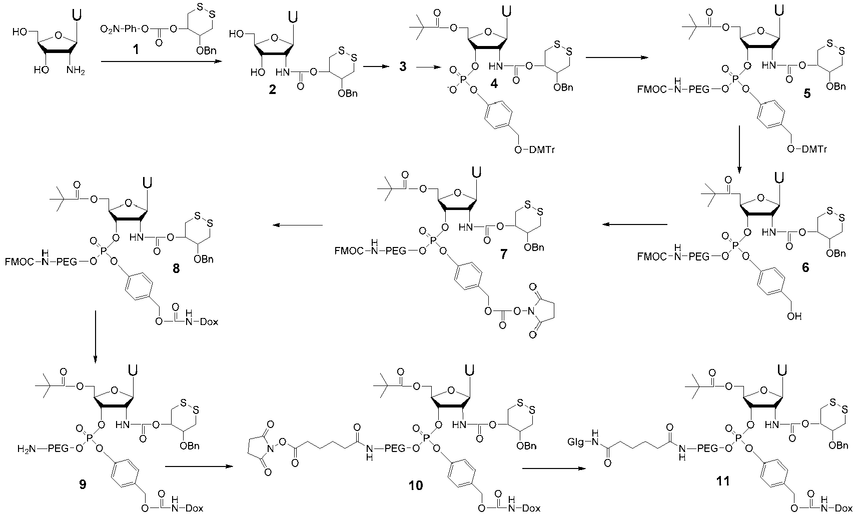 wherein U is uracil.

The cytotoxic drug, doxorubicin, shown in Scheme 3 has been selected from the large number of existing cytotoxic drugs to demonstrate the principle of the present methodology. Particularly, the characteristic light absorbance of doxyrubicin, with a maximum at 480 nm, allows for clear following of the drug at the different stages of ADC synthesis and its release upon addition of a dithiol reducing agent. As a model drug, doxorubicin has, however, some disadvantages. It contains a free and reactive carbonyl group, preventing use of aminooxy- and hydrazido-subjected linkers for covalent coupling to carbonylated antibodies. Therefore, Scheme 3 describes an example where the covalent linkage to an antibody was created by means of reacting an NHS activated drug moeity with one or more amino groups present on the surface of antibodies. See, for example, Example 1.

In some embodiments, compounds of Formula (I) can be prepared as described in **Scheme 4.** See, for example, Example 2.

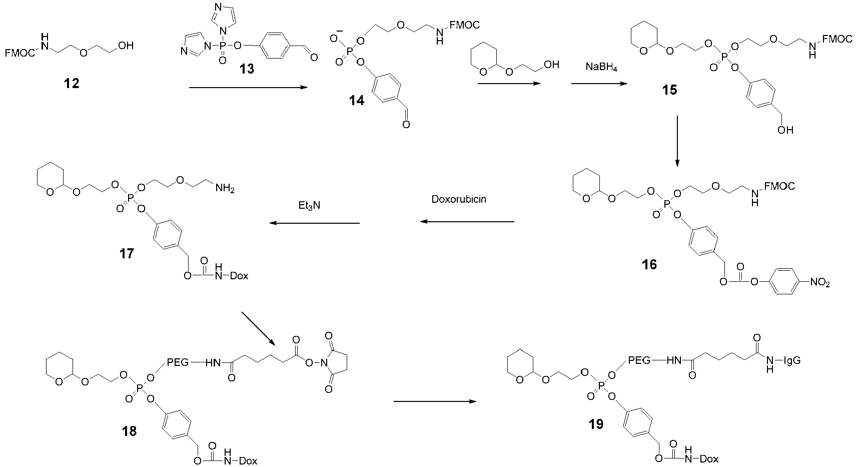

In some embodiments, compounds of Formula (I) can be prepared as described in Scheme 5. See, for example, Example 3.

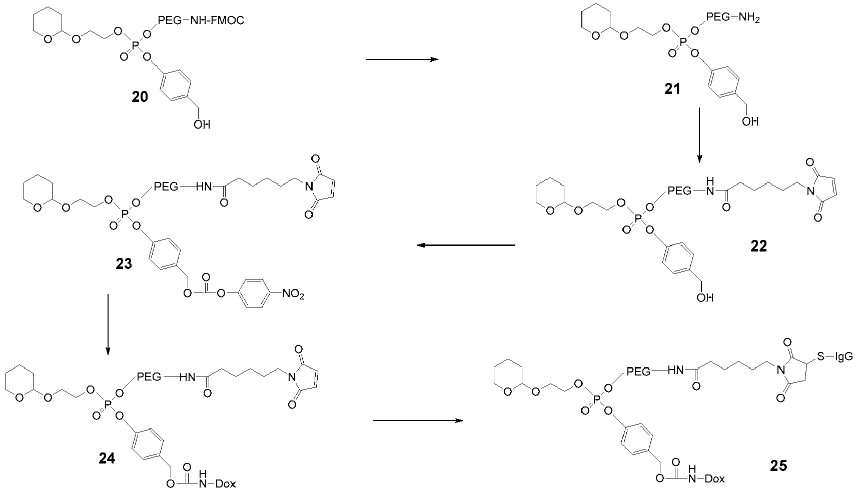

Incorporation of a maleimido group, besides increasing the scope of functional groups, has certain other strategic and synthetic advantages. A maleimido group is hydrolytically stable compared to NHS and therefore it can be incorporated into the conjugate before activation of the benzylic hydroxyl and reaction of a cytotoxic agent component. Also, addition of an amino group containing cytotoxic agent can be performed in the presence of a stronger base. It should be noted that there exist a large number of other functional groups that could be used in place of a maleimido group. For example, azido, alkyne aldehyde, aminoxy, and hydrazo groups, and appropriate reagents useful for introduction of these functions to the amino group are described in the literature. Scheme 5 describes application of a longer PEG than in Scheme 4. In some embodiments, increasing of linker length may improve reactivity of the reagent with antibody, and it may improve the water solubility of the reagent.
In some embodiments, compounds of Formula (I) can be prepared as described in **Scheme 6.** See, for example, Example 4.

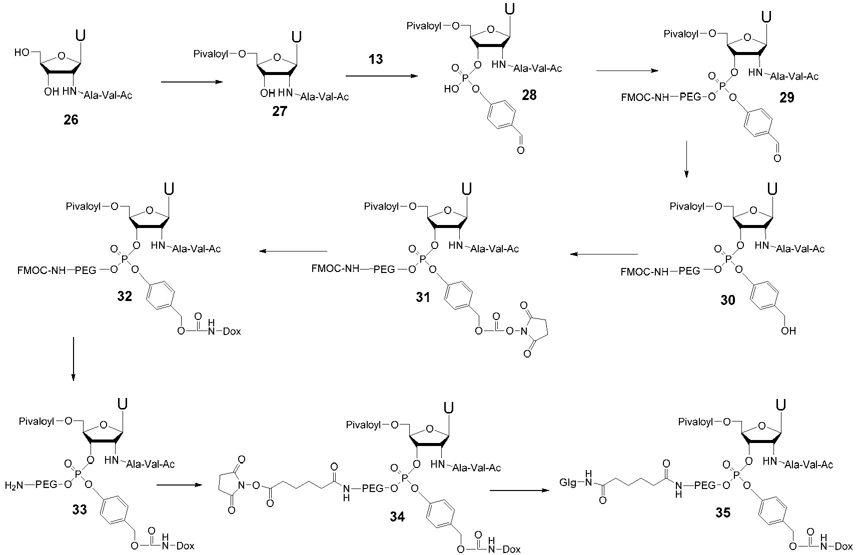

Suitable synthetic methods of starting materials, intermediates and products may be identified by reference to the literature, including reference sources such as: Advances in Heterocyclic Chemistry, Vols. 1-107 (Elsevier, 1963-2012); Journal of Heterocyclic Chemistry Vols. 1-49 (Journal of Heterocyclic Chemistry, 1964-2012); Carreira, et al. (Ed.) Science of Synthesis, Vols. 1-48 (2001-2010); Katritzky et al. (Ed.); Comprehensive Organic Functional Group Transformations II (Elsevier, 2nd Edition, 2004); Smith et al., March's Advanced Organic Chemistry: Reactions, Mechanisms, and Structure, 6th Ed. (Wiley, 2007); Trost et al. (Ed.), Comprehensive Organic Synthesis (Pergamon Press, 1991).

Preparation of the compounds provided herein can involve the protection and deprotection of various chemical groups. The chemistry of protecting groups can be found, for example, in P. G. M. Wuts and T. W. Greene, Protective Groups in Organic Synthesis, 4th Ed., Wiley & Sons, Inc., New York (2006). The chemistry and protecting group strategy related to the nucleosides and nucleotides can be found in Methods in Molecular Biology - Oligonucleotide Synthesis, edited by Piet Herdewijn, Humana Press Inc. 2005 and also in Protocols for oligonucleotide conjugate, edited by Sudhir Agrawal, Humana Press Inc. 1994. Suitable starting materials and intermediates are readily available from various commercial sources.

### METHODS OF USING THE COMPO UNDS OF THE PRESENT DOCUMENT

### Methods of treating cancer

In some embodiments, this document provides a method for treating a disease, disorder or condition in a mammal (e.g., a human in need of such treatment), comprising the step of administering to the mammal a compound of Formula (I), or Formula (III) as described herein, or a pharmaceutically acceptable salt thereof, or a pharmaceutical composition comprising same.

For example, the compounds of this documentare useful in the treatment of a disease or condition beneficially treated by administration of a cytotoxic or chemotherapeutic compound as described herein to a subject.

In some embodiments, the disease or condition is cancer.

In some embodiments, the cancer is selected from the group consisting of bladder cancer, brain cancer, breast cancer, colorectal cancer, cervical cancer, gastrointestinal cancer, genitourinary cancer, head and neck cancer, lung cancer, ovarian cancer, pancreatic cancer, prostate cancer, renal cancer, skin cancer, and testicular cancer.

In some embodiments, cancer is selected from the group selected from sarcoma, angiosarcoma, fibrosarcoma, rhabdomyosarcoma, liposarcoma, myxoma, rhabdomyoma, fibroma, lipoma, teratoma, lung cancer, bronchogenic carcinoma squamous cell, undifferentiated small cell, undifferentiated large cell, adenocarcinoma, alveolar bronchiolar carcinoma, bronchial adenoma, sarcoma, lymphoma, chondromatous hamartoma, mesothelioma, gastrointestinal cancer, cancer of the esophagus, squamous cell carcinoma, adenocarcinoma, leiomyosarcoma, lymphoma, cancer of the stomach, carcinoma, lymphoma, leiomyosarcoma, cancer of the pancreas, ductal adenocarcinoma, insulinoma, glucagonoma, gastrinoma, carcinoid tumor, vipoma, cancer of the small bowel, adenocarcinoma, lymphoma, carcinoid tumors, Kaposi's sarcoma, leiomyoma, hemangioma, lipoma, neurofibroma, fibroma, cancer of the large bowel or colon, tubular adenoma, villous adenoma, hamartoma, leiomyoma, genitourinary tract cancer, cancer of the kidney adenocarcinoma, Wilm's tumor (nephroblastoma), lymphoma, leukemia, cancer of the bladder, cancer of the urethra, squamous cell carcinoma, transitional cell carcinoma, cancer of the prostate, cancer of the testis, seminoma, teratoma, embryonal carcinoma, teratocarcinoma, choriocarcinoma, sarcoma, interstitial cell carcinoma, fibroma, fibroadenoma, adenomatoid tumors, lipoma, liver cancer, hepatoma hepatocellular carcinoma, cholangiocarcinoma, hepatoblastoma, angiosarcoma, hepatocellular adenoma, hemangioma, bone cancer, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochrondroma (osteocartilaginous exostoses), benign chondroma, chondroblastoma, chondromyxofibroma, osteoid osteoma giant cell tumor, nervous system cancer, cancer of the skull, osteoma, hemangioma, granuloma, xanthoma, osteitis deformans, cancer of the meninges meningioma, meningiosarcoma, gliomatosis, cancer of the brain, astrocytoma, medulloblastoma, glioma, ependymoma, germinoma (pinealoma), glioblastoma multiforme, oligodendroglioma, schwannoma, retinoblastoma, congenital tumors, cancer of the spinal cord, neurofibroma, meningioma, glioma, sarcoma, gynecological cancer, cancer of the uterus, endometrial carcinoma, cancer of the cervix, cervical carcinoma, pre tumor cervical dysplasia, cancer of the ovaries, ovarian carcinoma, serous cystadenocarcinoma, mucinous cystadenocarcinoma, unclassified carcinoma, granulosa-theca cell tumor, Sertoli Leydig cell tumor, dysgerminoma, malignant teratoma, cancer of the vulva, squamous cell carcinoma, intraepithelial carcinoma, adenocarcinoma, fibrosarcoma, melanoma, cancer of the vagina, clear cell carcinoma, squamous cell carcinoma, botryoid sarcoma, embryonal rhabdomyosarcoma, cancer of the fallopian tubes, hematologic cancer, cancer of the blood, acute myeloid leukemia (AML), chronic myeloid leukemia (CML), acute lymphoblastic leukemia (ALL), chronic lymphoblastic leukemia, chronic lymphocytic leukemia, myeloproliferative diseases, multiple myeloma, myelodysplastic syndrome, Hodgkin's lymphoma, non-Hodgkin's lymphoma (malignant lymphoma), Waldenstrom's macroglobulinemia, skin cancer, malignant melanoma, basal cell carcinoma, squamous cell carcinoma, Kaposi's sarcoma, moles dysplastic nevi, lipoma, angioma, dermatofibroma, keloids, psoriasis, adrenal gland cancer, and neuroblastoma.

### PHARMACEUTICAL COMPOSITIONS AND FORMULATIONS

This document also provides pharmaceutical compositions comprising an effective amount of a compound of any one of Formula (I), or Formula (III) disclosed herein, or a pharmaceutically acceptable salt thereof; and a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of the present document include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

The compositions or dosage forms may contain a compound of Formula (I), or Formula (III) described herein in the range of 0.005% to 100% with the balance made up from the suitable pharmaceutically acceptable excipients. The contemplated compositions may contain 0.001%-100% of a compound of Formula (I), or Formula (III) provided herein, in one embodiment 0.1-95%, in another embodiment 75-85%, in a further embodiment 20-80%, wherein the balance may be made up of any pharmaceutically acceptable excipient described herein, or any combination of these excipients.

### Routes of administration and dosage forms

The pharmaceutical compositions of the present document include those suitable for any acceptable route of administration. In some embodiments, the conjugates provided herein are administered by parenteral administration.

Compositions and formulations described herein can be conveniently presented in a unit dosage form, and prepared by any methods well known in the art of pharmacy. See, for example, Remington: The Science and Practice of Pharmacy, Lippincott Williams & Wilkins, Baltimore, MD (20th ed. 2000). Such preparative methods include the step of bringing into association with the molecule to be administered ingredients such as the carrier that constitutes one or more accessory ingredients.

Compositions suitable for parenteral administration include aqueous and non-aqueous sterile injection solutions or infusion solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, saline or 5% dextrose solution, immediately prior to use. Extemporaneous injection solutions and suspensions may be prepared from sterile powders, granules and tablets. The injection solutions may be in the form, for example, of a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

### Dosages and regimens

In the pharmaceutical compositions of the present document, a compound of Formula (I), or Formula (III) disclosed herein is present in an effective amount (e.g., a therapeutically effective amount).

Effective doses may vary, depending on the diseases treated, the severity of the disease, the route of administration, the sex, age and general health condition of the subject, excipient usage, the possibility of co-usage with other therapeutic treatments such as use of other agents and the judgment of the treating physician.

In some embodiments, an effective amount of a compound of Formula (I), or Formula (III) disclosed herein can range, for example, from about 0.001 mg/Kg to about 500 mg/Kg (e.g., from about 0.001 mg/Kg to about 200 mg/Kg; from about 0.01 mg/Kg to about 200 mg/Kg; from about 0.01 mg/Kg to about 150 mg/Kg; from about 0.01 mg/Kg to about 100 mg/Kg; from about 0.01 mg/Kg to about 50 mg/Kg; from about 0.01 mg/Kg to about 10 mg/Kg; from about 0.01 mg/Kg to about 5 mg/Kg; from about 0.01 mg/Kg to about 1 mg/Kg; from about 0.01 mg/Kg to about 0.5 mg/Kg; from about 0.01 mg/Kg to about 0.1 mg/Kg; from about 0. 1 mg/Kg to about 200 mg/Kg; from about 0. 1 mg/Kg to about 150 mg/Kg; from about 0. 1 mg/Kg to about 100 mg/Kg; from about 0.1 mg/Kg to about 50 mg/Kg; from about 0. 1 mg/Kg to about 10 mg/Kg; from about 0. 1 mg/Kg to about 5 mg/Kg; from about 0. 1 mg/Kg to about 1 mg/Kg; from about 0. 1 mg/Kg to about 0.5 mg/Kg).

The foregoing dosages can be administered on a daily basis (e.g., as a single dose or as two or more divided doses, e.g., once daily, twice daily, thrice daily) or non-daily basis (e.g., every other day, every two days, every three days, once weekly, twice weeks, once every two weeks, once a month).

### KITS

This document also includes pharmaceutical kits useful, for example, in the treatment of disorders, diseases and conditions referred to herein, which include one or more containers containing a pharmaceutical composition comprising a therapeutically effective amount of a compound provided herein. Such kits can further include, if desired, one or more of various conventional pharmaceutical kit components, such as, for example, containers with one or more pharmaceutically acceptable carriers, additional containers, etc. Instructions, either as inserts or as labels, indicating quantities of the components to be administered, guidelines for administration, and/or guidelines for mixing the components, can also be included in the kit.

### DEFINITIONS

The term "Cₙ₋ₘ alkyl" includes straight-chain alkyl groups (e.g., methyl, ethyl, propyl, butyl, pentyl, hexyl, heptyl, octyl, nonyl, decyl, *etc*.) and branched-chain alkyl groups (e.g., isopropyl, tert-butyl, isobutyl, *etc*.). In certain embodiments, a straight chain or branched chain alkyl has twelve or fewer carbon atoms in its backbone (e.g., C₁₋₁₂ for straight chain; C₃₋₁₂ for branched chain). For example, the term C₁₋₁₂ includes alkyl groups containing 1 to 12 carbon atoms.

As used herein, the term "Cₙ₋ₘ alkylene", employed alone or in combination with other terms, refers to a divalent alkyl linking group having n to m carbons. Examples of alkylene groups include, but are not limited to, ethan-1,1-diyl, ethan-1,2-diyl, propan-1,1,-diyl, propan-1,3-diyl, propan-1,2-diyl, butan-1,4-diyl, butan-1,3-diyl, butan-1,2-diyl, 2-methyl-propan-1,3-diyl, and the like. In some embodiments, the alkylene moiety contains 2 to 6, 2 to 4, 2 to 3, 1 to 6, 1 to 4, or 1 to 2 carbon atoms.

As used herein, "Cₙ₋ₘ alkenyl" refers to an alkyl group having one or more double carbon-carbon bonds and having n to m carbons. Example alkenyl groups include, but are not limited to, ethenyl, n-propenyl, isopropenyl, n-butenyl, *sec-*butenyl, and the like. In some embodiments, the alkenyl moiety contains 2 to 6, 2 to 4, or 2 to 3 carbon atoms. The term "Cₙ₋ₘ alkenylene" refers to divalent alkenyl linking groups.

As used herein, the term "Cₙ₋ₘ alkoxy", employed alone or in combination with other terms, refers to a group of formula -O-alkyl, wherein the alkyl group has n to m carbons. Example alkoxy groups include, but are not limited to, methoxy, ethoxy, propoxy (e.g., *n*-propoxy and isopropoxy), butoxy (e.g., *n*-butoxy and *tert-*butoxy), and the like. In some embodiments, the alkyl group has 1 to 6, 1 to 4, or 1 to 3 carbon atoms.

As used herein, the term "Cₙ₋ₘ alkylamino" refers to a group of formula -NH(alkyl), wherein the alkyl group has n to m carbon atoms. In some embodiments, the alkyl group has 1 to 6, 1 to 4, or 1 to 3 carbon atoms. Examples of alkylamino groups include, but are not limited to, N-methylamino, N-ethylamino, N-propylamino (e.g., N-(*n*-propyl)amino and N-isopropylamino), N-butylamino (e.g., N-(*n*-butyl)amino and N-(tert-butyl)amino), and the like.

As used herein, the term "di(Cₙ₋ₘ-alkyl)amino" refers to a group of formula - N(alkyl)₂, wherein the two alkyl groups each have, independently, n to m carbon atoms. In some embodiments, each alkyl group independently has 1 to 6, 1 to 4, or 1 to 3 carbon atoms.

As used herein, the term "thio" refers to a group of formula SH.

As used herein, the term "amino" refers to a group of formula -NH₂.

As used herein, the term "carboxy" or "carboxyl" refers to a -C(O)OH group.

As used herein, "halo" or "halogen" refers to F, Cl, Br, or I. In some embodiments, halo is F, Cl, or Br. In some embodiments, halo is F or Cl.

The term "n-membered" where n is an integer, typically describes the number of ring-forming atoms in a moiety where the number of ring-forming atoms is n. For example, piperidinyl is an example of a 6-membered heterocycloalkyl ring, pyrazolyl is an example of a 5-membered heteroaryl ring, pyridyl is an example of a 6-membered heteroaryl ring, and 1,2,3,4-tetrahydro-naphthalene is an example of a 10-membered cycloalkyl group.

As used herein, "cycloalkyl" refers to non-aromatic cyclic hydrocarbons including cyclized alkyl and/or alkenyl groups. Cycloalkyl groups can include mono- or polycyclic (e.g., having 2, 3 or 4 fused rings) groups and spirocycles. Ring-forming carbon atoms of a cycloalkyl group can be optionally substituted by oxo or sulfido (e.g., C(O) or C(S)). Also included in the definition of cycloalkyl are moieties that have one or more aromatic rings fused (i.e., having a bond in common with) to the non-aromatic cyclic hydrocarbon, for example, benzo or thienyl derivatives of cyclopentane, cyclohexane, and the like. A cycloalkyl group containing a fused aromatic ring can be attached through any ring-forming atom including a ring-forming atom of the fused aromatic ring. Cycloalkyl groups can have 3, 4, 5, 6, 7, 8, 9, 10, 11, or 12 ring-forming atoms. In some embodiments, the cycloalkyl is a 3-12 membered monocyclic or bicyclic cycloalkyl. In some embodiments, the cycloalkyl is a C₃₋₇ monocyclic cycloalkyl. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcamyl, cyclooctyl, cyclooctenyl, and the like. In some embodiments, cycloalkyl is cyclopropyl, cyclobutyl, cyclopentyl, cyclohexyl, cyclooctyl, or cyclooctenyl. In some embodiments, the cycloalkyl is a cyclooctenyl ring fused with 1 or 2 benzene rings. In some embodiments, the cycloalkyl is a 3-8 membered or 3-7 membered monocyclic cycloalkyl group (e.g., C₃₋₈ or C₃₋₇ cycloalkyl). In some embodiments, the cycloalkyl is a 8-12-membered bicyclic cycloalkyl. In some embodiments, the cycloalkyl is a 8-16-membered bicyclic or tricyclic cycloalkyl (e.g., C₈₋₁₆ cycloalkyl).

As used herein, "heteroaryl" refers to a monocyclic or polycyclic aromatic heterocycle having at least one heteroatom ring member selected from sulfur, oxygen, and nitrogen. In some embodiments, the heteroaryl ring has 1, 2, 3, or 4 heteroatom ring members independently selected from nitrogen, sulfur and oxygen. In some embodiments, any ring-forming N in a heteroaryl moiety can be an N-oxide. In some embodiments, the heteroaryl is a 5-10 membered monocyclic or bicyclic heteroaryl having 1, 2, 3 or 4 heteroatom ring members independently selected from nitrogen, sulfur and oxygen. In some embodiments, the heteroaryl is a 5-6 membered monocyclic heteroaryl having 1 or 2 heteroatom ring members independently selected from nitrogen, sulfur and oxygen. In some embodiments, the heteroaryl is a five-membered or six-membered heteroaryl ring. A five-membered heteroaryl ring is a heteroaryl with a ring having five ring atoms wherein one or more (e.g., 1, 2, or 3) ring atoms are independently selected from N, O, and S. Exemplary five-membered heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4-oxadiazolyl. A six-membered heteroaryl ring is a heteroaryl with a ring having six ring atoms wherein one or more (e.g., 1, 2, or 3) ring atoms are independently selected from N, O, and S. Exemplary six-membered heteroaryls are pyridyl, pyrazinyl, pyrimidinyl, triazinyl and pyridazinyl. The term "heteroarylene" refers to a divalent heteroaryl linking group.

The term "aromatic" refers to a carbocycle or heterocycle having one or more polyunsaturated rings having aromatic character (i.e., having (4n + 2) delocalized π (pi) electrons where n is an integer).

The term "aliphatic" refers to organic compounds (including polymers) in which carbon atoms and heteroatoms form open chains and which do not contain polyunsaturated rings having aromatic character. Aliphatic compounds may be linear or cyclic, saturated or unsaturated, straight chain or branched.

As used herein, the term "polymer" refers to a macromolecule containing a plurality of repeating subunits.

The term "aryl," employed alone or in combination with other terms, refers to an aromatic hydrocarbon group, which may be monocyclic or polycyclic (e.g., having 2, 3 or 4 fused rings). The term "Cₙ₋ₘ aryl" refers to an aryl group having from n to m ring carbon atoms. Aryl groups include, e.g., phenyl, naphthyl, anthracenyl, phenanthrenyl, indanyl, indenyl and the like. In some embodiments, aryl groups have from 6 to about 20 carbon atoms, from 6 to about 15 carbon atoms, or from 6 to about 10 carbon atoms. In some embodiments, the aryl group is phenyl. The term "arylene" refers to a divalent aryl linking group.

As used herein, "heterocycloalkyl" or "aliphatic heterocycle" refers to non-aromatic monocyclic or polycyclic heterocycles having one or more ring-forming heteroatoms selected from O, N, or S. Included in heterocycloalkyl are monocyclic 4-, 5-, 6-, 7-, 8-, 9- or 10-membered heterocycloalkyl groups. Heterocycloalkyl groups can also include spirocycles. Example heterocycloalkyl groups include pyrrolidin-2-one, 1,3-isoxazolidin-2-one, pyranyl, tetrahydropuran, oxetanyl, azetidinyl, morpholino, thiomorpholino, piperazinyl, tetrahydrofuranyl, tetrahydrothienyl, piperidinyl, pyrrolidinyl, isoxazolidinyl, isothiazolidinyl, pyrazolidinyl, oxazolidinyl, thiazolidinyl, imidazolidinyl, azepanyl, benzazapene, and the like. Ring-forming carbon atoms and heteroatoms of a heterocycloalkyl group can be optionally substituted by oxo or sulfido groups (e.g., C(O), S(O), C(S), or S(O)₂, etc.). The heterocycloalkyl group can be attached through a ring-forming carbon atom or a ring-forming heteroatom. In some embodiments, the heterocycloalkyl group contains 0 to 3 double bonds. In some embodiments, the heterocycloalkyl group contains 0 to 2 double bonds. Also included in the definition of heterocycloalkyl are moieties that have one or more aromatic rings fused (*i.e.*, having a bond in common with) to the non-aromatic heterocycle, for example, benzo or thienyl derivatives of piperidine, morpholine, azepine, etc. A heterocycloalkyl group containing a fused aromatic ring can be attached through any ring-forming atom including a ring-forming atom of the fused aromatic ring. In some embodiments, the heterocycloalkyl is a monocyclic 4 to 6 membered heterocycloalkyl having 1 or 2 heteroatoms independently selected from nitrogen, oxygen, or sulfur and having one or more oxidized ring members. In some embodiments, the heterocycloalkyl is a monocyclic or bicyclic 4 to 10 (written below as 4-10) membered heterocycloalkyl having 1, 2, 3, or 4 heteroatoms independently selected from nitrogen, oxygen, or sulfur and having one or more oxidized ring members. In some embodiments, the heterocycloalkyl is a 8-12 membered heterocycloalkyl (e.g., bicyclic heterocycloalkyl). In some embodiments, the heterocycloalkyl is an 8-16 membered heterocycloalkyl (e.g., bicyclic or tricyclic heterocycloalkyl). In some embodiments, the 8 -12 membered bicyclic heterocycloalkyl is an 8-12 membered fused heterocycloalkylaryl group or an 8-12 membered fused heterocycloalkylheteroaryl group. In some embodiments, the heterocycloalkyl is a 9-12 membered bicyclic heterocycloalkyl. In some embodiments, the 9-10 membered bicyclic heterocycloalkyl is a 9-10 membered fused heterocycloalkylaryl group or a 9-10 membered fused heterocycloalkylheteroaryl group. The term "heterocycloalkylene" refers to a divalent heterocycloalkyl linking group.

As used herein, the term "individual" or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to the amount of active compound or pharmaceutical agent that elicits the biological or medicinal response in a tissue, system, animal, individual, or human that is being sought by a researcher, veterinarian, medical doctor or other clinician.

As used herein the term "treating" or "treatment" refers to 1) inhibiting the disease; for example, inhibiting a disease, condition or disorder in an individual who is experiencing or displaying the pathology or symptomatology of the disease, condition or disorder (*i.e.*, arresting further development of the pathology and/or symptomatology), and/or 2) ameliorating the disease; for example, ameliorating a disease, condition or disorder in an individual who is experiencing or displaying the pathology or symptomatology of the disease, condition or disorder (*i.e.*, reversing the pathology and/or symptomatology).

The terms "protecting group" and "protective group" refer to a moiety that reversibly chemically modifies a functional group in order to obtain chemoselectivity or in order to reduce degradation in one or more subsequent chemical reactions. Suitable protecting groups are well known in the art (e.g., Greene and Wuts, Protective Groups in Organic Synthesis, 3rd Ed., John Wiley & Sons, New York, N.Y., 1999).

The term "leaving group", as used herein, refers to a molecule or a molecular fragment (e.g., an anion) that is displaced in a chemical reaction as a stable species taking with it the bonding electrons. Examples of leaving groups include an arylsulfonyloxy group or an alkylsulfonyloxy group, such as a mesylate or a tosylate group. Common anionic leaving groups also include halides such as Cl-, Br-, and I-.

As used herein, the term "ribose ring system" refers to, e.g., an optionally substituted ribofuranose, arabinofuranose, xylofuranose or lyxofuranose ring system having the following general structure:

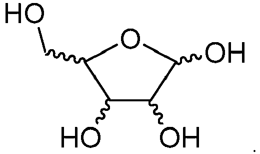

In some embodiments, the ribose ring system comprises a part of an optionally substituted ribonucleoside having the following structure:

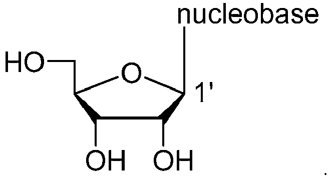

In other embodiments, the ribose ring system comprises a part of an optionally substituted lyxonucleoside having the following structure:

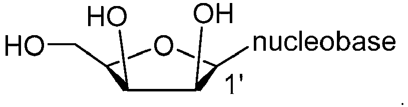

As used herein, the term "self-immolative" refers to a moiety or residue that provides stable bond formation between two groups of a compound or conjugate, but which becomes labile upon activation (e.g., nucleophilic attack) leading to rapid cleavage of the moiety or residue and separation of the two groups. The chemistry of self-immolative groups is described in the literature, for example, in Alouane, A. et al., "Self-immolative spacers: kinetic aspects, structure-property relationships, and applications", Angew. Chem. Int. Ed., 2015, 54, 7492 - 7509; and Kolakowski, R. V. et al., "The methylene alkoxy carbamate self-immolative unit: Utilization of the targeted delivery of alcohol-containing payloads with antibody-drug conjugates", Angew. Chem. Int. Ed., 2016, 55, 7948 - 7951.

As used herein, the term "optionally substituted" refers to a group (e.g., alkyl group, cycloalkyl group, alkylene group, aryl group, heteroaryl group, and the like), where one or more hydrogens on the designated atom, usually a carbon, oxygen, or nitrogen atom, may be replaced with a designated substituent, provided that the designated atom's normal valency is not exceeded, and that the substitution results in a stable compound. When a substituent is keto or oxo (*i.e.*, =O), then 2 hydrogens on the atom are replaced. The one or more substituents can be independently selected from C₁₋₆ alkyl, C₁₋₄ haloalkyl, C₃₋₇ cycloalkyl, C₂₋₆ alkenyl, C₂₋₆ alkynyl, C₆₋₁₀ aryl, 4 to 7 membered heterocycloalkyl, substituted 5- to 14-membered heteroaryl, halo, CN, NO₂, OR^{a}, SR^{a}, C(O)R^{a}, C(O)NR^{a}R^{a}, C(O)OR^{a}, OC(O)R^{a}, OC(O)NR^{a}R^{a}, N₃, NHR^{a}, NR^{a}R^{a}, NR^{a}C(O)R^{a}, NR^{a}C(O)OR^{a}, NR^{a}C(O)NR^{a}R^{a}, NR^{a}S(O)₂R^{a}, S(O)₂R^{a}, and S(O)₂NR^{a}R^{a}, wherein each R^{a} is independently selected from H, C₁₋₆ alkyl, C₃₋₇ cycloalkyl, C₆₋₁₀ aryl, 4 to 7 membered heterocycloalkyl, substituted 5- to 14-membered heteroaryl, C₁₋₄ haloalkyl, C₂₋₆ alkenyl and C₂₋₆ alkynyl.

In some embodiments, the one or more optional substituents are selected from C₁₋₆ alkyl, C₁₋₄ haloalkyl, OH, NO₂, CN, and acetyl.

In some embodiments, the optional substituent is SH.

In some embodiments, the optional substituent is an azide (N₃).

In some embodiments, the optional substituent is a group of formula:

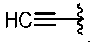

In some embodiments, the optional substituent is a maleimide of formula:

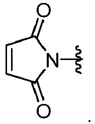

In some embodiments, the optional substituent is a cyclooctyne, such as dibenzocyclooctyne (DBCO), difluorobenzocyclooctyne (DIFBO), biarylazacyclooctynone (BARAC), dibenzocyclooctyne (DIBO), difluorinated cyclooctyne (DIFO), monofluorinated cyclooctyne (MOFO), dimethoxyazacyclooctyne (DIMAC), or aryl-less octyne (ALO), each of which is optionally substituted with 1, 2, 3, 4 or 5 optional substituents described herein.

In some embodiments, the optional substituent is a cyclooctyne selected from the group consisting of:

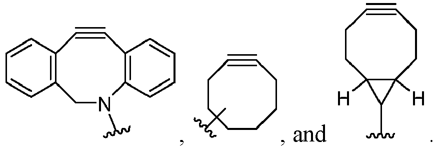

As used herein, the term "about" is meant to account for variations due to experimental error. As used herein, the singular forms "a," "an," and "the" include plural referents unless the context clearly dictates otherwise.

As used herein, the term antibody refers to an antibody, or antibody derived or related molecule such as an antibody fragment, bi- or tri-specific antibody, single chain antibody, nanobody, etc. The chemical approaches used herein to couple a pharmaceutical agent to a targeting antibody agent can readily be used with other protein targeting agents (e.g. lectins, aptamers, or non-antibody proteins that bind to specific cell surface receptors) or ligands that contain reactive groups similar to proteins (see references provided herein).

### EXAMPLES

### Example 1

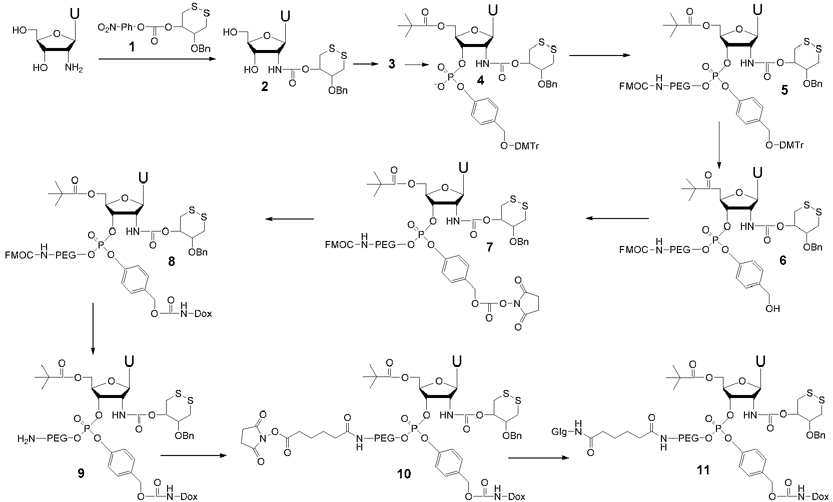

### Compound 1

(±)-Trans-5-benzyloxy-1,2-dithiane-4-ol (Urata. H. et al. BMCL 27, 2017, 3135-3138) (1.5 g, 6.2 mmol) was dissolved in dry dichloromethane (DCM) (95 mL) followed by addition of dry pyridine (2.5 mL, 31 mmol) and the solution was cooled to 0°C.

4-Nitrophenylchloroformate (1.87 g, 9.3 mmol) was dissolved in dry DCM (10 mL) and the solution was added dropwise to the solution of dithianol. The resulting mixture was stirred for 30 min. The mixture in DCM was extracted with 1) water 2) saturated aqueous sodium bicarbonate (2x), 3) 0.5M citric acid and 4) brine. The organic phase was filtered through Na₂SO₄, evaporated to dryness and the residue was co-evaporated with toluene. The residue solidified while kept under vacuum. The material was practically pure product 4-nitrophenyl-[(±)-trans-5-benzyloxy-1,2-dithiane-4-yl]carbonate **1.**

### Compound 2

4-Nitrophenyl-[(±)-trans-5-benzyloxy-1,2-dithiane-4-yl]carbonate 1 (2.3 g, 5.5 mmol) was dissolved in dry dimethylformamide (DMF) (10 mL) and the solution was added dropwise with a syringe to a solution of amino-uridine (1.23 g, 5 mmol) in dry DMF (50 mL). The mixture was stirred overnight at room temperature (RT) and then evaporated to dryness, co-evaporated with toluene, and the residue was flash chromatographed through silica gel 60A with methanol methanol/dichloromethane gradient (2-4-7%MeOH) to afford the title product 2. Yield (2 g, 73%).

### Compound 3

Compound **2** (2 g, 3.9 mmol) was dissolved in dry pyridine (8 mL) and cooled to 0°C. Pivaloyl chloride (0.5 mL, 4 mmol), dissolved in dichloromethane (8 mL), was slowly added dropwise with vigorous stirring and the mixture was stirred for 5-10 minutes at 0°C, and then stirred at RT overnight. The reaction mixture was quenched with methanol, concentrated on rotary evaporator, and the concentrated solution partitioned between dichloromethane and saturated aqueous sodium bicarbonate. The organic phase was evaporated and the residue co-evaporated with toluene. The residue was then chromatographed through silica gel 60A with MeOH/DCM gradient (2-3 % MeOH) to afford the title product 3. Yield (1.7 g, 75%).

### Compound 4

To an acetonitrile solution (0.2 M) of phosphoryl-tris-triazolide (obtained following literature procedure - Tetrahedron Letters, 21, 1980, 2935-2936 with slight modification) (11 mL, 2.2 mmol), dry 4-(DMTr-methyloxy)phenol (Iyer, R. P. et al. Tetrahedron Letters, 2001, 42, 3669-3672) (1.2 g, 2 mmol) dissolved in pyridine (3 mL), was added. The reaction mixture was stirred at RT for 60 min followed by addition of compound **3** (1.0 g, 1.74 mmol). The second step of this reaction proceeded for 15 hours and the reaction mixture was quenched by addition of 1M triethylammonium bicarbonate solution (with pH adjusted to 7.5 - 8.0). The mixture was extracted with dichloromethane, the organic phase was evaporated and the residue co-evaporated with toluene. The residue was flash chromatographed on silica gel 60A with MeOH/DCM gradient (2-15 % MeOH) containing pyridine (0.2 %) to afford the phosphodiester **4** as white foam in a form of the triethylammonium salt. Yield (1.7 g, 70%).

### Compound 5

Phosphodiester **4** (0.6 g, 0.5 mmol), FmocNHPEGOH* (average mw ~700 Da, 0.74 g, 1 mmol) and N-methylimidazole (0.33 mL, 4 mmol) were dried by coevaporation with acetonitrile (3x5 mL), and dissolved in dry acetonitrile (5 mL). Mesitylenesulfonylchloride (0.44 g, 2 mmol) was dissolved in 1 mL dry acetonitrile and added dropwise. The reaction mixture was stirred at RT for 3 hours, quenched by addition of water, concentrated on rotary evaporator, and the concentrate partitioned between dichloromethane and saturated aqueous sodium bicarbonate. The organic phase was evaporated and the residue co-evaporated with toluene. The residue was separated using preparative RP-HPLC chromatography, using 0.1M triethylammonium acetate buffer pH 7.0 / acetonitrile gradient system. Yield of pure product **5:** 0.4 g, (45%).
*This FMOC protected amino-PEG-linker was prepared from MMTr-aminoethoxy)ethyl]tosylate and mono sodium salt of PEG400 to generate the MMTrNH-PEGOH. This product was detritylated and the amino group was protected by an FMOC group. (Modified procedures of Lazaro, R. et al. Letters in Peptide Science, 4, 1997, 455-461 and Livingston, A.G. et al. Nature Chemistry, 2018**).**

### Compound 6

Purified compound **5** (200 mg , 0.11 mmol) was dissolved in 80% acetic acid (AcOH) (15 mL) and stirred for 2 hours at RT. The volatiles were evaporated and the residue was co-evaporated with toluene. The residue was flash chromatographed on silica gel 60A with methanol/dichloromethane gradient (0 - 6 % ) to afford the title product **6.** Yield (138 mg, 85%).

### Compound 7

The hydroxy compound **6** (100 mg , 68 µmol) was dried by co-evaporation with toluene (2 x 10 mL), dissolved in dichloromethane (4 mL), and 1.4 M phosgene in toluene (5 mL) was added. The mixture was stirred at RT for 3 hours and then all volatile materials were evaporated. The residue was co-evaporated with dry toluene three times to give the crude chloroformate.

This crude chloroformate was dissolved in dry tetrahydrofuran (THF) (5 mL) and dry DCM (2 mL) followed by addition of N-hydroxysuccinimide (78 mg, 0.68 mmol, 10 eq.) and dry pyridine (0.27 mL, 3.4 mmol, 50 eq.). This mixture was stirred at RT for 2 hours. All volatile materials were evaporated and the residue was co-evaporated with toluene. The residue was triturated three times with diethyl ether (3 x 10 mL) and the residue was dried under vacuum and used in the next step (title product 7) without additional purification.

### Compound 8

To a 10 mL, round-bottom (RB) glass flask containing a magnetic stir bar was sequentially added anhydrous dimethylformamide (DMF) (2.5 mL), doxorubicin HCl salt (25 mg, 0.043 mmol), and diisopropylethylamine (DIPEA) (38 µL, 0.19 mmol). The reaction flask was wrapped with aluminum foil and the reaction mixture stirred at room temperature under nitrogen for 30 min.

The DMF and excess DIPEA were evaporated on rotavapor and the residue was redissolved in anhydrous DMF (2.5 mL). To this solution, a solution of the NHS-carbonate 7 (104 mg, 0.064 mmol in dry DMF, 0.75 mL) was added. The reaction flask was wrapped with aluminum foil and the reaction was kept under nitrogen and monitored by TLC (dichloromethane/methanol/acetic acid, 6/2/2, v/v/v). After 2 - 3 hours at room temperature, the solvent was removed under high vacuum at 30 - 35°C. The purification of the crude product was done with RP HPLC using 0.1M triethylammonium acetate pH 7.0/acetonitrile, and the distinct UV product peak **8** was collected, evaporated and coevaporated with toluene to obtain 24 mg (30%) of reddish material.

### Compound 9

Compound **8** (20 mg, 10 µmol) was dissolved in dry DMF (0.25 mL) followed by addition of dry triethylamine (70 µL, 0.5 mmol), and the mixture was stirred for 12 hours at room temperature in darkness. The volatiles were removed by rotavap and the residue was subjected to RP HPLC purification with the neutral eluent mentioned in the process of preparing Compound **8** to yield product **9** as a dry reddish solid after co-evaporations with toluene. 14 mg (75%).

### Compound 10

Compound **9** (10 mg, 5.3 µmol) was dissolved in anhydrous DMF (0.2 mL), This solution was added slowly over a minute into a solution of dry DMF (0.2 mL) containing adipic acid bis(hydroxysuccinimidyl) ester (9 mg, 26 µmol) and diisopropylethylamine (5 µL, 26 µmol). The reaction mixture was stirred under nitrogen in darkness at room temperature and progress was monitored by TLC (dichloromethane/methanol/acetic acid, 6 / 2 / 2, v/v/v). After 2 hours, the volatiles were removed with rotavap under high vacuum at 30 - 35°C. The residue was triturated with diethyl ether three times and the insoluble material was dried under vacuum to provide crude product **10** that was used without further purification in the next step.

### Compound 11

Human serum IgG (10 mg, 67 nmol,) was dissolved in HEPES buffer, 0.1M, pH 7.4 (2 mL (34uM)). To this solution, a solution of doxorubicin-mono-NHS ester **9** (0.67 µmol, 1.5 mg, 10 equiv. in DMF (0.2 mL). DMF total concentration was 9%) was added.. The mixture was kept at room temperature with occasional shaking for 6 hours. The mixture was evaporated to dryness and the antibody containing ADC product **11** was separated from unreacted reagent by SEC chromatography.

### Example 2

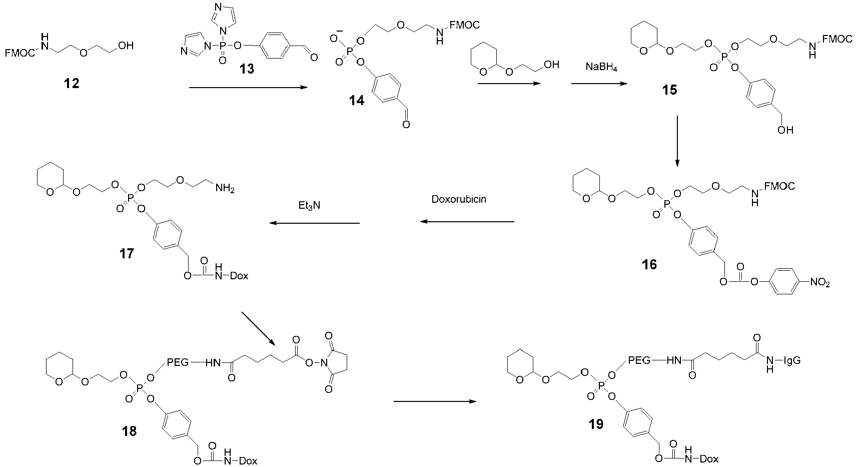

### Compound 13

4-Formylphenyl phosphorodichloridate, prepared and distilled according to McMillen et al. Biochemistry, 25, 1, 1986, 183, (1.91 g, 8 mmol) was dissolved in dry acetonitrile (35 mL) in a dried 50 mL Falcon tube, and then dried 1,2,4-triazole (1.22 g, 17.6 mmol) was added. To the stirred suspension, triethylamine (2.23 mL, 16 mmol) was added dropwise, and the mixture was stirred for 1 hour at RT. The suspension was rapidly filtered and the clear slightly orange 0.2 M solution of the bis-triazolide **13** was collected into a round bottomed flask.

### Compound 14

N-FMOC [2-(2-hydroxyethoxy)ethyl]carbamate **12,** was preparred conveniently from 2-(2-hydroxyethoxy)ethylamine (1.5 mol eq) and FMOC-Cl (1.0 mol eq) in saturated sodium bicarbonate (5 mL/mmol) and THF (2 mL/mmol), acidification until pH 2, extraction of the product by dichloromethane, evaporation and crystallization of the pure material from small amount of toluene. To a solution of **12** (1.61 g, 4.9 mmol) in dry pyridine (10 mL), bis-triazolide **13** (37 mL, 7.4 mmol) was added in a single portion, and the reaction mixture was stirred for 1 hour at RT. The reaction was quenched with 1M triethylammonium bicarbonate (5 mL) and the mixture was partitioned between dichloromethane and 1 M triethylammonium bicarbonate. The organic phase was evaporated; residue co-evaporated with toluene and the product **14** was isolated by flash-chromatography on silica gel 60A with methanol / dichloromethane gradient. Yield 2.15 g (85 %).

### Compound 15

Phosphodiester **14** (2.0 g, 3.18 mmol), 2-(tetrahydro-2*H*-pyran-2-yloxy)ethanol (0.94 g, 6.4 mmol) and N-methylimidazole (2.1 g, 25.5 mmol) were dried by coevaporation with dry acetonitrile (30 mL), then dissolved in dry acetonitrile (20 mL). Mesitylene-sulfonylchloride (2.8 g, 12.7 mmol) was added in one portion. The reaction solution was stirred at RT for 3 hours. The reaction was quenched with water and concentrated on a rotary evaporator and the concentrate was partitioned between dichloromethane and saturated aqueous sodium bicarbonate.

The organic phase was evaporated, the residue was coevaporated with toluene and dissolved in methanol (30 mL). To this magnetically stirred solution at RT, solid sodium borohydride (0.15 g, 4 mmol) was added in a single portion. The extensive gas evolution stopped after 30 seconds and after 60 seconds the mixture was partitioned between saturated sodium bicarbonate and dichloromethane. The organic phase was collected, evaporated and coevaporated with acetonitrile. The residual material was purified by preparative RP-HPLC to afford the title product **15** after removal of residual TEAA at low pressure. Total yield of **15** calculated from **14** (1.27 g, 61 %).

### Compound 16

Compound **15** (1.15 g, 1.7 mmol) was dissolved in dry DCM (15 mL) followed by addition of dry pyridine (0.69 mL, 8.5 mmol) and the solution was cooled to 0°C. 4-nitrophenyl chloroformate (0.5 g, 2.55 mmol), dissolved in dry DCM (5 mL), was added dropwise to the solution of **15,** and the resulting mixture was stirred for 30 minutes. The mixture was extracted with 1) water, 2) saturated aqueous NaHCO₃ (2x), and 3) brine. The organic phase was filtered through Na₂SO₄, evaporated to dryness and the residue was coevaporated with toluene and was dried under vacuum. The material was practically pure product **16.**

### Compound 17

To a 25 mL, round-bottom (RB) glass flask containing a magnetic stir bar was sequentially added anhydrous DMF (3 mL), doxorubicin HCl salt (30 mg, 0.052 mmol), and diisopropylethylamine (45 µL, 0.26 mmol). The reaction flask was kept in darkness and the reaction mixture stirred at room temperature under nitrogen for 30 minutes.
The DMF and excess DIPEA were evaporated on a rotary evaporator and the residue was redissolved in anhydrous DMF (3 mL). To this solution, a solution of the 4-nitrophenyl carbonate **16** (63 mg, 0.078 mmol in dry DMF, 0.75 mL) was added. The reaction flask was kept in darkness and the reaction was kept under nitrogen and monitored by RP-HPLC using a triethylammonium acetate buffer system. After overnight reaction at room temperature, the solvent was removed under high vacuum at 30 - 35°C.

Purification of the crude product was done with RP-HPLC using 0.1 M triethylammonium acetate pH 7.0/acetonitrile, and a distinct UV (485 nm) product peak was collected, evaporated, and coevaporated with toluene to obtain 24 mg of reddish material.

The obtained doxorubicin-conjugate (20 mg, 10 µmol) was dissolved in dry DMF (0.25 mL) followed by addition of dry triethylamine (70 µL, 0.5 mmol), and the mixture was stirred for 12 hours at room temperature in darkness. The volatiles were removed by rotary evaporator and the residue was subjected to RP-HPLC purification with the neutral eluent mentioned above to yield the amino-product **17** as a dry reddish solid after co-evaporations with toluene. Yield 14 mg.

### Compound 18

In a 5 mL RB glass flask, compound **17** (10 mg, 10 µmol) was dissolved in anhydrous DMF (0.4 mL), and then diisopropylethylamine (14 µL, 80 µmol) was added. The mixture was stirred at room temperature in darkness under nitrogen for 30 minutes. This solution was added dropwise over a few minutes into a solution of dry DMF (0.4 mL) containing adipic acid bis(hydroxysuccinimidyl) ester (17 mg, 50 µmol) and diisopropylethylamine (10 µL, 50 µmol). The reaction mixture was stirred in darkness at room temperature and progress was monitored by RP-HPLC using a TFA buffer system (A: 5% acetonitrile / 0.1%TFA. B: 0.1%TFA in acetonitrile). After 4 hours, the volatiles were removed rotary evaporator under high vacuum at 30 - 35°C. The residue was triturated with diethyl ether three times and the insoluble material was dried under vacuum to provide crude product 18 that was used without further purification in the next step.

### Compound 19

Human serum IgG (10 mg, 67 nmol) was dissolved in HEPES buffer, 0.1 M, pH 7.4 (2 mL (34 µM)). To this solution, a solution of doxorubicin-mono-NHS ester **18** (0.67 µmol, 10 equiv. in DMF (0.2 mL). DMF total concentration was 9% was added. The mixture was kept at room temperature with occasional shaking for 6 hours. The mixture was evaporated to dryness and the ADC conjugate **19** was isolated by SEC chromatography on Zorbax GF-250 HPLC-column.

The chemical structure and functionality was evidenced by treatment of a pure, high molecular conjugate with 10 % acetic acid (14 hours), removal of acid, and neutralization of the residual material until pH 7.4, resulting in quantitative formation of free doxorubicin.

### Example 3

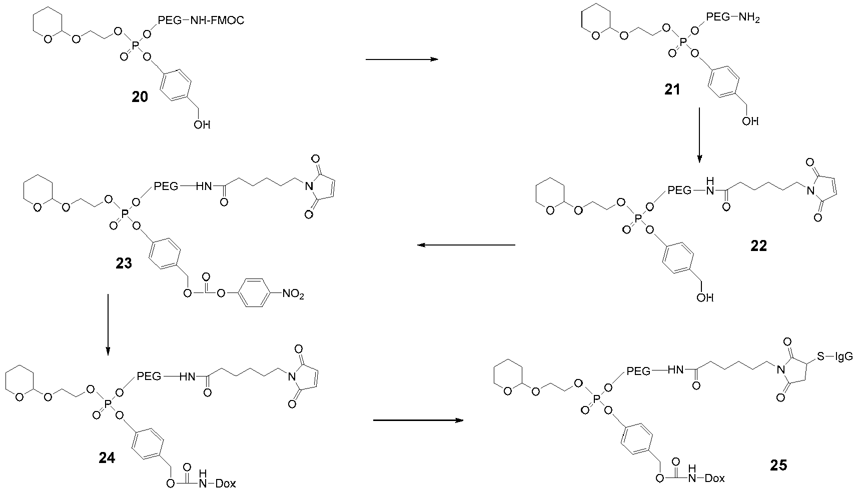

### Compound 20

A phosphotriester **20** was obtained in a manner similar to what was described for compound **15** in Example 2, with exception that FMOC-NH-PEG500 was used instead of a short diethylene glycol analogue, and the order of incorporation of the acid labile trigger and the PEG was switched. The benzaldehyde phosphotriester was reduced to benzalcohol by means of borohydride as previously described and the final compound **20** was obtained after silica gel flash chromatography. Yield 49% (3 steps).

### Compound 21

Compound **20** (1.22 g, 1.13 mmol), dissolved in dry acetonitrile (5 mL), was treated with diisopropylethylamine (2 mL) at RT for 2 days. The TLC analysis showed complete consumption of starting material and all volatile matters were evaporated. The residual material was dissolved in a minimal volume of DCM followed by addition of petroleum ether (40 mL) causing precipitation **of 21** in the form of an oil and separation of liberated dibenzofulvene. The product was isolated by centrifugation and vacuum drying. Yield 0.87 g (92%).

### Compound 22

Commercial N-succinamide 6-maleimidohexanoate (0.41 g, 1.34 mmol) was added to compound **21** (0.75 g, 0.89 mmol) dissolved in dry pyridine (10 mL). The reaction mixture was stirred at RT for 6 hr and it was partitioned between saturated sodium bicarbonate and dichloromethane. The organic phase was evaporated, dried by coevaporation with toluene, and the product **22** was isolated after short silica gel column chromatography. Yield 0.59 g, 63%.

### Compound 23

Phosphotriester **22** (0.40 g, 0.42 mmol) was converted to nitrophenyl carbonate **23** following the methodology described in Example 2. The product was obtained in the form of an oil with practically quantitative yield.

### Compound 24

Doxorubicine hydrochloride (20 mg, 0.035 mmol) in dry DMF (2 mL) was deprotonated by addition of diisopropylethylamine and evaporated to dryness. The residue was redissolved in dry DMF (1 mL) and active ester **23** (80 mg, 0.07 mmol), dissolved in DMF (0.75 mL), was added followed by dry triethylamine (15 mL, 0.1 mmol). The mixture was stirred at RT for 14 hours, evaporated and quenched by addition of water / pyridine (1:9 v/v). After 60 minutes all volatile matters were evaporated, the reaction mixture dissolved in acetonitrile / water 7:3, and the product **24** isolated by preparative RP HPLC using TEAA/MeCN gradient and following the deep red product at 485 nm absorbance, clearly separated from the starting doxorubicin. Yield (based on HPLC) ca.70%.

### Compound 25

Maleimide derivative **24** was conjugated to human IgG following essentially the literature procedure of B. A. Mendelsohn et al., Bioconjugate Chemistry, 2017, 28, 371-381 to prepare compound **25.** The reaction mixture was desalted using a PD 10 desalting column and analysed by Zorbax GF-250 gel filtration HPLC column. A sample of isolated material was treated with 10% acetic acid for 16 hours at RT and the mixture was concentrated on a SpeedVac vacuum concentrator. The residual liquid was brought to pH 7.4 by addition of an excess of TRIS buffer and after 60 minutes this mixture was analyzed again on the same Zorbax column to determine that all 485 nm absorbing material was limited to low molecular weight components. Reverse phase HPLC analyze evidenced that this component is indeed the starting doxorubicin.

### Example 4

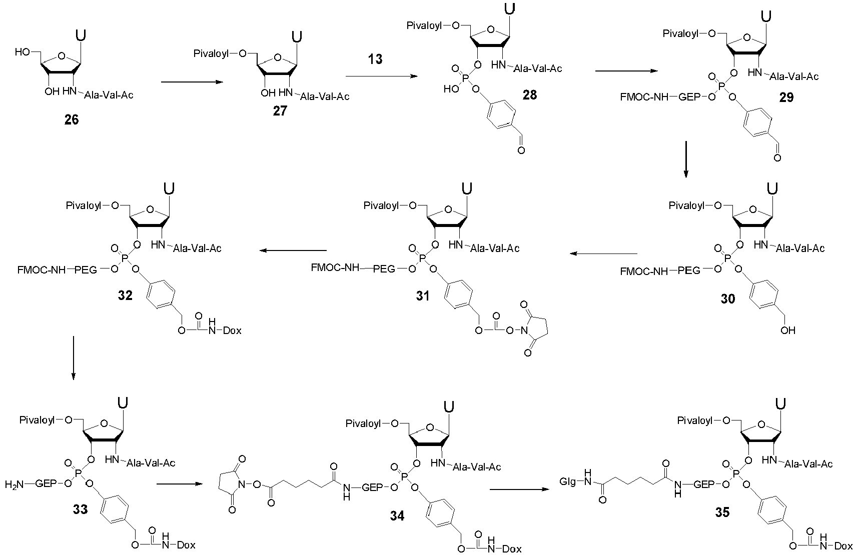

### Compound 26

N-Ala-Val-Ac substituted 2'-amino uridine was prepared by solution phase sequential addition of N-FMOC aminoacyl chloride (1.3 molar equivqlent) (prepared freshly before each addition) dissolved in dry THF, to the vigorously stirred solution of 2'-aminouridine in sodium bicarbonate 1 M. Each intermediate was purified by silica gel chromatography and the FMOC group was removed upon treatment with diisopropyl-ethylamine. Final removal of FMOC was followed by acetylation with acetic anhydride in sodium bicarbonate 1 M, and the pure 2'-N-sustituted uridine derivative **26** was obtained after preparative RP column chromatography with a total yield of 37 %.

### Compound 27

Pivaloylation of compound **26** (2 g, 4.5 mmol) using the same procedure as in Example 1, gave compound **27** (1.9 g) in 77% yield.

### Compound 28

A 0.2 M acetonitrile solution of 4-formylphenylphosphorobistriazolide **13** (Example 2) (18 mL, 3.6 mmol) was added to a RB flask, and with stirring, a pyridine solution of compound **27** (1.85 g, 3.4 mmol / 6 mL pyridine) was added dropwise. The reaction mixture was then stirred for 2 hours at RT. The reaction was quenched with 1 M triethylammonium bicarbonate (1 mL) and the mixture was partitioned between dichloromethane and 1 M triethylammonium bicarbonate. The organic phase was evaporated, the residue co-evaporated with toluene, and the product **28** was isolated by flash-chromatography on silica gel 60A with a methanol / dichloromethane gradient. Yield 1.8 g (73%).

### Compound 29

Phosphodiester **28** (1.75 g, 2.4 mmol), FMOC-NH-PEG₅₀₀OH (3.4 g, 4.8 mmol) and N-methylimidazole (1.5 mL, 19.2 mmol) were dried by coevaporation with acetonitrile (2 x 50 mL), then dissolved in dry acetonitrile (25 mL). Mesitylenesulfonylchloride (2.1 g, 9.6 mmol) was added in one portion with vigorous stirring. The reaction solution was stirred at RT for 3 hours. The reaction was quenched with water and concentrated on a rotary evaporator. The concentrate was then partitioned between dichloromethane and saturated aqueous sodium bicarbonate. The organic phase was evaporated and the residue co-evaporated with toluene. The residue was then flash chromatographed through silica gel 60A with ethanol / dichloromethane gradient (0 - 2% ethanol) to afford the title product **29.** Yield (2.4 g, 70%).

### Compound 30

Phosphotriester **29** (2.3 g, 1.6 mmol) was dissolved in methanol (20 mL). Solid NaBH₄ (300 mg, 8 mmol) was added in one portion with stirring. After the gas evolution stopped (15-30 seconds), the mixture was poured into a saturated aqueous sodium bicarbonate solution and extracted with dichloromethane. The organic phase was evaporated and the residue co-evaporated with toluene. The residue was chromatographed through silica gel 60A with ethanol / dichloromethane gradient (0 - 4% ethanol) to afford the title product **30.** Yield (2 g, 90%).

### Compound 31

The hydroxyl block **30** (1.9 g , 1.34 mmol) was dried by co-evaporation with toluene (2 x 50 mL) and dissolved in dry dichloromethane (15 mL). To this solution, 1.4 M phosgene in toluene (20 mL) was added. The mixture was stirred at RT for 3 hours followed by evaporation of all volatile materials. The residue was co-evaporated with dry toluene three times to give the crude chloroformate.

This crude chloroformate was dissolved in dry THF (30 mL) and dry DCM (10 mL), followed by addition of N-hydroxysuccinimide (0.75 g, 6.7 mmol, 5 eq.) and dry pyridine (5.4 mL, 67 mmol, 50 eq.), The resulting mixture was stirred at RT for 2 hours. All volatile materials were evaporated and the product residue was co-evaporated with toluene. The residue was triturated three times with diethyl ether (3x50 mL) and the residue was dried under vacuum to provide crude product **31** and used as such in the next step.

### Compound 32

To a 25 mL round-bottom (RB) glass flask containing a magnetic stir bar was sequentially added anhydrous DMF (5 mL), doxorubicin HCl salt (50 mg, 0.086 mmol, 1eq), and diisopropylethylamine (75 µL, 0.38 mmol, 5 eq). The reaction mixture was stirred at room temperature for 30 minutes.

The DMF and excess DIPEA were removed via evaporation on a rotary evaporator and the residue was redissolved in anhydrous DMF (5 mL). To this solution, a solution of the NHS-carbonate **31** (210 mg, 0.13 mmol, 1.5eq, in dry DMF, 2 mL) was added. After overnight reaction at room temperature, the solvent was removed under high vacuum at 30 - 35°C.

The purification of the crude product was performed using RP-HPLC with 0.1M triethylammonium acetate pH 7.0/acetonitrile, and the distinct UV (485 nm) product peak **32** was collected, evaporated and coevaporated with toluene to obtain 45 mg (26%) of reddish material.

### Compound 33

Compound **32** (30 mg, 15 µmol) was dissolved in dry DMF (0.4 mL) followed by addition of dry triethylamine (105 µL, 0.75 mmol) and the mixture was stirred for 12 hours at room temperature in darkness. The volatiles were removed by rotary evaporator and the residue was subjected to RP HPLC purification with the neutral eluent used to isolate Compound **8** to yield product **33** as a dry reddish solid after co-evaporations with toluene. Yield 19 mg (70%).

### Compound 34

In a 5 mL RB glass flask, the acetate salt of compound **33** (10 mg, 5.5 µmol) was dissolved in anhydrous DMF (0.4 mL), and then diisopropylethylamine (8 µL, 44 µmol) was added. The mixture was stirred at room temperature in darkness for 30 minutes. This solution was added dropwise over a few minutes into a solution of dry DMF (0.4 mL) containing adipic acid bis(hydroxysuccinimidyl) ester (9 mg, 30 µmol) and diisopropylethylamine (8 µL, 44 µmol). The reaction mixture was stirred in darkness at room temperature and progress was monitored by RP-HPLC using a TFA buffer system (A: 5% acetonitrile / 0.1%TFA. B: 0.1%TFA in acetonitrile). After 4 hours, the volatiles were removed by rotary evaporator under high vacuum at 30 - 35°C. The residue was triturated with diethyl ether three times and the insoluble material was dried under vacuum to provide crude product **34,** which was used without further purification in the next step.

### Compound 35

Human serum IgG (10 mg, 67 nmol,) was dissolved in HEPES buffer, 0.1 M, pH 7.4 (2 mL (34 µM)). To this solution, a solution of doxorubicin-mono-NHS ester **34** (137 mg, 0.67 µmol, 10 equiv. in DMF (0.5 mL) (DMF total concentration was 9%) was added. The mixture was kept at room temperature with occasional shaking for 6 hours. The mixture was evaporated to dryness and the ADC conjugate **35** was isolated by SEC chromatography on Zorbax GF-250 HPLC-column.

The chemical structure and functionality was evidenced by treatment of a pure, high molecular conjugate with 10% acetic acid (14 hours), removal of acid, and neutralization of the residual material until pH 7.4, resulting in quantitative formation of free doxorubicin.

### Example 5

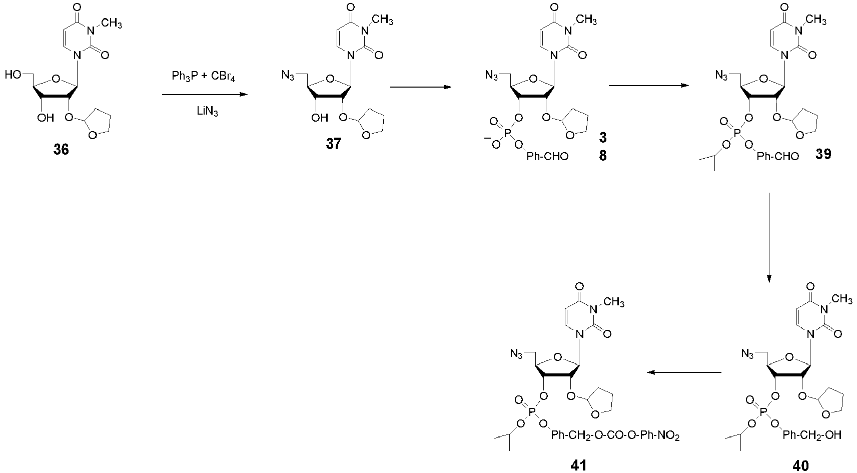

### Compound 36

Uridine (5.0g, 20.5 mmol) was suspended in dry methanol (100 mL) and evaporated after addition of toluene (200 mL). This water-free material was dissolved in dry THF (75 mL) and dry methanol (8 mL) and triphenyl phosphine (8 g, 30 mmol) was added followed by diisopropylazodicarboxylate (6.26 g, 31 mmol). The mixture was stirred at RT for 60 minutes and TLC analysis showed complete disapperance of the uridine starting material and formation of a material with slightly higher Rf. All volatile material was evaporated and the residual oil was coevaporated with dry pyridine at 60°C. The final oil was dissolved in dry pyridine (100 mL) and 1,3-dichloro -1,1,3,3-tetraisopropyldisiloxane (7.8 g, 24.7 mmol) was added. The reaction mixture was stirred at RT for 12 hours and quenched by addition of methanol. The mixture was then partitioned between saturated sodium bicarbonate and dichloromethane. The organic extracts were combined and evaporated. The residue was dried by coevaporation with toluene, the crystallized triphenylphosphine oxide was filtered out, and the 5',3'-tetraisopropyldisyloxyl N3-methyl uridine was briefly purified on a large silica gel column. The collected material was dried by coevaporation with toluene, dissolved in dioxane (50 mL) followed by addition of 2,3-dihydrofuran (14 g, 0.2 mol) and benzenesulfonic acid (0.16 g, 1.0 mmol). The mixture was stirred at RT for 6 hours and the acid was neutralized by addition of saturated sodium bicarbonate solution, followed by extraction with dichloromethane and evaporation. The residual material was dissolved in methanol (200 mL) and solid ammonium fluoride (6 g, 0.16 mol) was added. The suspension was magnetically stirred for 16 hours until complete disapperance of high-Rf material and formation of compound **36.** The mixture was evaporated, dried by coevaporation with toluene, dissolved in dichloromethane and filtered. The filtered material was concentrated and purified by silica gel chromatography. Evaporated fractions containing **36** yielded material in a form of a foam (4.2 g, 63% total yield).

### Compound 37

The 2'-O-thf- N3-methyl-uridine **36** (3.0 g, 9.15 mmol) was converted to its 5'-azido derivative **37** in a modified Mitsunobu reaction using the method described in T. Hata et al., J. C. S. Perkin I, 1980, 306-310. Compound **37** was obtained as an oil (2.29 g, 71%).

### Compound 38

Nucleoside **37** (1.5 g, 4.2 mmol) in dry pyridine (20 mL) was reacted with bis-triazolide **13** (0.2 M in MeCN) (40 mL, 8.0 mmol) for 2 hours at RT. Excess triazolide was hydrolyzed by addition of 1 M triethylammonium bicarbonate and after 10 minutes the product was extracted into dichloromethane. Silica gel column chromatography yielded triethylammonium salt of **38** as a colorless oil. Yield 2.25 g, 82%.

### Compound 39

Phosphodiester **38** (2.0 g, 3.06 mmol) was converted to phosphotriester **39** by means of a standard Efimov reaction, as it was earlier described in Example 2, with exception that here isopropanol was used as the hydroxyl component. Other alcohols, such as MeO-PEG-OH could be used, for example, to prepare a phosphotriester exhibiting improved water solubility properties.

The product **39** was isolated after short silica gel chromatography. Yield 1.20 g, 66%.

### Compound 40

Aldehyde phosphotriester **39** (1.1 g, 1.85 mmol), dissolved in methanol (15 mL), was reduced to the benzyl alcohol product **40** by addition of solid sodium borohydride (2 eq) under vigorus stirring. The vigorus reaction decayed after 30 seconds and the mixture was partitioned between a bicarbonate solution and dichloromethane, yielding a TLC pure material without sign of azido group reduction. Yield quantitative.

### Compound 41

The hydroxyl group of compound **40** (1.0 g, 1.67 mmol) was activated by reation with p-nitrophenyl chloroformate, according to the standardized procedures described in Example 2. After work-up, the product **41** was essentially pure. It was evaporated, coevaporated with toluene and dried under low pressure.

### Example 6

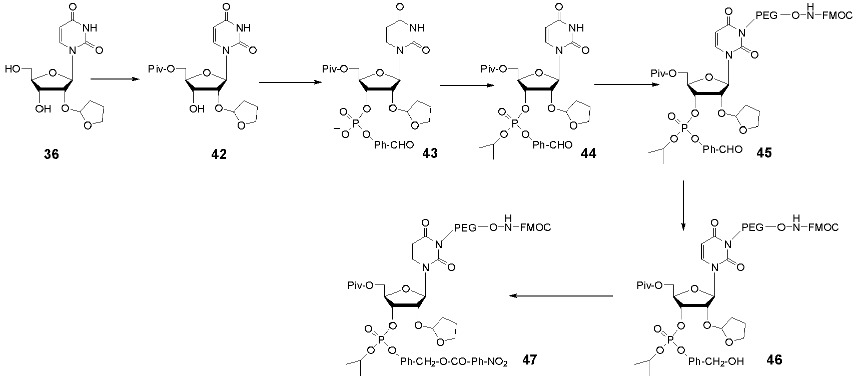

### Compound 44

### Synthesis of 2'-O-thf phosphotriester 44.

This synthesis started with 2'-O-thf uridine **36,** which is a well-known derivative of uridine. It was obtained from 1,1,3,3-tetraisopropyldisiloxyuridine and 2,3-dihydrofuran in an acid catalyzed reaction, followed by removal of disiloxy protection by ammonium fluoride. The chromatographically purified material was protected at the 5'-O position by pivaloyl chloride in pyridine (Example 1) to obtain, after purification, the product **42.** Phosphorylation of this nucleoside with bis-triazolide **13** gave a phosphodiester **43** which was converted to the isopropyl phosphotriester **44.**

### Compound 45

Compound **44** (0.62 g, 0.96 mmol) and N-FMOC aminoxy PEG 1500 (3.5 g, 2.0 mmol) were dried by repetitive coevaporation with dry acetonitrile (3 x 50 mL) and dissolved in dry acetonitrile (5 mL). Subsequently, triphenylphosphine (0.26 g, 1 mmol) was added in a single portion, followed by dropwise addition of diisopropyl azodicarboxylate (0.25 g, 1.2 mmol). The mixture was slowely stirred at RT for a period of 18 hours. This reaction mixture was analyzed on an analytical C18-HPLC column and purified on a preparative RP-HPLC column using triethylammonium acetate pH 7.0 /acetonitrile gradient for elution. The isolated, pure material **45** was evaporated, coevaporated with toluene and dried overnight on a high vacuum pump. Yield 1.54 g, 67%.

### Compound 46

Aldehyde phosphotriester **45** (0.8 g, 0.34 mmol) in methanol (10 mL) was reduced by addition of solid sodium borohydride as in Example 2. After 1 minute, the mixture was partitioned between sodium bicarbonate and dichloromethane. The organic phase was evaporated, coevaporated with toluene, and analyzed by analytical HPLC. The yield of **46** was assumed to be quantitative.

### Compound 47

Activation of benzylic hydroxyl of **46** was performed using (0.25 g, 0.1 mmol) of **46** according to the standard procedure (Example 2). HPLC analysis of the isolated material showed a complete conversion of compound **46** to the reagent **47.** The material was evaporated and dried by coevaporation with toluene.

## Claims

1. A compound of Formula (I) 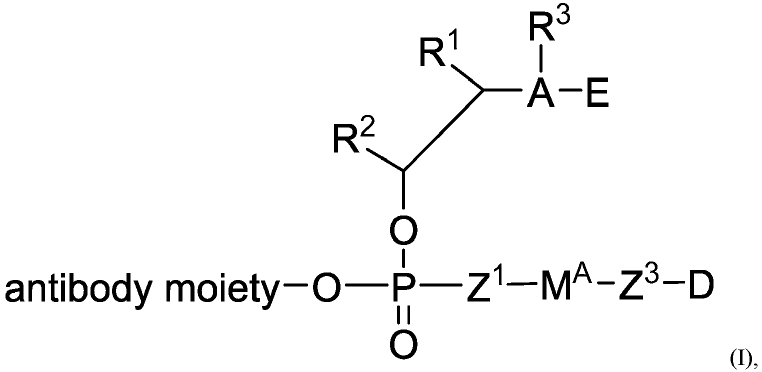 or a pharmaceutically acceptable salt thereof, wherein:
the antibody moiety is selected from:
antibody-L-(CH₂)_{q}- and antibody-L-(aliphatic moiety)-;
the aliphatic moiety is selected from a polymer, R^{P}, and a group selected from:
-polymer-L-(CH₂)ₘ- and -polymer-L-(CH₂-CH₂-O)ₚ-(CH₂)ₘ-;
R^{P} is selected from optionally substituted C₁₋₆ alkyl, optionally substituted C₁₋₃ alkyl-O-(CH₂-CH₂-O)ₚ-(CH₂)ₘ-, and optionally substituted C₃₋₇ cycloalkyl;
each L is independently a linking group;
m, p, and q are each independently an integer from 1 to 10;
D is a residue of a cytotoxic or chemotherapeutic compound;
Z¹ is selected from O, S, and N(R^{N});
Z³ is selected from O and N(R^{N}), or Z³ is absent;
A is O or N, wherein when A is O then R³ is absent;
R^{N} is selected from H and optionally substituted C₁₋₆ alkyl;
R³ is selected from H and C₁₋₆ alkyl, or
R³ and R¹, together with A and the carbon atom to which R¹ is attached, form an optionally substituted 4 to 7 membered aliphatic heterocyclic ring; or
R³ and R², together with A, the carbon atom to which R¹ is attached, and the carbon atom to which R² is attached, form an optionally substituted 4 to 8 membered aliphatic heterocyclic ring;
M^{A} is a self-immolative group having any one of formulae (a)-(i): 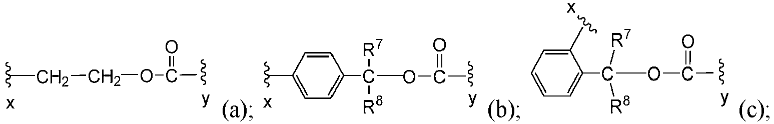 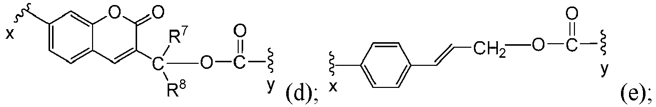 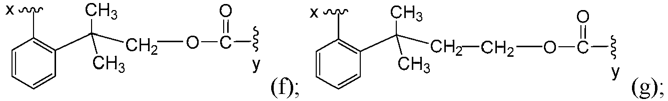 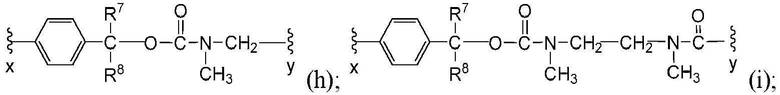 wherein x denotes a point of attachment to Z¹ and y denotes a point of attachment to z3;
R¹ and R² are independently selected from the group consisting of hydrogen, optionally substituted C₁₋₆ alkyl, optionally substituted C₆₋₁₀ aryl and optionally substituted 5- to 14-membered heteroaryl;
or R¹ and R² are joined together with the carbon atoms to which they are attached to form an optionally substituted C₃₋₇ cycloalkyl ring, an optionally substituted 4 to 7 membered aliphatic heterocyclic ring, an optionally substituted C₆₋₁₀ aryl or an optionally substituted 5- to 14-membered heteroaryl;
or R¹ and R² are joined together to form a ribose ring system;
R⁷ and R⁸ are independently selected from H and C₁₋₆ alkyl; and
E is a cleavable moiety.

2. The compound of claim 1, wherein the cytotoxic compound is selected from group of alkylating agents, antimetabolites, inhibitors of mitosis, and topoisomerase inhibitors.

3. The compound of any one of claims 1-2, wherein E is cleavable by an enzyme (e.g., an intracellular enzyme) selected from the group consisting of an esterase, a specific or an unspecific peptidase, a reductase, an oxidase, a glycosidase, a hydrolase, a glycosyl transferase, and a transaminase, or
wherein E is non-enzymatically cleavable at acidic pH, or
wherein E contains a dithio group which is cleavable by a biogenic thiol.

4. The compound of any one of claims 1-3, wherein E is an acyl group, an O-methyl-acyl group, a methyl azido group, a sugar residue, a protected acetal, or a carbonate ester.

5. The compound of claim 4, wherein A is O and E is a group of formula: 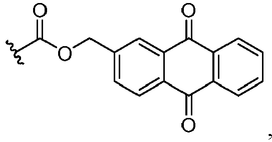 or wherein E is a group of any one of the following formulae: 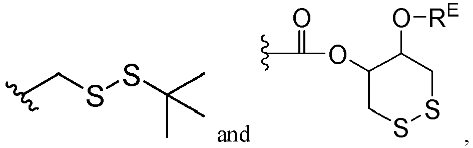 wherein R^{E} is selected from the group consisting of C₁₋₆ alkyl and benzyl, or wherein A is O, and E is a group of formula:

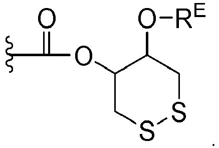

6. The compound of any one of claims 1-2, wherein a cleavable moiety E is attached to A using a group of formula (L^{E}): 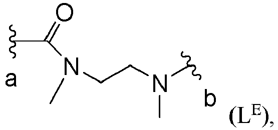 wherein a denotes a point of attachment to A, and b denotes a point of attachment to E.

7. The compound of any one of claims 1-6, wherein R¹ and R² together form C₃₋₇ cycloalkyl ring, optionally wherein the C₃₋₇ cycloalkyl ring is selected from the group consisting of cyclopropyl, cyclobutyl, cyclopentyl and cyclohexyl, or
wherein R¹ and R² together form a 4 to 7 membered aliphatic heterocyclic ring,
optionally wherein the 4 to 7 membered aliphatic heterocyclic ring is selected from the group consisting of pyrrolidine, piperidine, tetrahydrofuran and tetrahydropyran.

8. The compound of any one of claims 1-6, wherein R¹ and R² together form a ribose ring system of a ribonucleoside of formula: 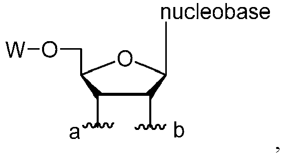 wherein either a denotes a point of attachment to O and b denotes a point of attachment to A, a denotes a point of attachment to A and b denotes a point of attachment to O, and wherein W is selected from the group consisting of H, an acyl group, and a protecting group,
optionally wherein the nucleobase is selected from the group consisting of adenine, cytosine, guanine, thymine, uracil, and other natural and non-natural nucleobases, or
wherein R¹ and R² together form a ribose ring system of formula:

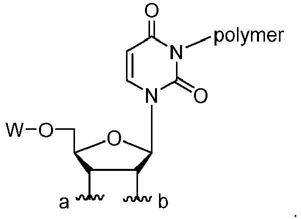

9. The compound of any one of claims 1-8, wherein A is NR³, wherein R³ and R¹, together with A and the carbon atom to which R¹ is attached, form an optionally substituted 4 to 7 membered aliphatic heterocyclic ring,
optionally wherein the 4 to 7 membered aliphatic heterocyclic ring is selected from the group consisting of: 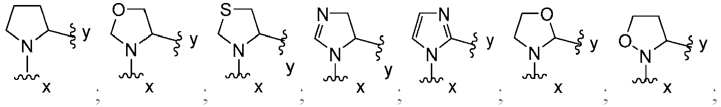 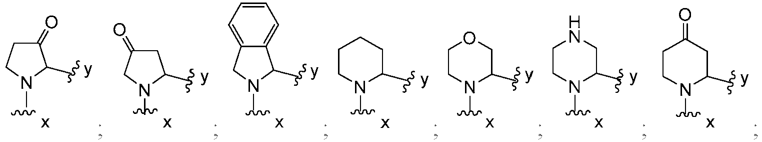 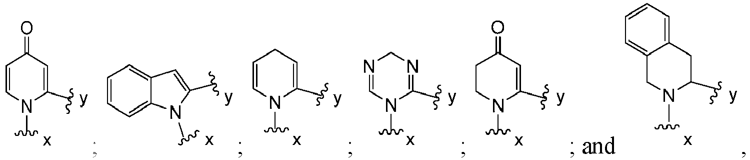 wherein x denotes a point of attachment to E, and y denotes a point of attachment to the carbon atom to which R¹ is attached.

10. The compound of any one of claims 1-9, wherein the aliphatic moiety is selected from a polymer, R^{P}, and a group of formula:
-polymer-L-(CH₂)ₘ-;
R^{P} is selected from optionally substituted C₁₋₆ alkyl and optionally substituted C₃₋₇ cycloalkyl; and
m is an integer from 1 to 10.

11. The compound of any one of claims 1-10, wherein L is a linking group of any one of the following formulae: 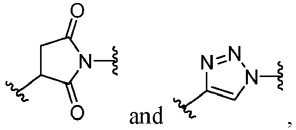
wherein 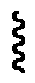 indicates a point of attachment of the linking group to the polymer or to the CH₂ group, or
wherein the linking group L is a linking group of formulae: 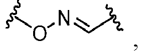
wherein 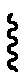 indicates a point of attachment of the linking group to the polymer or to the CH₂ group, or
wherein the linking group L comprises a group of formula (L¹): 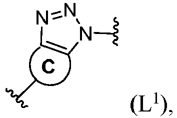
wherein ring C is selected from the group consisting of an optionally substituted C₈₋₁₆ cycloalkyl and an optionally substituted 8-16-membered heterocycloalkyl, and 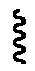 indicates a point of attachment of the linking group to the polymer or to the CH₂ group.

12. The compound of any one of claims 1-11, wherein the polymer is selected from the group consisting of poly(alkylene glycol), poly(oxyethylated polyol), poly(olefinic alcohol), poly(α-hydroxy acid), poly(vinyl alcohol), polyoxazoline, and copolymers thereof wherein the polymer is a linear or branched polyethylene glycol.

13. The compound of any one of claims 1-12, wherein Z¹ is S and M^{A} is a self-immolative group of formula (a): 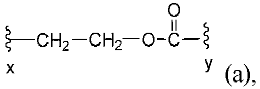 wherein x denotes a point of attachment to Z¹ and y denotes a point of attachment to Z³.

14. A pharmaceutical composition comprising the compound of any one of claims 1-13, or a pharmaceutically acceptable salt thereof, and a pharmaceutically acceptable carrier.

15. The compound of any one of claims 1-13, or a pharmaceutically acceptable salt thereof, or the pharmaceutical composition of claim 14 for use in a method of treating cancer in a subject in need thereof, the method comprising administering to the subject a therapeutically effective amount of said compound orpharmaceutically acceptable salt thereof, or of said pharmaceutical composition.

## Patentansprüche

1. Verbindung der Formel (I) 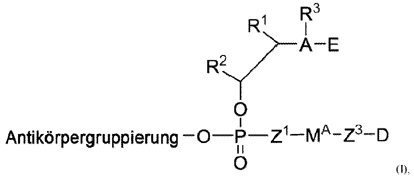 oder ein pharmazeutisch unbedenkliches Salz davon, wobei:
die Antikörpergruppierung aus:
Antikörper-L-(CH₂)_{q}- und Antikörper-L-(aliphatische Gruppierung)-
ausgewählt ist;
die aliphatische Gruppierung aus einem Polymer, R^{P} und einer Gruppe ausgewählt aus:
Polymer-L-(CH₂)ₘ- und Polymer-L-(CH₂-CH₂-O)_{P}-(CH₂)ₘ-ausgewählt ist;
R^{P} aus gegebenenfalls substituiertem C₁₋₆-Alkyl, gegebenenfalls substituiertem C₁₋₃-Alkyl-O-(CH₂-CH₂-O)ₚ-(CH₂)ₘ- und gegebenenfalls substituiertem C₃₋₇-Cycloalkyl ausgewählt ist;
L jeweils unabhängig für eine Verbindungsgruppe steht;
m, p und q jeweils unabhängig für eine ganze Zahl von 1 bis 10 stehen;
D für einen Rest einer cytotoxischen oder chemotherapeutischen Verbindung steht;
Z¹ aus O, S und N(R^{N}) ausgewählt ist;
Z³ aus O und N(R^{N}) ausgewählt ist oder Z³ fehlt;
A für O oder N steht, wobei dann, wenn A für O steht,
R³ fehlt;
R^{N} aus H und gegebenenfalls substituiertem C₁₋₆-Alkyl ausgewählt ist;
R³ aus H und C₁₋₆-Alkyl ausgewählt ist oder
R³ und R¹ zusammen mit A und dem Kohlenstoffatom, an das R¹ gebunden ist, einen gegebenenfalls substituierten 4- bis 7-gliedrigen aliphatischen heterocyclischen Ring bilden oder
R³ und R², zusammen mit A, dem Kohlenstoffatom, an das R¹ gebunden ist, und dem Kohlenstoffatom, an das R² gebunden ist, einen gegebenenfalls substituierten 4- bis 8-gliedrigen aliphatischen heterocyclischen Ring bilden;
M^{A} für eine sich selbst eliminierende Gruppe mit einer der Formeln (a)-(i) steht: 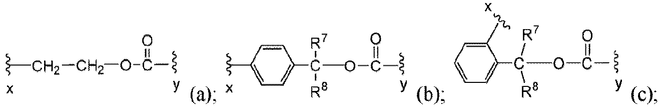 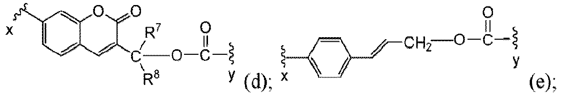 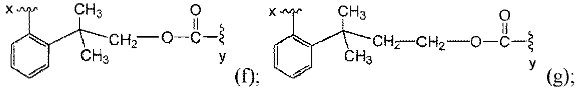 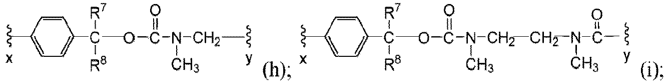
wobei x einen Anbindungspunkt an Z¹ kennzeichnet und y einen Anbindungspunkt an Z³ kennzeichnet;
R¹ und R² unabhängig aus der aus Wasserstoff, gegebenenfalls substituiertem C₁₋₆-Alkyl, gegebenenfalls substituiertem C₆₋₁₀-Aryl und gegebenenfalls substituiertem 5- bis 14-gliedrigem Heteroaryl bestehenden Gruppe ausgewählt sind oder R¹ und R² zusammen mit den Kohlenstoffatomen, an die sie gebunden sind, einen gegebenenfalls substituierten C₃₋₇-Cycloalkylring, einen gegebenenfalls substituierten 4- bis 7-gliedrigen aliphatischen heterocyclischen Ring, ein gegebenenfalls substituiertes C₆₋₁₀-Aryl oder ein gegebenenfalls substituiertes 5- bis 14-gliedriges Heteroaryl bilden
oder R¹ und R² zusammen ein Ribose-Ringsystem bilden;
R⁷ und R⁸ unabhängig aus H und C₁₋₆-Alkyl ausgewählt sind und
E für eine abspaltbare Gruppierung steht.

2. Verbindung nach Anspruch 1, wobei die cytotoxische Verbindung aus der Gruppe der Alkylierungsmittel, Antimetabolite, Mitoseinhibitoren und Topoisomeraseinhibitoren ausgewählt ist.

3. Verbindung nach einem der Ansprüche 1-2, wobei E durch ein aus der aus einer Esterase, einer spezifischen oder einer unspezifischen Peptidase, einer Reduktase, einer Oxidase, einer Glykosidase, einer Hydrolase, einer Glykosyltransferase und einer Transaminase bestehenden Gruppe ausgewähltes Enzym (z. B. ein intrazelluläres Enzym) abspaltbar ist oder
wobei E nichtenzymatisch bei saurem pH-Wert abspaltbar ist oder
wobei E eine durch ein biogenes Thiol spaltbare Dithiogruppe enthält.

4. Verbindung nach einem der Ansprüche 1-3, wobei E für eine Acylgruppe, eine O-Methylacylgruppe, eine Methylazidogruppe, einen Zuckerrest, ein geschütztes Acetal oder einen Carbonatester steht.

5. Verbindung nach Anspruch 4, wobei A für O steht und E für eine Gruppe der Formel: 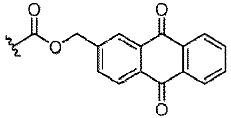 steht oder wobei E für eine Gruppe einer der folgenden Formeln steht: 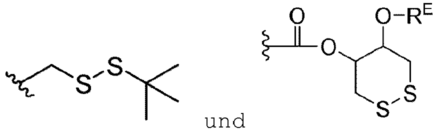 steht, wobei R^{E} aus der Gruppe bestehend aus C₁-C₆-Alkyl und Benzyl ausgewählt ist, oder wobei A für O steht und E für eine Gruppe der Formel: 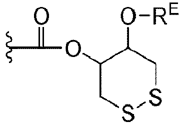 steht.

6. Verbindung nach einem der Ansprüche 1-2, wobei eine abspaltbare Gruppierung E unter Verwendung einer Gruppe der Formel (L^{E}) : 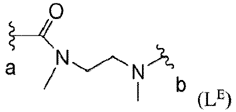 an A gebunden ist, wobei a einen Anbindungspunkt an A kennzeichnet und b einen Anbindungspunkt an E kennzeichnet.

7. Verbindung nach einem der Ansprüche 1-6, wobei R¹ und R² zusammen einen C₃₋₇-Cycloalkylring bilden, gegebenenfalls wobei der C₃₋₇-Cycloalkylring aus der aus Cyclopropyl, Cyclobutyl, Cyclopentyl und Cyclohexyl bestehenden Gruppe ausgewählt ist, oder
wobei R¹ und R² zusammen einen 4- bis 7-gliedrigen aliphatischen heterocyclischen Ring bilden, gegebenenfalls wobei der 4- bis 7-gliedrige aliphatische heterocyclischen Ring aus der Gruppe bestehend aus Pyrrolidin, Piperidin, Tetrahydrofuran und Tetrahydropyran ausgewählt ist.

8. Verbindung nach einem der Ansprüche 1-6, wobei R¹ und R² zusammen ein Ribose-Ringsystem eines Ribonukleosids der Formel: 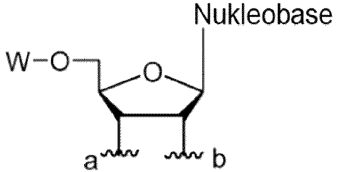
bilden, wobei entweder a einen Anbindungspunkt an O kennzeichnet und b einen Anbindungspunkt an A kennzeichnet, a einen Anbindungspunkt an A kennzeichnet und b einen Anwendungspunkt an O kennzeichnet und wobei W aus der aus H, einer Acylgruppe und einer Schutzgruppe bestehenden Gruppe ausgewählt ist,
gegebenenfalls wobei die Nukleobasen aus der aus Adenin, Cytosin, Guanidin, Thymin, Uracil und anderen natürlichen und nicht natürlichen Nukleobasen bestehenden Gruppe ausgewählt ist, oder wobei R¹ und R² zusammen ein Ribose-Ringsystem der Formel: 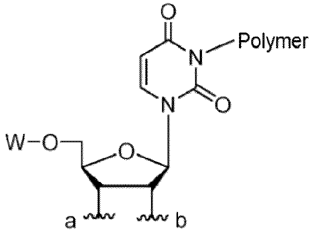 bilden.

9. Verbindung nach einem der Ansprüche 1-8, wobei A für NR³ steht, wobei R³ und R¹ zusammen mit A und dem Kohlenstoffatom, an das R¹ gebunden ist, einen gegebenenfalls substituierten 4- bis 7-gliedrigen aliphatischen heterocyclischen Ring bilden,
gegebenenfalls wobei der 4- bis 7-gliedrige aliphatische heterocyclischen Ring aus der aus: 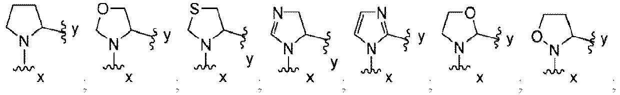 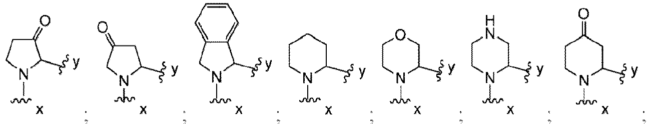 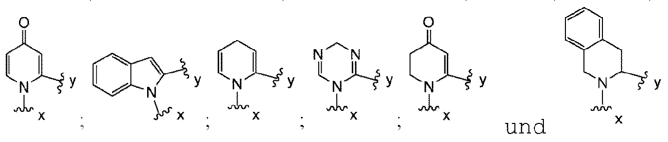
bestehenden Gruppe ausgewählt ist,
wobei x einen Anbindungspunkt an E kennzeichnet und y einen Anbindungspunkt an das Kohlenstoffatom, an das R¹ gebunden ist, kennzeichnet.

10. Verbindung nach einem der Ansprüche 1-9, wobei die aliphatische Gruppierung aus einem Polymer, R^{P} und einer Gruppe der Formel:
Polymer-L-(CH₂)ₘ-
ausgewählt ist;
R^{P} aus gegebenenfalls substituiertem C₁₋₆-Alkyl und gegebenenfalls substituiertem C₃₋₇-Cycloalkyl ausgewählt ist und
m für eine ganze Zahl von 1 bis 10 steht.

11. Verbindung nach einem der Ansprüche 1-10, wobei L für eine Verbindungsgruppe einer der folgenden Formeln steht: 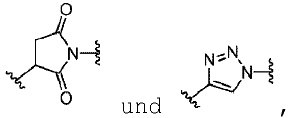
wobei 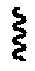 einen Anbindungspunkt der Verbindungsgruppe an das Polymer oder an die CH₂-Gruppe kennzeichnet, oder
wobei die Verbindungsgruppe L für eine Verbindungsgruppe der Formel: 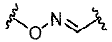
steht, wobei 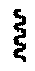 einen Anbindungspunkt der Verbindungsgruppe an das Polymer oder an die CH₂-Gruppe kennzeichnet, oder
wobei die Verbindungsgruppe L eine Gruppe der Formel (L1) : 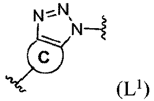
umfasst, wobei Ring C aus der aus gegebenenfalls substituiertem C₈₋₁₆-Cycloalkyl und gegebenenfalls substituiertem 8- bis 16-gliedrigem Heterocycloalkyl bestehenden Gruppe ausgewählt ist und 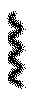 einen Anbindungspunkt der Verbindungsgruppe an das Polymer oder an die CH₂-Gruppe kennzeichnet.

12. Verbindung nach einem der Ansprüche 1-11, wobei das Polymer aus der aus Poly(alkylenglykol), Poly(oxyethyliertem Polyol), Poly(olefinischem Alkohol), Poly(α-hydroxysäure), Poly(vinylalkohol), Polyoxazolin und Copolymeren davon bestehenden Gruppe ausgewählt ist, wobei es sich bei dem Polymer um ein lineares oder verzweigtes Polyethylenglykolen handelt.

13. Verbindung nach einem der Ansprüche 1-12, wobei Z¹ für S steht und M^{A} für eine sich selbst eliminierende Gruppe der Formel (a): 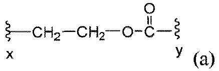 steht, wobei x einen Anbindungspunkt an Z¹ kennzeichnet und y einen Anbindungspunkt an Z³ kennzeichnet.

14. Pharmazeutische Zusammensetzung, umfassend die Verbindung nach einem der Ansprüche 1-13 oder ein pharmazeutisch unbedenkliches Salz davon und einen pharmazeutisch unbedenklichen Träger.

15. Verbindung nach einem der Ansprüche 1-13 oder ein pharmazeutisch unbedenkliches Salz davon oder pharmazeutische Zusammensetzung nach Anspruch 14 zur Verwendung in einem Verfahren zur Behandlung von Krebs in einem dessen bedürftigen Subjekt, wobei das Verfahren die Verabreichung einer therapeutisch wirksamen Menge der Verbindung oder des pharmazeutisch unbedenklichen Salzes davon oder der pharmazeutischen Zusammensetzung an das Subjekt umfasst.

## Revendications

1. Composé de formule (I) 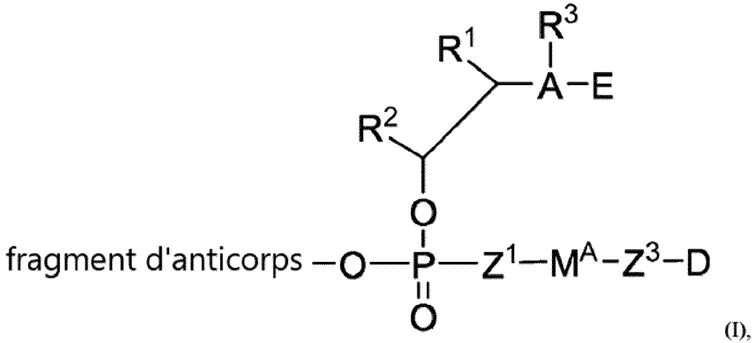 ou sel pharmaceutiquement acceptable correspondant, le fragment d'anticorps étant choisi parmi :
anticorps-L-(CH₂)_{q}- et anticorps-L-(groupement aliphatique)- ;
le groupement aliphatique étant choisi parmi un polymère, R^{p}, et un groupe choisi parmi :
-polymère-L-(CH₂)m- et -polymère-L-(CH₂-CH₂-O)ₚ-(CH₂)ₘ- ;
R^{p} étant choisi parmi C₁₋₆ alkyle éventuellement substitué, C₁₋₃ alkyle éventuellement substitué-O-(CH₂-CH₂-O)ₚ-(CH₂)ₘ-, et C₃₋₇ cycloalkyle éventuellement substitué ;
chaque L étant indépendamment un groupe de liaison ;
m, p et q étant chacun indépendamment un entier de 1 à 10 ;
D étant un radical d'un composé cytotoxique ou chimiothérapeutique ;
Z¹ étant choisi parmi O, S, et N(R^{N}) ;
Z³ étant choisi parmi O et N(R^{N}) ou Z³ étant absent ;
A étant O ou N, dans lequel lorsque A est O alors R³ est absent ;
R^{N} étant choisi parmi H et C₁₋₆ alkyle éventuellement substitué ;
R³ étant choisi parmi H et C₁₋₆ alkyle, ou
R³ et R¹, conjointement avec A et l'atome de carbone auquel R¹ est fixé, formant un cycle hétérocyclique aliphatique à 4 à 7 chaînons éventuellement substitué ; ou
R³ et R², conjointement avec A, l'atome de carbone auquel R¹ est fixé, et l'atome de carbone auquel R² est fixé, formant un cycle hétérocyclique aliphatique à 4 à 8 chaînons éventuellement substitué ;
M^{A} étant un groupe auto-immolateur ayant l'une quelconque des formules (a)-(i) : 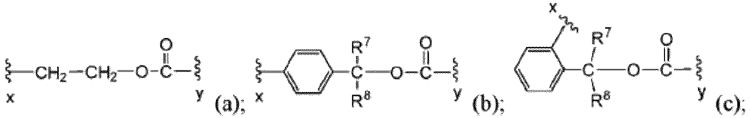 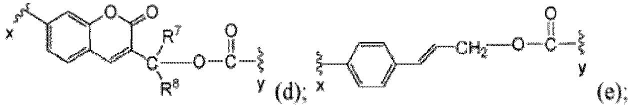 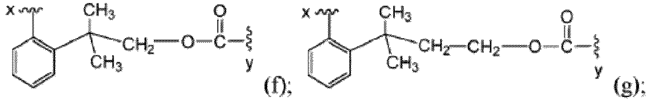 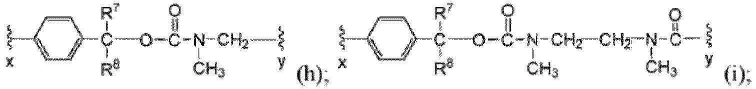
x désignant un point de fixation à Z¹ et y désignant un point de fixation à Z³ ;
R¹ et R² étant indépendamment choisis dans le groupe constitué par hydrogène, C₁₋₆ alkyle éventuellement substitué, C₆₋₁₀ aryle éventuellement substitué et hétéroaryle à 5 à 14 chaînons éventuellement substitué ;
ou R¹ et R² étant joints conjointement avec les atomes de carbone auxquels ils sont fixés pour former un cycle C₃₋₇ cycloalkyle éventuellement substitué, un cycle hétérocyclique aliphatique à 4 à 7 chaînons éventuellement substitué, un C₆₋₁₀ aryle éventuellement substitué ou un hétéroaryle à 5 à 14 chaînons éventuellement substitué ;
ou R¹ et R² étant joints ensemble pour former un système cyclique de ribose ;
R⁷ et R⁸ étant indépendamment choisis parmi H et C₁₋₆ alkyle ; et
E étant un groupement clivable.

2. Composé selon la revendication 1, le composé cytotoxique étant choisi dans le groupe composé par les agents alkylants, les antimétabolites, les inhibiteurs de mitose et les inhibiteurs de topoisomérase.

3. Composé selon l'une quelconque des revendications 1 et 2, E étant clivable par une enzyme (par ex., une enzyme extracellulaire) choisie dans le groupe constitué par une estérase, une peptidase spécifique ou une peptidase non spécifique, une réductase, une oxydase, une glycosidase, une hydrolase, une glycosyltransférase et une transaminase, ou
E n'étant pas clivable de manière enzymatique à pH acide, ou
E contenant un groupe dithio qui est clivable par un thiol biogénique.

4. Composé selon l'une quelconque des revendications 1 à 3, E étant un groupe acyle, un groupe O-méthyl-acyle, un groupe méthylazido, un radical de sucre, un acétal protégé ou un ester de carbonate.

5. Composé selon la revendication 4, A étant O et E étant un groupe de formule : 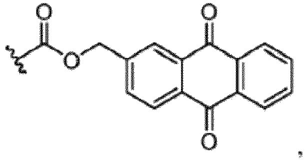 ou
E étant un groupe parmi l'une quelconque des formules suivantes : 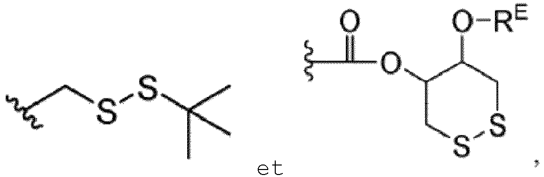
R^{E} étant choisi dans le groupe constitué par C₁₋₆ alkyle et benzyle, ou
A étant O, et E étant un groupe de formule :

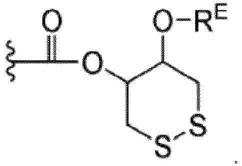

6. Composé selon l'une quelconque des revendications 1 et 2, un groupement clivable E étant fixé à A en utilisant un groupe de formule (L^{E}) : 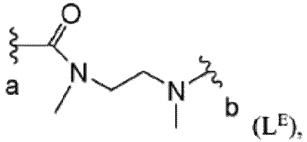 a désignant un point de fixation à A, et b désignant un point de fixation à E.

7. Composé selon l'une quelconque des revendications 1 à 6, R¹ et R² formant ensemble un cycle C₃₋₇ cycloalkyle, éventuellement, le cycle C₃₋₇ cycloalkyle étant choisi dans le groupe constitué par cyclopropyle, cyclobutyle, cyclopentyle et cyclohexyle, ou
R¹ et R² formant ensemble un cycle hétérocyclique aliphatique à 4 à 7 chaînons,
éventuellement, le cycle hétérocyclique aliphatique à 4 à 7 chaînons étant choisi dans le groupe constitué par pyrrolidine, pipéridine, tétrahydrofuranne et tétrahydropyranne.

8. Composé selon l'une quelconque des revendications 1 à 6, R¹ et R² formant ensemble un système cyclique de ribose d'un ribonucléoside de formule : 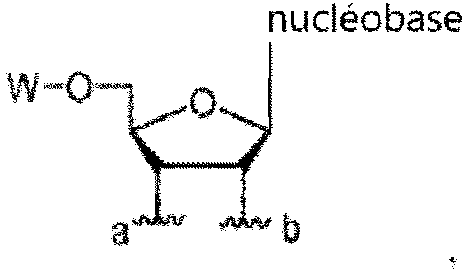
soit a désignant un point de fixation à O et b désignant un point de fixation à A, a désignant un point de fixation à A et b désignant un point de fixation à O, et W étant choisi dans le groupe constitué par H, un groupe acyle et un groupe protecteur,
éventuellement, la nucléobase étant choisie dans le groupe constitué par adénine, cytosine, guanine, thymine, uracile et d'autres nucléobases naturelles et non naturelles, ou
R¹ et R² formant ensemble un système cyclique de ribose de formule :

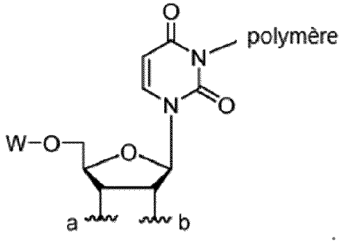

9. Composé selon l'une quelconque des revendications 1 à 8, A étant NR³, dans lequel R³ et R¹, conjointement avec A et l'atome de carbone auquel R¹ est fixé, formant un cycle hétérocyclique aliphatique à 4 à 7 chaînons éventuellement substitué,
éventuellement, le cycle hétérocyclique aliphatique à 4 à 7 chaînons étant choisi dans le groupe constitué par : 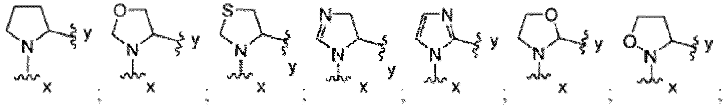 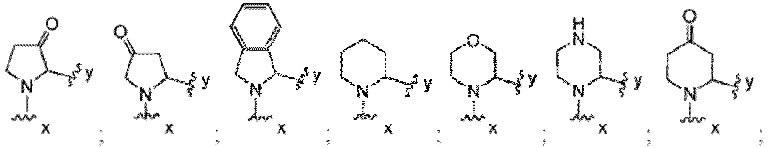 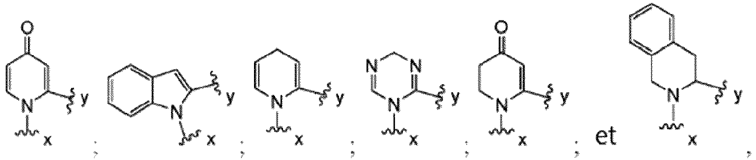
x désignant un point de fixation à E, et y désignant un point de fixation à l'atome de carbone auquel R¹ est fixé.

10. Composé selon l'une quelconque des revendications 1 à 9, le groupement aliphatique étant choisi parmi un polymère, R^{P} et un groupe de formule :
-polymère-L-(CH₂)ₘ-;
R^{P} étant choisi parmi C₁₋₆ alkyle éventuellement substitué et C₃₋₇ cycloalkyle éventuellement substitué ; et
m étant un entier de 1 à 10.

11. Composé selon l'une quelconque des revendications 1 à 10, L étant un groupe de liaison selon l'une quelconque des formules suivantes : 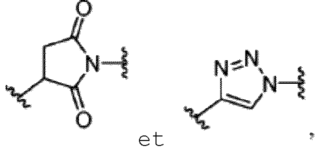
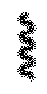 indiquant un point de fixation du groupe de liaison au polymère ou au groupe CH₂, ou
le groupe de liaison L étant un groupe de liaison des formules : 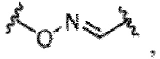
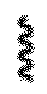 indiquant un point de fixation du groupe de liaison au polymère ou au groupe CH₂, ou
le groupe de liaison L comprenant un groupe de formule (L¹) : 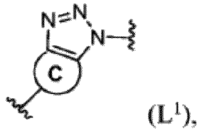
le cycle C étant choisi dans le groupe constitué par un C₈₋₁₆ cycloalkyle éventuellement substitué et un hétérocycloalkyle à 8 à 16 chaînons éventuellement substitué, et 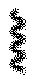 indiquant un point de fixation du groupe de liaison au polymère ou au groupe CH₂.

12. Composé selon l'une quelconque des revendications 1 à 11, le polymère étant choisi dans le groupe constitué par un poly(alkylène glycol), un poly(polyol oxyéthylé), un poly(alcool oléfinique), un poly(a-hydroxyacide), un poly(alcool vinylique), une polyoxazoline et des copolymères correspondants, le polymère étant un polyéthylène glycol linéaire ou ramifié.

13. Composé selon l'une quelconque des revendications 1 à 12, Z¹ étant S et M^{A} étant un groupe auto-immolateur de formule (a) : 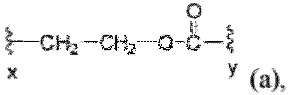 x désignant un point de fixation à Z¹ et y désignant un point de fixation à Z³.

14. Composition pharmaceutique comprenant le composé selon l'une quelconque des revendications 1 à 13, ou un sel pharmaceutiquement acceptable correspondant et un support pharmaceutiquement acceptable.

15. Composé selon l'une quelconque des revendications 1 à 13, ou sel pharmaceutiquement acceptable correspondant, ou composition pharmaceutique selon la revendication 14 pour une utilisation dans un procédé de traitement d'un cancer chez un sujet qui en a besoin, le procédé comprenant une administration au sujet d'une quantité thérapeutiquement efficace dudit composé ou du sel pharmaceutiquement acceptable correspondant, ou de ladite composition pharmaceutique.
